# FASCICULE DE BREVET EUROPEEN

(11) **EP 1 370 547 B1**
(45) Date de publication et mention de la délivrance du brevet: **15.09.2004**
(21) Numéro de dépôt: 02759802.8
(22) Date de dépôt: 20.03.2002
(51) Int. Cl.: C07D 327/02, C07C 323/22, A61K 31/39, A61P 9/10

(54) **DERIVES BENZOAXATHIEPINES ET LEUR UTILISATION COMME MEDICAMENTS**
BENZOAXATHIEPIN-DERIVATE UND DEREN MEDIZINISCHE VERWENDUNG
BENZOAXATHIEPIN DERIVATIVES AND THEIR USE AS MEDICINES

(30) Priorité: 22.03.2001 FR 0103877
(43) Date de publication de la demande: 17.12.2003
(73) Titulaire: PIERRE FABRE MEDICAMENT, 92100 Boulogne-Billancourt (FR)
(72) Inventeur: VACHER, Bernard, F-81100 Castres (FR); CASTANT-CUISIAT, Florence, F-81100 Castres (FR); JOHN, Gareth, F-81100 Castres (FR); LEGRAND, Bruno, F-81440 Lautrec (FR)
(74) Mandataire: Warcoin, Jacques
(86) Numéro de dépôt international: PCT/FR2002/000969
(87) Numéro de publication internationale: WO 2002/081464

(56) Documents cités:
- EP-A- 0 300 088

## Description

La présente invention a pour objet des dérivés de la 3-arylthio-propyl-amino-3,4-dihydro-2H-1,5-benzoxathiépine, leur procédé de préparation et leur utilisation comme médicament

Des dérivés indoliques de formule: 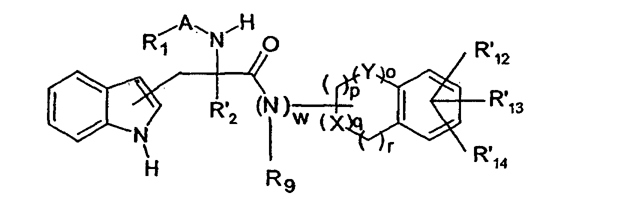 dans laquelle:
R₁ = cycloalkyle en C₃-C₁₂ ou polycycloalkyle en C₃-C₁₂;
A = (CH₂)nCO, SO₂, S(O), NHCO, (CH₂)nCOO, SCO, O(CH₂)nCO, HC=CHCO;
R'₂ = alkyle en C₁-C₆, HC=CH₂, CCH, (CH₂)n'CH=CH₂, (CH₂)n'CCH, (CH₂)nAr, (CH₂)nOR', (CH₂)nOAr, (CH₂)nCO₂R', (CH₂)nNR₅R₆;
R₉ = H, alkyle en C₁-C₆, (CH₂)nCO₂R', (CH₂)nOAr, (CH₂)nAr, (CH₂)nNR₅R₆;
R'₁₂ = R'₁₃ = R'₁₄ = halogène;
X, Y = O, S, N, CH₂, CHR₁₂, NR₁₂, NR₁₂CO, CN, C=C, CO ou une liaison;
w = 0, 1; n = n' = 0-6;
R₅, R₆ = H ou alkyle en C₁-C₆;
R' = H ou alkyle en C₁-C₆;
Ar = résidu mono ou polycyclique, carbo ou hétérocyclique, carbo ou hétéroaromatique;
R₁₂=H
sont revendiqués dans la demande internationale WO 93/03721 comme antagonistes de la colecystokinine utiles dans le traitement des dépressions.

Des 3-oxo-3,4-dihydro-2H- 1,5-benzoxathiépine de formule: 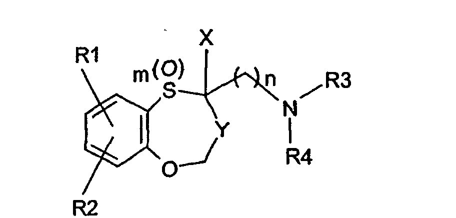 dans laquelle:
R₁, R₂ = H, alkyle, alcoxy, OH, halo;
R₃, R₄ = H, alkyle, cycloalkyle, aralkyle, hétérocyclique;
X = H, CO₂H, alkyle, aryle;
Y = (C=O), CH₂OR₅; m = 0-2; n = 1-6;
R₅= H, C₁-C₆ alkyle, phényl-C₁-C₆ alkyle non substitué ou substitué par 1 à 3 atomes d'halogène ou un groupe C₁-C₄ alkyle ou C₁-C₄ alcoxy ou methylenedioxy ou amino ou nitro ou hydroxy ; un groupe carbamoyle non substitué ou substitué par un groupe C₁-C₄ alkyle, phényle non substitué ou substitué par 1 à 3 atomes d'halogène ou C₁-C₄ alkyl ou C₁-C₄ alcoxy ou methylenedioxy ou amino ou nitro ou hydroxy ; un groupe phényle- C₁-C₄ alkyle non substitué ou substitué par 1 à 3 atomes d'halogène ou C₁-C₄ alkyle ou C₁-C₄ alcoxy ou methylenedioxy ou amino ou nitro ou hydroxy,
sont revendiqués dans les brevets EP 300 088, EP 145 494 et dans la demande internationale WO 85/02617 à la fois comme des antagonistes des récepteurs de la sérotonine du sous type 5-HT₂ et des antagonistes du canal calcique. Les mêmes composés sont revendiqués dans le brevet EP 667 156 comme des agents utiles dans le traitement des maladies oculaires.

La préparation des 3-amino-3,4-dihydro-2H-1,5-benzoxathiépine-4-carbonitriles de formule: 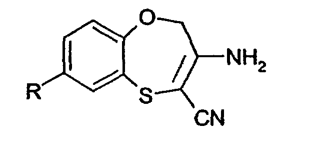 dans laquelle:
R = H, OCH₃, CH₃, Cl;
est décrite dans Chem. Pharm. Bull. 1987, 35, 1919 et WO 85/02617.

Des 1,5-benzoxathiépine-2-one-4-aryles sont rapportés comme analogues de benzodiazépines dans Synth. Commun., 1996, 26, 4459 et Med. Sci. Res. 1996, 24, 589.

Des 4-hydroxy-I,5-benzoxathiépines sont décrites dans Phosphorus Sulfur 1983, 14, 151 et J. Heterocyclic. Chem. 1994, 31, 1151.

Des 1,5-benzoxathiépine-2,4-dione sont rapportées dans J. Heterocyclic. Chem. 1982, 19, 1241 et Rapid Commun. Mass Spectrom. 1991, 5, 137.

Des 2,3-dihydro-1,5 benzothiazépines différemment substituées, apparentées au diltiazem, sont décrites dans J. Org. Chem., 1999, 64, 2219. D'autres sont revendiquées comme agonistes des récepteurs de la bradikinine (FR 2 756 566; J. Med. Chem. 2000, 43, 23 82 et 2387) ou comme inhibiteurs du neuropeptide Y (WO 98/35941) ou comme inhibiteurs de l'enzyme de conversion (US 5 723 457).

Des acides di-phosphoniques de formule : 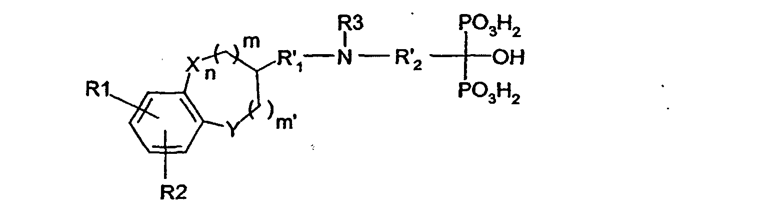 dans laquelle:
R₁ et R₂ représentent, indépendamment l'un de l'autre, un atome d'hydrogène, un radical alkyle en C₁-C₇, un radical alcoxy en C₁-C₇, un halogène ou un groupe trifluorométhyle;
R₃ représente un atome d'hydrogène ou un radical alkyle en C₁-C₇;
X et Y représentent, indépendamment l'un de l'autre, un atome de soufre ou d'oxygène;
R'₁ et R'₂ identiques ou différents représentent un radical alcoxy en C₁-C₇;
n = 0 ou 1; m et m'indépendamment l'un de l'autre = 0, 1 ou 2, la somme de n, m et
m'=1, 2 ou 3
sont revendiqués dans le brevet EP 481 920 comme régulateurs des échanges calciques.

Des dérivés de 1,5-benzoxathiépine de formule : 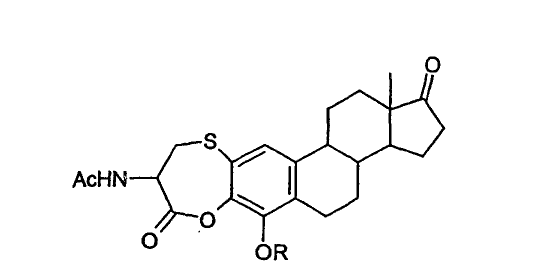 sont décrits dans Steroids 1998, 63(12), 672 et 1996, 61(5), 296 et utilisés comme outils pharmacocinétiques.

Des dérivés 3-arylthio-propyl-amino-3,4-dihydro-2H-1,5-benzoxathiépine n'ont jamais été décrits comme étant des ouvreurs, activateurs, agonistes, modulateurs, bloqueurs, inhibiteurs ou antagonistes des canaux sodiques voltage dépendants.

L'insuffisance coronarienne qui englobe différentes pathologies (e.g. l'ischémie silencieuse, l'angor stable, l'angor instable, l'infarctus du myocarde...) constitue l'une des principales causes de morbidité et de mortalité dans les pays industrialisés. Le vieillissement de la population devrait encore contribuer à aggraver cette situation dans les années à venir (Nature Medecine 1998, 4, 1241). Dans l'insuffisance coronarienne, l'état de la fonction contractile est le principal déterminant du pronostic. Or, l'atteinte de la fonction contractile ne peut être limitée que par des traitements qui préservent la viabilité des cardiomyocytes dans la zone compromise par l'ischémie.

Deux principes permettent de surseoir à la mort des cellules cardiaques exposées à l'ischémie et donc de limiter le degré de dysfonction subséquent :
- la réoxygénation rapide du tissu;
- le maintien de l'homéostasie ionique des cellules.

Si d'un côté les progrès effectués en thrombothérapie et en chirurgie cardiaque ont eu un impact positif, quantifiable en termes de bénéfices cliniques (Lancet 1994, 343, 311 ; Arch. Intern. Med. 1996, 156, 1382) de l'autre, en revanche, la contribution apportée par des agents cytoprotecteurs *per se,* est actuellement quasi inexistante (Scrip Magazine Nov. 1998, p. 15).

En effet, les médicaments utilisés dans l'insuffisance coronarienne (e.g., béta-bloquants, inhibiteurs calciques, dérivés nitrés) agissent tous de manière indirecte, principalement par un phénomène hémodynamique. Ainsi, les dérivés nitrés agissent par vasodilatation veineuse et coronaire, les béta-bloquants diminuent la fréquence et donc le travail cardiaque et les inhibiteurs des canaux calciques améliorent la perfusion cardiaque. Le Nicorandil qui est à la fois un nitrate et un activateur des canaux potassiques ATP-dépendants, est vasodilatateur et réduit le travail cardiaque (Eur. Heart J. 1999, 20, 51 ; Drugs 2000, 60(4), 955). La trimétazidine a des effets vasodilatateurs et agit sur le métabolisme énergétique des cellules exposées à l'ischémie ( Le Dictionnaire VIDAL® 74^{ième} édition, p. 1940, 1998).

Il s'ensuit que des médicaments capables de protéger directement la cellule cardiaque en situation d'ischémie (chronique ou aiguë) et donc de contribuer à préserver la fonction cardiaque en l'absence d'effet hémodynamique notable sont fortement souhaitables.

Les mécanismes impliqués dans la mort cellulaire ainsi que ceux qui s'opposent à la récupération de la fonction cardiaque après le rétablissement de la circulation sanguine sont multiples et complexes. En effet, leur contribution relative varie au cours du temps et leurs effets sont additifs. ll est néanmoins admis que l'ischémie myocardique perturbe, entre autres, le fonctionnement des canaux sodiques et de la pompe Na⁺ / K⁺. Cette dernière constitue le mécanisme principal d'expulsion des ions Na⁺ dans les cellules cardiaques (J. Mol. Cell Cardiol. 1998, 30, 337). Ces effets combinés sont probablement, impliqués dans l'accumulation intracellulaire d'ions sodium observée pendant l'ischémie (Circ. Res. 1999, 84, 1401). Cette accumulation intracellulaire d'ions sodium induit, par l'intermédiaire de l'échangeur sodium-calcium, une surcharge calcique déjà pendant l'épisode ischémique et qui est encore amplifiée pendant le processus de reperfusion (Circulation 1994, 90, 391; J. Mol. Cell. Cardiol. 2000, 32, 1169). L'élévation excessive de la concentration intracellulaire en ion calcium diminue la contractilité et fragilise le cytosquelette. Une contracture peut en résulter et entraîner la mort de la cellule cardiaque. De plus, la contracture d'une cellule peut endommager les cellules adjacentes et étendre encore la zone de nécrose à l'intérieur du tissu (Circ. Res. 1999, 85, 280 ; News Physiol. Sc. 2000, 15, 326). L'altération de la fonction contractile des cellules cardiaques exposées se traduit, globalement, par une altération de la fonction cardiaque.

Compte tenu du rôle majeur joué par la surcharge sodique dans l'initiation des processus conduisant à la mort du myocyte cardiaque, de nombreux composés visant à la prévenir ont été décrits (Pharmacol. Res. 1999, 39, 169). Actuellement, deux voies différentes d'entrée des ions sodium dans la cellule font l'objet de tentatives d'interventions thérapeutiques : le canal sodique voltage dépendant et l'échangeur sodium-proton, quoique le rôle de ce dernier durant l'épisode ischémique soit controversé (J. Mol. Cell. Cardiol. 1998, 30, 829 ; Circulation 2000, 102, 1977; J. Mol. Cell Cardiol. 2000, 32, 1897). Le co-transporteur Na⁺/HCO₃⁻ constitue une troisième voie d'entrée d'ions Na⁺ dans la cellule mais sa contribution pendant l'ischémie est à l'heure actuelle inconnue (Am. J. Physiol. 1999, 276, C576).

Plusieurs inhibiteurs de l'échangeur sodium-proton sont décrits, tels que, par exemple, les composés FR 183998 et FR 168888 (Fujisawa), SM-20550 (Sumitomo), KB-R9032 (Organon), MS-31-038 (Mitsui), EMD-96785 (Merck KgaA), cariporide (Aventis), TY-12533 (Eur. J. Pharmacol. 2000,404, 221), BIIB-513 (Am. J. Physiol. 2000, 279, H1563) et ceux objets des demandes internationales WO 99/43663, WO 99/61414 et WO 99/55690. Le bénéfice clinique de cette classe de composés dans les maladies coronariennes reste, cependant, à valider (Circulation 2000, 102, 3032).

Les bloqueurs des canaux sodiques voltage dépendant ont fait l'objet, quant à eux, d'une recherche intensive depuis plusieurs décennies. Un grand nombre de composés sont, par conséquent, disponibles. Ces derniers peuvent être divisés en trois sous-classes principales suivant leur mode d'interaction avec les canaux sodiques.

La première sous-classe regroupe les antiarythmiques de classe I, les anesthésiques locaux et certains anticonvulsants (Trends in Phannacological Science 1992, 13, 352). Plusieurs représentants de cette sous-classe sont disponibles cliniquement. Les antiarythmiques de classe I, les anesthésiques locaux et certains anticonvulsants tels que, par exemple, lidocaïne, phénytoïne, flécaïnide et quinidine, possèdent un site commun d'interaction au niveau des canaux sodiques cardiaques et neuronaux (Proc. Natl. Acad. Sci. USA 1996, 93, 9270). Ces agents, néanmoins, n'exercent aucune ou n'exercent qu'une faible activité cytoprotectrice cardiaque. De plus, leur utilisation dans le traitement des maladies coronariennes présente un risque élevé d'effets secondaires. En effet, il a été montré cliniquement que des composés tels que encainide et flecainide ont un fort potentiel arythmogène lorsque le terrain électrophysiologique est altéré tel que, par exemple, au cours de l'ischémie (Am. J. Cardiol. 1996,7 (supp. 4A), 12).

La deuxième sous-classe comporte des bloqueurs ou des modulateurs des canaux sodiques neuronaux qui ne semblent pas affecter, de façon majeure, les canaux sodiques voltage dépendant cardiaques. Les composés appartenant à cette sous-classe sont principalement revendiqués pour le traitement des maladies et des troubles du système nerveux central et/ou périphérique (Exp. Opin. Pharmacother. 1999, 1, 61 ; Brain Res. Rev. 1998, 26, 16; Trends in Pharmacological Science 1995, 16, 309). Cette sous-classe regroupe des composés de classes chimiques diverses (Ion Channel Modulator 1997, 12, 594; Annual Reports in Médicinal Chemistry 1998, 33, 51 ; J. Med. Chem. 2001, 44, 115), M50463 (Brain Res. 1999, 815, 131), NS-7 (Naunyn-Schmiedeberg's Arch. Pharmacol. 1997, 355, 601), T-477 (Eur. J. Pharmacol. 2000, 398(2), 209), SUN N8075 (J. Med. Chem. 2000, 43, 3372), certains dérivés d'arylpipéridines (Bioorg. Med. Chem. Lett. 1999, 9, 2999), d'arylpipéridino-propanol (WO 99/23072), de pipéridinol (WO 00/61558), de pyrazines (WO 98/38174), de N,N-diarylguanidines (J. Med. Chem. 1998, 41, 3298), de benzoylguanidines (EP 822182), de sulfonylcyanamides. (DE 19820064 et DE 19804251), de 4-aminopyridines (Drug Dev. Res. 1998, 44, 8), de 3-aminopyrroles (J. Med Chem. 1998,41,63), d'aryl-hétérocycles aromatiques (WO 00/57877), de 5-Naphthen-1-yl-1,3-dioxane (WO 9855474), de chromanes (WO 98/47889), d'éthers cycliques (WO 98/08842), de quinones (WO 97/07109), d'hétérocycles substitués par des groupes diphényles (DE 19816880), de benzomorphanes (DE 19740110) et de benzindoles (DE 19834714). L'intérêt de ces dérivés en tant qu'agents cytoprotecteurs cardiaques parait limité.

La troisième sous-classe comprend des composés qui agissent au niveau des canaux sodiques cardiaques mais par un mécanisme différent de celui des agents antiarythmiques de classe I. En effet, ils bloquent le canal sodique non-inactivé et réduisent, ainsi, la composante du courant sodique à inactivation lente. C'est le cas du dérivé R 56865, développé à l'origine comme agent anti-anoxique / anti-hypoxique (EP 0184257), et dont l'action cardio-protectrice via le canal sodique voltage dépendant n'a été révélé qu'ultérieurement (J. Cardiovasc. Pharmacol. 1998, 31, 800). D'autres dérivés, revendiqués entre autres comme agents cytoprotecteurs cardiaques, pourraient faire partie de cette sous-classe. Il s'agit, par exemple, du dérivé CRE-319M2 (Naunyn-Schmiedeberg's Arch. Pharmacol. 1998, 358 (supp.2), 508), 1-cis-diltiazem (Eur. J. Pharmacol. 2000, 391, 217), KC 12291 (Naunyn-Schmiedeberg's Arch. Pharmacol. 1998, 358, 554), CP-060S (J. Cardiovasc. Pharmacol. 1999, 33, 70), ST-6 (Drug Data Report 2000, 22, 790), des benzofuranones décrites dans la demande internationale WO 96/12718, des benzo(thia/oxa)zines décrites dans les demandes internationales WO 97/05134 et WO 00/43391 et des aryl-isothiourées décrites dans la demande internationale WO 00/43011.

Cependant, bien que les composés appartenant à la troisième sous-classe présentent un fort potentiel en tant qu'agents cytoprotecteurs cardiaques, aucun n'est entièrement satisfaisant :
- soit à cause de leur sélectivité insuffisante vis à vis des autres canaux ioniques voltages dépendants, en particulier des canaux K⁺ et/ou Ca⁺⁺;
- soit à cause de leur sélectivité insuffisante vis à vis des canaux sodiques voltages dépendants neuronaux et/ou musculaires (squelettiques et/ou lisses);
- soit à cause de leur sélectivité insuffisante vis à vis de la composante du courant sodique à inactivation rapide;
- soit à cause de leur interaction avec d'autres systèmes réceptoriels et/ou enzymatiques.

A ce titre, la mise au point de molécules nouvelles, appartenant à la troisième sous-classe mais plus sélectives que les molécules antérieures est fortement souhaitable.

Or, les inventeurs ont découvert de manière surprenante que des composés dérivés de la 3-arylthio-propyl-amino-3,4-dihydro-2H-1,5-benzoxathiépine peuvent s'opposer spécifiquement à la surcharge sodique induite par l'ischémie en agissant directement et sélectivement au niveau du canal sodique voltage dépendant non-inactivé. De tels composés, capables d'atténuer la surcharge sodique induite par l'ischémie sont cytoprotecteurs, donc, globalement, cardioprotecteurs et, de ce fait, potentiellement utiles pour le traitement des maladies liées à une surcharge sodique, notamment de l'insuffisance coronarienne pour laquelle il existe un besoin thérapeutique important.

La présente invention a donc pour objet une nouvelle famille de composés qui répondent à la formule générale (1) 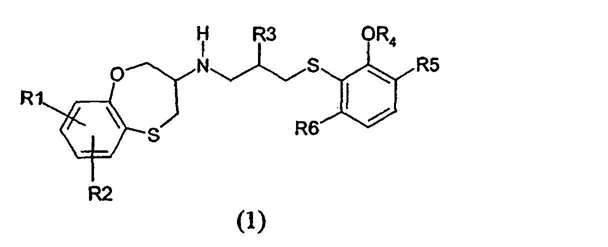 dans laquelle
R₁ et R₂, identiques ou différents, représentent :
- un atome d'hydrogène ;
- un atome de fluor ou un atome de chlore ;
- un groupe hydroxy ;
- un radical alkyle, ramifié ou non, renfermant de 1 à 3 atomes de carbone ;
- un radical cyclopropyle ;
- un radical cyclopropoxy ou
- lorsque les groupes R₁ et R₂ occupent des positions adjacentes sur le cycle aromatique, alors ils forment avec les atomes de carbone qui les portent un cycle carboné ou un hétérocycle oxygéné à cinq chaînons non aromatique ;
R₃ représente :
- un radical alkyle, ramifié ou non, renfermant de 1 à 3 atomes de carbone ;
- un groupe hydroxy ou un radical méthoxy ;
R₄ représente :
- un atome d'hydrogène ou un radical méthyle et
R₅ et R₆ identiques ou différents représentent :
- un atome d'hydrogène ;
- un radical alkyle, ramifié ou non, renfermant de 1 à 3 atomes de carbone ;
- un radical alcoxy ramifié ou non, renfermant de 1 à 3 atomes de carbone ;
- un radical alkylthio ramifié ou non, renfermant de 1 à 3 atomes de carbone ;
- un radical alkylamino,
pourvu que lorsque R₄ représente un radical méthyle alors R₅ représente un atome d'hydrogène, un radical alcoxy renfermant de 1 à 3 atomes de carbone, un radical alkylthio ramifié ou non renfermant de 1 à 3 atomes de carbone ou un radical alkylamino, ou
les groupes OR₄ et R₅ forment avec les carbones qui les portent un hétérocycle non aromatique à cinq ou six chaînons contenant au moins un atome d'oxygène, et R₆ est tel que défini précédemment,
leurs sels d'addition et les hydrates de ces sels d'addition avec les acides minéraux ou les acides organiques pharmaceutiquement acceptables,
ainsi que leurs formes tautomères, les énantiomères et les mélanges d'énantiomères, et les stéréoisomères purs ou en mélange racémique ou non.

L'invention a plus particulièrement pour objet des dérivés de formule (1) dans laquelle:
R₁ et R₂, identiques ou différents, représentent :
   - un atome d'hydrogène ;
   - un atome de fluor ou un atome de chlore ;
   - un groupe hydroxy ;
   - un radical alkyle choisi dans le groupe comprenant les radicaux méthyle, éthyle, propyle et isopropyle ;
   - un radical cyclopropyle ;
   - un radical alcoxy choisi dans le groupe comprenant les radicaux méthoxy, éthoxy, propoxy et isopropoxy;
   - un radical cyclopropoxy ou
   - lorsque les groupes R₁ et R₂ occupent des positions adjacentes sur le cycle aromatique, alors R₁R₂ représentent -CH₂CH₂CH₂-, -OCH₂CH₂-, -OCH₂O- ou -CH₂CH₂O- ;
R₃ représente :
   - un radical alkyle choisi dans le groupe comprenant les radicaux méthyle, éthyle, propyle et isopropyle;
   - un groupe hydroxy ou un radical méthoxy ;
R₄ représente :
   - un atome d'hydrogène ou un radical méthyle et
R₅ et R₆ identiques ou différents représentent :
   - un atome d'hydrogène ;
   - un radical alkyle choisi dans le groupe comprenant les radicaux méthyle, éthyle et isopropyle ;
   - un radical alcoxy choisi dans le groupe comprenant les radicaux méthoxy, éthoxy, propoxy et isopropoxy ;
   - un radical alkylthio choisi dans le groupe comprenant les radicaux méthylthio, éthylthio et isopropylthio ;
   - un radical alkylamino choisi dans le groupe comprenant les radicaux N-méthylamino et N,N-diméthylamino; ou
R₄R₅ représente un radical choisi dans le groupe comprenant -CH₂CH₂-, -CH₂O-, -CH₂CH₂O-, -CH₂CH₂S-, et -CH₂CH₂NR₄-, et R₆ est tel que défini précédemment,
leurs sels d'addition et les hydrates de ces sels d'addition avec les acides minéraux ou les acides organiques pharmaceutiquement acceptables,
ainsi que leurs formes tautomères, les énantiomères et les mélanges d'énantiomères, et les stéréoisomères purs ou en mélange racémique ou non.

Dans un mode particulier de réalisation de l'invention, des dérivés de formule (1) sont choisis dans le groupe comprenant:
3-[3-(2-Méthoxy-phénylthio)-2-méthoxy-propyl]amino-3,4-dihydro-2H-1,5-benzoxathiépine ;
3-[3-(2-Méthoxy phénylthio)-2-hydroxy:propyl]amino-3,4-dihydro-2H-1,5-benzoxathiépine ;
3-[3-{2-Hydroxy-phénylthio)-2-hydroxy-propyl]amino-3,4-dihydro-2H-1,5-benzoxathiépine ;
3-[3-(2-Méthoxy-phénylthio}-2-méthyl-propyl]amino-3,4-dihydro-2H-1,5-benzoxathiépine ;
3-[3-(2-Méthoxy-phénylthio)-2-éthyl-propyl]amino-3,4-dihydro-2H-1,5-benzoxathiépine;
3-[3-(2-Méthoxy-phénylthio)-2-n-propyl-propyl]amino-3,4-dihydro-2H-1,5-benzoxathiépine ;
3-[3-(2-Méthoxy-phénylthio)-2-iso-propyl-propyl]amino-3,4-dihydro-2H-1,5-benzoxathiépine ;
3-[3-(2-Méthoxy-phénylthio)-2-méthyl-propyl]amino-7-méthyl-3,4-dihydro-2H-1,5-benzoxathiépine ;
3-[3-(2-Méthoxy-phénylthio)-2-méthyl-propyl]amino-6-méthyl-3,4-dihydro-2H-1,5-benzoxathiépine ;
3-[3-(2-Hydroxy-3-méthyl-phénylthio)-2-méthyl-propyl]amino-3,4-dihydro-2H-1,5-benzoxathiépine ;
3-[3-(2-Hydroxy-phénylthio)-2-méthyl-propyl]amino-3,4-dihydro-2H-1,5-benzoxathiépine ;
3-[3-(2-Hydroxy-3-éthyl-phénylthio)-2-méthyl-propyl]amino-3,4-dihydro-2H-1,5-benzoxathiépine ;
3-[3-(2,3-Dihydro-benzofuran-7-thio)-2-méthyl-propyl]amino-3,4-dihydro-2H-1,5-benzoxathiépine ;
3-[3-(2-Hydroxy-3-méthyl-phénylthio)-2-éthyl-propyl]amino-3,4-dihydro-2H-1,5-benzoxathiépine ;
3-[3-(2-Hydroxy-3-méthyl-phénylthio)-2-iso-propyl-propyl]amino-3,4-dihydro-2H-1,5-benzoxathiépine ;
3-[3-(2-Hydroxy-3-méthyl-phénylthio)-2-méthyl-propyl]amino-6-méthyl-3,4-dihydro-2H-1,5-benzoxathiépine ;
3-[3-(2-Hydroxy-3-méthoxy-phénylthio)-2-méthyl-propyl]amino-3,4-dihydro-2H-1,5-benzoxathiépine ;
3-[3-(2,3-Diméthoxy-phénylthio)-2-méthyl-propyl]amino-3,4-dihydro-2H-1,5-benzoxathiépine ;
3-[3-(2-Hydroxy-3-iso-propyl-phénylthio)-2-méthyl-propyl]amino-3,4-dihydro-2H-1,5-benzoxathiépine ;
3-[3-(2-Hydroxy-6-méthyl-phénylthio)-2-méthyl-propyl]amino-3,4-dihydro-2H-1,5-benzoxathiépine,
leurs sels d'addition et les hydrates de ces sels d'addition avec les acides minéraux ou les acides organiques pharmaceutiquement acceptables,
ainsi que leurs formes tautomères, les énantiomères et les mélanges d'énantiomères, et les stéréoisomères purs ou en mélange racémique ou non.

Les composés de formule générale (1) peuvent exister sous plusieurs formes tautomères. De telles formes tautomères quoique non explicitement rapportées dans la présente demande pour simplifier la représentation graphique des formules développées sont néanmoins incluses dans le champ d'application de l'invention.

Les composés de l'invention comportent deux atomes de carbone asymétriques dans leur structure. De ce fait, ils existent sous la forme d'énantiomères et de diastéréoisomères. L'invention concerne aussi bien chaque stéréoisomère pur, c'est à dire associé avec moins de 5% d'un autre stéréoisomère ou d'un mélange d'autres stéréoisomères, que le mélange d'un ou plusieurs stéréoisomères en toutes proportions. Les composés de l'invention peuvent donc intervenir en tant que stéréoisomères purs ou mélanges racémiques ou non-racémiques de stéréoisomères. Cependant, parmi les quatre stéréoisomères existants d'un composé de formule (1), l'énantiomère dont l'atome de carbone asymétrique C(3) du fragment 3,4-dihydro-2H-1,5-benzoxathièpine est de configuration absolue (R) et dont l'atome de carbone asymétrique qui porte le groupe R₃ est de configuration absolue (S) est, dans tous les cas, préféré.

Les descripteurs R et S, utilisés pour spécifier la configuration absolue des atomes de carbone stéréogéniques contenus dans les molécules de formule (1), sont définis tels que dans la règle de priorité de Cahn-Ingold-Prelog (E.L. Eliel and S.H. Wilen Stereochemistry of Organic Compounds, 1994, John Wiley & Sons, Inc., chap. 5, 104-12).

Dans un autre mode particulier de réalisation de l'invention, les dérivés de formule générale (1) sont de configuration absolue (R) au niveau de l'atome de carbone asymétrique C(3) du fragment 3,4-dihydro-2H-1,5-benzoxathièpine et de configuration absolue (S) au niveau de l'atome de carbone asymétrique qui porte le groupe R₃.

Dans un autre mode particulièrement avantageux de réalisation de l'invention, les dérivés de formulé générale (1) sont choisis dans le groupe comprenant les stéréoisomères suivants :
3-(R)-[3-(2-Méthoxy-phénylthio)-2-(S)-méthoxy-propyl]amino-3,4-dihydro-2H-1,5-benzoxathiépine ;
3-(R)-[3-(2-Méthoxy-phénylthio)-2-(S)-hydroxy-propyl]amino-3,4-dihydro-2H-1,5-benzoxathiépine ;
3-(R)-[3-(2-Hydroxy-phénylthio)-2-(S)-hydroxy-propyl]amino-3,4-dihydro-2H-1,5-benzoxathiépine ;
3-(R)-[3-(2-Méthoxy-phénylthio)-2-(S)-méthyl-propyl]amino-3,4-dihydro-2H-1,5-benzoxathiépine ;
3-(R)-[3-(2-Méthoxy-phénylthio)-2-(S)-éthyl-propyl]amino-3,4-dihydro-2H-1,5-benzoxathiépine ;
3-(R)-[3-(2-Méthoxy-phénylthio)-2-(S)-n-propyl-propyl]amino-3,4-dihydro-2H-1,5-benzoxathiépine ;
3-(R)-[3-(2-Méthoxy-phénylthio)-2-(S)-iso-propyl-propyl]amino-3,4-dihydro-2H-1,5-benzoxathiépine ;
3-(R)-[3-(2-Méthoxy-phénylthio)-2-(S)-méthyl-propyl]amino-7-méthyl-3,4-dihydro-2H-1,5-benzoxathiépine ;
3-(R)-[3,-(2-Méthoxy-phénylthio)-2-(S)-méthyl-propyl]amino-6-méthyl-3,4-dihydro-2H-1,5-benzoxathiépine ;
3-(R)-[3-(2-Hydroxy-3-méthyl-phénylthio)-2-(S)-méthyl-propyl]amino-3,4-dihydro-2H-1,5-benzoxathiépine ;
3-(R)-[3-(2-Hydroxy-phénylthio)-2-(S)-méthyl-propyl]amino-3,4-dihydro-2H-1,5-benzoxathiépine ;
3-(R)-[3-(2-Hydroxy 3-éthyl-phénylthio)2-(S)-méthyl-propyl]amino-3,4-dihydro-2H-1,5-benzoxathiépine ;
3-(R)-[3-(2,3-Dihydro-benzofuran-7-thio)-2-(S)-méthyl-propyl]amino-3,4-dihydro-2H-1,5-benzoxathiépine ;
3-(R)-[3-(2-Hydroxy-3-méthyl-phénylthio)-2-(S)-éthyl-propyl]amino-3,4-dihydro-2H-1,5-benzoxathiépine ;
3-(R)-[3-(2-Hydroxy-3-méthyl-phénylthio)-2-(S)-iso-propyl-propyl]amino-3,4-dihydro-2H-1,5-benzoxathiépine ;
3-(R)-[3-(2-Hydroxy-3-méthyl-phénylthio)-2-(S)-méthyl-propyl]amino-6-méthyl-3,4-dihydro-2H-1,5-benzoxathiépine;
3-(R)-[3-(2-Hydroxy-3-méthoxy-phénylthio)-2-(S)-méthyl-propyl]amino-3,4-dihydro-2H-1,5-benzaxathiépine ;
3-(R)-[3-(2,3-Diméthoxy-phénylthio)-2-(S)-méthyl-propyl]amino-3,4-dihydro-2H-1,5-benzoxathiépine ;
3-(R)-[3-(2-Hydroxy-3-iso-propyl-phénylthio)-2-(S)-méthyl-propyl]amino-3,4-dihydro-2H-1,5-benzoxathiépine ;
3-(R)-[3-(2-Hydroxy-6-méthyl-phénylthio)-2-(S)-méthyl-propyl]amino-3,4-dihydro-2H-1,5-benzoxathiépine,
leurs sels d'addition et les hydrates de ces sels d'addition avec les acides minéraux ou les acides organiques pharmaceutiquement acceptables,
ainsi que leurs formes tautomères, les énantiomères et les mélanges d'énantiomères, et les stéréoisomères purs ou en mélange racémique ou non.

L'invention concerne également les sels d'addition et éventuellement les hydrates des sels d'addition des composés de formule générale (1) avec les acides minéraux ou les acides organiques pharmaceutiquement acceptables.

L'invention s'étend également au procédé de préparation des dérivés de formule générale (1).

Le procédé chimique utilisé pour la préparation des composés de formule générale (1) dépend de la nature des substituants R₃ et R₄.

Les composés de formule (1) peuvent être obtenus par l'un des procédés (a), (b) ou (c) décrits dans le schéma A qui suit est illustré à l'annexe 1.

### Schéma A

Selon le procédé (a), lorsque le radical R₃ est différent d'un groupe hydroxy et R₄ représente un groupe méthyle : le composé de formule (1) est préparé par amination réductrice de l'aldéhyde de formule (II) au moyen d'une amine primaire de formule (1), ou d'un sel de l'amine primaire de formule (I). L'aldéhyde de formule (II) peut être isolé avant d'être engagé dans la réaction d'amination réductrice ou engagé dans la réaction d'amination réductrice sans être isolé au préalable. L'agent réducteur utilisé dans la réaction d'amination réductrice en question peut être un hydrure de bore simple ou complexe tel que, par exemple, le borohydrure de sodium, le borohydrure de potassium, le cyanoborohydrure de sodium ou le triacétoxyborohydrure de sodium.

Selon le procédé (b), lorsque R₃ est un groupe hydroxy : le composé de formule (1) est préparé par ouverture régio-sélective de l'époxyde de formule (III) au moyen de l'aryl-thiophénol approprié de formule (IV (Synth. Commun. 1996, 26(23), 4459). L'époxyde de formule (III) est lui même obtenu à partir d'un précurseur du type 3-[(1-chloro-2-hydroxy-propyl)amino]-3,4-dihydro-2H-1,5-benzoxathiépine, non rapporté dans le schéma A car non isolé. En effet, les inventeurs ont découvert qu'il était expérimentalement plus avantageux de ne pas isoler l'intermédiaire en question mais d'effectuer l'étape suivante de cyclisation intramoléculaire *in situ* et de n'isoler pur que l'époxyde de formule (III). Le dit époxyde (III) provient de la réaction entre l'amine primaire de formule (I) et une épichlorhydrine, disponible commercialement, selon des techniques classiques de la chimie organique (J. Org. Chem. 1990, 55(9), 2920; WO 0048987). Dans les conditions expérimentales utilisées par les inventeurs, l'attaque nucléophile de l'amine de formule (I) sur l'épichlorhydrine est à la fois chimio- et régio-sélective.

Selon le procédé (c), lorsque R₃ est différent d'un groupe hydroxy et R₄ est un atome d'hydrogène : le composé intermédiaire de formule (VI) est préparé à partir de l'amine de formule (I) et de l'aldéhyde de formule (V) selon un procédé identique à celui décrit dans le procédé (a). Une étape de déprotection de la fonction phénol du composé (VI) permet ensuite d'aboutir au composé de formule (1) attendu (Eur. J. Org. Chem. 2000, 18, 3223).

Les composés de formule (1) peuvent être purifiés par une ou plusieurs méthodes choisies parmi les techniques de chromatographie en phase liquide. Ils peuvent être ensuite, si on le désire, salifiés au moyen d'un acide organique ou inorganique pharmacologiquement acceptable.

La préparation des amines primaires de formule (I) est décrite dans le schéma B qui suit et est illustré à l'annexe 2.

### Schéma B

L'intermédiaire du type N-Boc-(2-hydroxyphényl)-cystéinc (VIII) est préparé d'une façon similaire à celle du N-Boc-(4-hydroxyphényl)-L-cystéine décrite dans la demande internationale WO 00/20441. La fonction acide carboxylique du composé de formule (VIII) est ensuite convertie en une fonction alcool primaire. Cette réaction peut être effectuée avantageusement par réduction d'un anhydride mixte intermédiaire, formé *in situ,* au moyen d'un hydrure de bore simple ou complexe selon une technique *"one pot"* bien connue du chimiste organicien. L'alcool primaire de formule (IX) est ensuite cyclisé, par exemple au moyen d'une réaction de Mitsunobu intramoléculaire, pour donner le composé cyclique correspondant (X). L'amine primaire de formule (1) est obtenue par coupure du groupement *tert*-butoxycarbonyle au moyen d'un acide protique tel que, par exemple, l'acide trifluoroacétique (T. W. Greene et P.G.M. Wuts Protective Groups in Organic Synthesis, 1999, John Wiley & Sons, Inc., 3rd ed.; chap. 7, 518-25). L'amine primaire de formule (I) peut être, si on le désire, salifiée et conservée sous forme de chlorhydrate ou de bromhydrate cristallisé, non hygroscopique et stable dans des conditions normales de température et de lumière.

Le procédé chimique utilisé pour la préparation des aldéhydes de formule générale (II) et (V) dépend de la nature des substituants R₃ et R₄.

Les aldéhydes de formule (IIa) et (Va), cas particuliers des composés de formule (II) et (V) dans lesquels R₃ est un groupe méthoxy, peuvent être préparés selon la méthode décrite dans le schéma C qui suit et est illustré à l'annexe 3.

### Schéma C

La fonction alcool primaire de l'intermédiaire du type 3-arylthio-1,2-propanediol (XI), préparé d'une façon similaire à celle décrite dans le brevet FR 1 064 619 est protégée sous forme d'éther tritylique selon une méthode analogue à celle décrite par Kim (J. Org. Chem. 1992, 57(5), 1605). La fonction alcool secondaire du composé de formule (XII), activée sous la forme d'un alcoolate alcalin, est alors méthylée au moyen d'un halogénure ou du sulfate de méthyle pour donner le composé de formule (XIII) correspondant. La fonction alcool primaire du composé de formule (XIII) est ensuite libérée par hydrolyse du groupement triphénylméthyle en milieu acide protique (T. W. Greene et P.G.M. Wuts Protective Groups in Organic Synthesis, 1999, John Wiley & Sons, Inc., 3rd ed.; chap. 2, 102-4).

Selon la voie (a), lorsque R₄ est un radical méthyle, l'aldéhyde de formule (IIa) est obtenu par oxydation de l'alcool primaire de formule (XIV). La réaction en question peut être effectuée au moyen d'un dérivé activé du diméthylsulfoxyde tel que par exemple du diméthylsulfoxyde activé par le complexe trioxyde de sulfure-pyridine, du diméthylsulfoxyde activé par le chlorure d'oxalyle ou par un agent oxydant du type iode hypervalent tel que, par exemple, le réactif de Dess-Martin, selon des techniques classiques bien connues du chimiste organicien.

Au niveau de l'intermédiaire de formule (XIII), lorsque le radical R représente un groupe méthoxyméthyle (MOM), la détritylation en milieu acide de la fonction alcool primaire s'accompagne de l'hydrolyse du groupe méthoxyméthyle (MOM) portée par la fonction phénol. Le composé obtenu est donc, dans ce cas, le dérivé dihydroxylé (XIV, R₄ = H). La fonction phénol du dit intermédiaire (XIV, R₄ = H) doit être protégée avant d'effectuer l'oxydation de la fonction alcool primaire selon la voie (b). Ceci est réalisé par alkylation chimio-sélective de la fonction phénol au moyen de Chlorométhylméthyl éther selon un protocole expérimental identique à celui décrit dans J. Org. Chem. 1998, 63(10), 3260. L'oxydation de l'alcool primaire (XV) en l'aldéhyde de formule (Va) est ensuite conduite d'une façon similaire à celle utilisée pour l'oxydation de l'alcool (XIV, R₄ = CH₃) en l'aldéhyde (IIa), cf. voie (a).

La préparation des aldéhydes de formule (II) et (V) dans lesquels le radical R₃ représente un groupe alkyle, notamment celle des aldéhydes de formule (IIb-g) et (Vb-j), est décrite dans le schéma D qui suit et est illustré à l'annexe 4.

### Schéma D

Les aldéhydes de formule (IIb-g) et (Vb-j) dérivent d'un précurseur commun du type 2-alkyl-3-arylthio-propan-1-ol de formule (XX). Cet intermédiaire est obtenu par réaction de l'arylthiol approprié de formule (IV), ou d'un sel alcalin dérivé du dit arylthiol, sur le 3-bromo-2-méthyl-propan-1-ol, disponible commercialement, ou sur le 2-alkyl-3-hydroxy-propyl-p-toluènesulfonate de formule (XIX) dont la méthode de préparation est décrite ci-après.

Le composé de formule (XVI), préparé selon la méthode décrite par Fukumoto dans J. Org. Chem. 1996, 61(2), 677, est réduit en l'alcool (XVII) au moyen de borohydrure de lithium dans le tétrahydrofurane (THF) selon un protocole expérimental similaire à celui décrit dans J. Org. Chem. 1994, 59(18), 5317 ou dans Synth. Commun. 1990, 20(2), 307. La fonction alcool primaire du composé de formule (XVIT) est d'abord convertie en l'ester de l'acide p-toluènesulfonique (tosylate) de formule (XVIII). Le composé de formule (XVIII) peut être alors débenzylé par hydrogénolyse en présence d'un catalyseur au palladium pour donner le composé attendu (XIX), selon une méthode similaire à celle décrite dans J. Am. Chem. Soc. 1999, 121(43), 9967.

Selon la voie (a), l'intermédiaire de formule (XX), dans lesquels R₄ est un radical méthyle ou forme avec le groupe R₅ adjacent un hétérocycle, peut être oxydé directement en l'aldéhyde de formule (II), selon une méthode similaire à celle décrite précédemment pour l'oxydation de l'alcool (XIV, R₄ = CH₃) en l'aldéhyde (IIa) (cf. schéma C, voie (a)). Cependant, dans le cas où la pureté énantiomérique de l'atome de carbone asymétrique qui porte le radical R₃ dans le précurseur de formule (XX) doit être préservée dans le composé final de formule (1), les limitations suivantes s'appliquent :
- la réaction d'oxydation de l'alcool (XX) en l'aldéhyde (II) est effectuée selon la méthode de Swern modifiée selon Evans (J. Am. Chem. Soc. 1993, 115(24), 11446) ;
- l'aldéhyde de formule (II) n'est pas isolé mais utilisé *in situ* dans l'étape suivante d'amination réductrice (cf. schéma A, procédé (a)).

Selon la voie (b), la fonction phénol du composé de formule (XX) est convertie soit en l'éther méthoxyméthylique (XXIa, R = MOM), soit en l'éther méthylique (XXIb, R = CH₃). L'oxydation de l'alcool (XXI) en l'aldéhyde (II) ou (V) est ensuite effectuée selon une méthode identique à celle utilisée pour la préparation de l'aldéhyde (II) à partir de l'alcool (XX) (cf. schéma D, voie (a)). Les limitations qui s'appliquent à l'oxydation de l'alcool (XX) en l'aldéhyde (II) ainsi qu'à l'utilisation du dit aldéhyde dans la réaction suivante d'amination réductrice s'appliquent également à l'oxydation de l'alcool (XXI) en l'aldéhyde (II) ou (V) ainsi qu'à leur utilisation dans la réaction d'amination réductrice.

Les composés du type arylthiol de formule (IV), utiles comme intermédiaires dans la préparation des aldéhydes de formule (II) et (V), ainsi que dans la préparation de certaines amines primaires de formule (I), sont soit disponibles commercialement, soit décrites dans la littérature (i.e. Heterocycles 1999, 50(2), 681 ; J. Heterocyclic Chem. 1998, 35(3), 699; JP 08143533 ; JP 06293640 ; Synth. Commun. 1994, 24(1), 35 ; J. Org. Chem. 1994, 59(16), 4618 ; Drug Metab. Dispos. 1992, 20(5), 688 ; J. Org. Chem. 1990, 55(9), 2736 ; J. Med. Chem. 1990, 33(5), 1491 ; J. Med. Chem. 1989, 32(10), 2399 ; EP 200212 ; J. Org. Chem. 1979, 44(26), 4971 ; J. Pharm. Sci. 1976, 65(10), 1554; DE 2411826; Gazz. Chim. Ital. 1969, 99(11), 1095 ; Gazz. Chim. Ital. 1969, 99(4), 397 ; J. Am. Chem. Soc. 1955, 77, 568), soit préparés selon les modes opératoires décrits dans les exemples illustrant la présente invention.

L'invention a également pour objet les amines de formule (I) 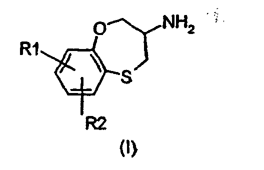 dans laquelle
R₁ et R₂, identiques ou différents, représentent :
- un atome d'hydrogène ;
- un atome de fluor ou un atome de chlore ;
- un groupe hydroxy ;
- un radical alkyle, ramifié ou non, renfermant de 1 à 3 atomes de carbone;
- un radical cyclopropyle ;
- un radical alcoxy ramifié ou non, renfermant de 1 à 3 atomes de carbone ;
- un radical cyclopropoxy ou
- lorsque les groupes R₁ et R₂ occupent des positions adjacentes sur le cycle aromatique, ils forment avec les atomes de carbone qui les portent un cycle carboné ou un hétérocycle oxygéné à cinq chaînons non aromatique;
   utiles comme intermédiaires dans la synthèse des composés de formule (I).

Dans un mode de réalisation particulièrement avantageux de l'invention, les amines de formule (1) sont celles dans lesquelles l'atome de carbone asymétrique C(3) est de configuration absolue (R).

L'invention a également pour objet le procédé de préparation des composés de formule (I) caractérisé en ce qu'on transforme la N-Boc-(2-hydroxyphényl)-cystéine de formule (VIII) 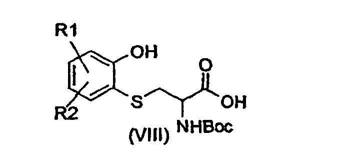 en alcool primaire de formule (IX) 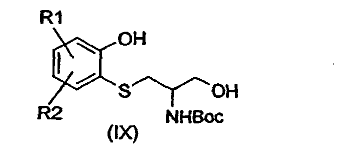 par réduction d'un anhydride mixte intermédiaire, formé *in situ,* au moyen d'un hydrure de bore simple ou complexe selon une technique "*one-pot*",
puis on cyclise ledit composé (IX), pour obtenir le composé cyclique correspondant (X) 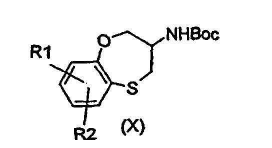 que l'on traite par un acide protique pour obtenir l'amine de formule (I) que l'on salifie si on le souhaite.

L'invention a également pour objet les aldéhydes de formule (II) 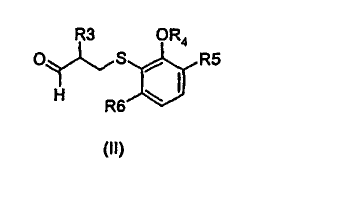 dans laquelle
R₃ représente
- un radical alkyle, ramifié ou non, renfermant de 1 à 3 atomes de carbone ou un radical méthoxy,
R₄ représente :
- un atome d'hydrogène ou un radical méthyle et
R₅ et R₆ identiques ou différents représentent :
- un atome d'hydrogène ;
- un radical alkyle, ramifié ou non, renfermant de 1 à 3 atomes de carbone ;
- un radical alcoxy ramifié ou non, renfermant de 1 à 3 atomes de carbone ;
- un radical alkylthio ramifié ou non renfermant de 1 à 3 atomes de carbone ;
- un radical alkylamino,
pourvu que lorsque R₄ représente un radical méthyle alors R₅ représente un atome d'hydrogène, un radical alcoxy renfermant de 1 à 3 atomes de carbone, un radical alkylthio ramifié ou non renfermant de 1 à 3 atomes de carbone ou un radical alkylamino, ou
- les groupes OR₄ et R₅ forment avec les carbones qui les portent un hétérocycle non aromatique à cinq ou six chaînons contenant au moins un atome d'oxygène, et R₆ est tel que défini précédemment,
utiles comme intermédiaires dans la synthèse des composés de formule (1).

Dans un mode particulier de réalisation de l'invention, les aldéhydes de formule (II) sont ceux dans lesquels l'atome de carbone asymétrique porteur du groupe R₃ est de configuration absolue (S).

L'invention a également pour objet les aldéhydes de formule (V) 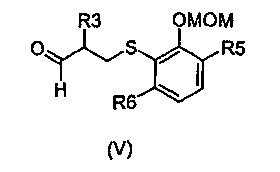 dans laquelle
R₃ représente
- un radical alkyle, ramifié ou non, renfermant de 1 à 3 atomes de carbone ou un radical méthoxy et
R₅ et R₆ identiques ou différents représentent :
- un atome d'hydrogène ;
- un radical alkyle, ramifié ou non, renfermant de 1 à 3 atomes de carbone ;
- un radical alcoxy ramifié ou non, renfermant de 1 à 3 atomes de carbone ;
- un radical alkylthio ramifié ou non renfermant de 1 à 3 atomes de carbone ;
- un radical alkylamino,
utiles comme intermédiaires dans la synthèse des composés de formule (1).

Dans un mode avantageux de réalisation de l'invention, les aldéhydes de formule (V) sont ceux dans lesquels l'atome de carbone asymétrique porteur du groupe R₃ est de configuration absolue (S).

L'invention a également pour objet le procédé de préparation des composés de formule (IIa) dans laquelle R₃ représente un groupe méthoxy et des composés de formule (Va) dans laquelle R₃ représente un groupe méthoxy, caractérisé en ce que la fonction alcool primaire de l'intermédiaire du type 3-arylthio-1,2-propanediol (XI) 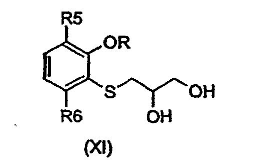 dans laquelle R représente un radical méthyle ou méthoxyméthyle (MOM) et R₅ et R₆ sont tels que définis dans la revendication 1 est protégée sous forme d'éther tritylique de formule (XII), 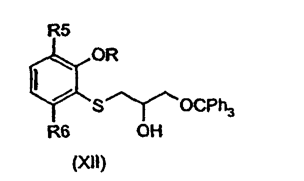 activée sous la forme d'un alcoolate alcalin, puis méthylée au moyen d'un halogénure ou du sulfate de méthyle pour donner le composé de formule (XIII) 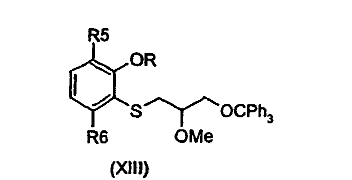 dans laquelle R est tel que défini précédemment et on libère la fonction alcool primaire par hydrolyse du groupement triphénylméthyle en milieu acide protique et on obtient,
- lorsque R est un radical méthyle, un composé de formule (XIVa1) 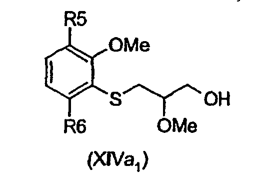 dont on oxyde l'alcool primaire et on obtient un composé de formule (IIa) 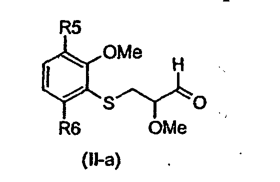
- lorsque R est un radical méthoxyméthyle, on obtient un composé de formule (XIVa2) 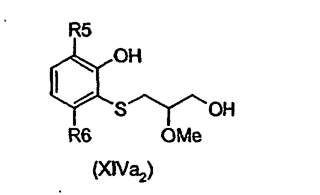
dont on protège la fonction phénol par alkylation chimio-sélective au moyen de chlorométhylméthyl éther et on obtient un composé de formule (XV) 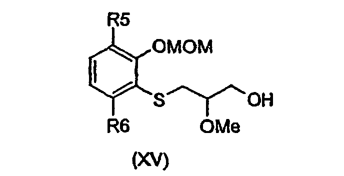 dont on oxyde l'alcool primaire et on obtient un composé de formule (Va)

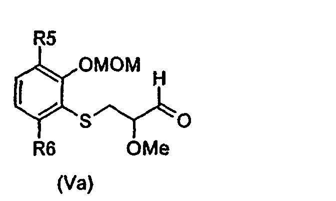

L'invention a également pour objet le procédé de préparation des composés de formule (II) et (V), notamment des composés de formule (IIb-g) et des composés de formule (Vb-j), dans lesquelles R₃ représente un radical alkyle caractérisé en ce que
- soit on réduit un composé de formule (XVI) 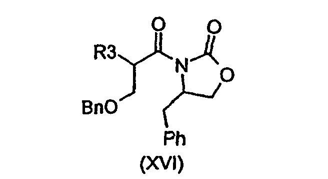 dans laquelle R₃ représente un radical alkyle, ramifié ou non, renfermant de 1 à 3 atomes de carbone,
et on obtient un alcool de formule (XVII) 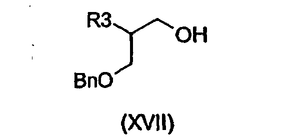 que l'on transforme en un ester de l'acide p-toluènesulfonique (tosylate) de formule (XVIII) 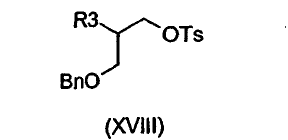 que l'on soumet à une hydrogénolyse en présence d'un catalyseur au palladium pour donner le composé (XIX) 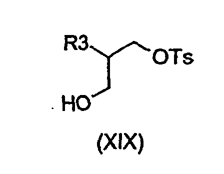 que l'on fait réagir avec un composé de formule (IV), éventuellement sous forme de sel alcalin, 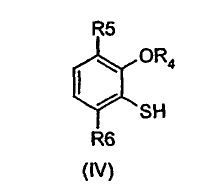 dans laquelle
R₄ représente :
- un atome d'hydrogène ou un radical méthyle et
R₅ et R₆ identiques ou différents représentent :
- un atome d'hydrogène ;
- un radical alkyle, ramifié ou non, renfermant de 1 à 3 atomes de carbone ;
- un radical alcoxy ramifié ou non, renfermant de 1 à 3 atomes de carbone ;
- un radical alkylthio ramifié ou non renfermant de 1 à 3 atomes de carbone ;
- un radical alkylamino,
pourvu que lorsque R₄ représente un radical méthyle alors R₅ représente un atome d'hydrogène, un radical alcoxy renfermant de 1 à 3 atomes de carbone, un radical alkylthio ramifié ou non renfermant de 1 à 3 atomes de carbone ou un radical alkylamino, ou
les groupes OR₄ et R₅ forment avec les carbones qui les portent un hétérocycle non aromatique à cinq ou six chaînons contenant au moins un atome d'oxygène, et R₆ est tel que défini précédemment,
- soit on fait réagir le 3-Bromo-2-méthyl-propan-1-ol avec un composé de formule (IV)
et on obtient un composé de formule (XX) 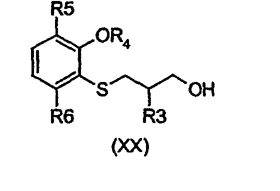 puis, lorsque R₄ est un radical alkyle ou forme avec le groupe R₅ adjacent un hétérocycle, on réalise directement une oxydation du composé de formule (XX) en aldéhyde de formule (IIb-g) 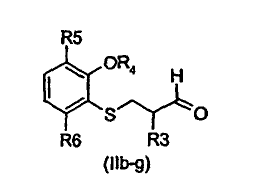 ou lorsque R₄ est un atome d'hydrogène, alors la fonction phénol du composé de formule (XX) est convertie en composé de formule (XXI) 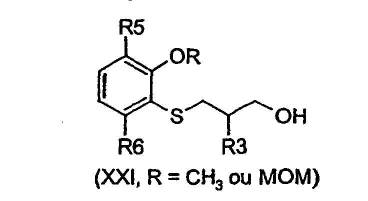 dans laquelle R représente un radical méthoxyméthyle (XXIa, R = MOM), ou un radical méthyle (XXIb, R = CH₃),
puis l'alcool (XXIa) est oxydé en aldéhyde (II) 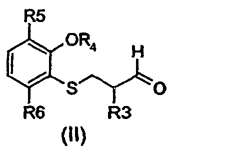 et l'alcool (XXIb) en aldéhyde (V)

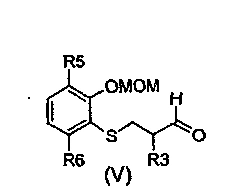

L'invention a aussi pour objet les compositions pharmaceutiques contenant à titre de principe actif au moins un des dérivés de formule générale (1) ou un de ses sels d'addition ou hydrates de ses sels d'addition associé à un ou plusieurs support pharmaceutique inerte ou autres véhicules pharmaceutiquement acceptables et éventuellement à un autre médicament.

Les compositions pharmaceutiques selon l'invention peuvent être, à titre d'exemple, des compositions administrables par voie orale, nasale, sublinguale, rectale ou parentérale. A titre d'exemple de compositions administrables par voie orale on peut citer les comprimés, les gélules, les granules, les poudres et les solutions ou suspensions orales.

Les formulations appropriées pour la forme d'administration choisie sont connues et décrites, par exemple dans: Remington, The Science and Practice of Pharmacy, 19^{ème} édition, 1995, Mack Publishing Company.

La dose efficace d'un composé de l'invention varie en fonction de nombreux paramètres tels que, par exemple, la voie d'administration choisie, le poids, l'âge, le sexe, la nature de la pathologie et la sensibilité de l'individu à traiter. En conséquence, la posologie optimale devra être déterminée, en fonction des paramètres jugés pertinents, par le spécialiste en la matière. Bien que les doses efficaces d'un composé de l'invention puissent varier dans de larges proportions, les doses journalières pourraient s'échelonner entre 0,01 mg et 100 mg par kg de poids corporel de l'individu à traiter. Une dose journalière d'un composé de l'invention comprise entre 0,10 mg et 50 mg par kg de poids corporel de l'individu à traiter étant, toutefois, préférée.

Les compositions pharmaceutiques selon l'invention sont utiles dans le traitement de l'angor stable, l'angor instable, l'insuffisance cardiaque, le syndrome du QT long d'origine congénitale, l'infarctus du myocarde et les troubles du rythme cardiaque.

Elles peuvent également être utiles dans le traitement de l'ischémie cérébrale, l'attaque ischémique transitoire, les neuropathies d'ordre traumatique ou ischémique et l'épilepsie, et dans celui des traitements des douleurs d'origine neuropathique et des maladies neurodégénératives.

### Exemples

Les exemples suivants illustrent l'invention mais ne la limitent en aucune façon:
- les exemples 1 à 4 illustrent la synthèse des intermédiaires I selon le schéma B,
- les exemples 5 et 6 illustrent la synthèse des intermédiaires IIa selon le schéma C,
- les exemples 7 à 15 illustrent la synthèse des intermédiaires Ilb-g selon le schéma D,
- les exemples 16 et 17 illustrent la synthèse des intermédiaires III du procédé b illustré dans le schéma A,
- les exemples 18 à 25 illustrent la synthèse des intermédiaires V selon la voie c illustrée dans le schéma D,
- les exemples 26 à 34 illustrent la synthèse des intermédiaires VIa-j selon le procédé c illustré dans le schéma A, et
- les exemples de référence 1 à 26 illustrent la synthèse des composés de formule (1) selon le schéma A.

Dans les exemples et les exemples de référence ci-après :
(i) l'avancement des réactions est suivi par chromatographie sur couche mince (CCM) et par conséquent les temps de réaction ne sont mentionnés qu'à titre indicatif;
(ü) des formes cristallines différentes peuvent donner des points de fusion différents, les points de fusion rapportés dans la présente demande sont ceux des produits préparés selon la méthode décrite et ne sont pas corrigés;
(iii) la structure des produits obtenus selon l'invention est confirmée par les spectres de résonance magnétique nucléaire (RMN), infrarouge (IR) et l'analyse centésimale, la pureté des produits finaux est vérifiée par CCM, la pureté énantiomérique des intermédiaires réactionnels et des produits finaux est déterminée par HPLC sur phase chirale;
(iv) les spectres RMN sont enregistrés dans le solvant indiqué. Les déplacements chimiques (δ) sont exprimés en partie par million (ppm) par rapport au tétraméthylsilane. La multiplicité des signaux est indiquée par: s, singulet; d, doublet; t, triplet; q, quadruplet; m, multiplet; I, large;
(v) les différents symboles des unités ont leur signification habituelle : µg (microgramme); mg (milligramme); g (gramme); ml (millilitre); mV (millivolt); °C (degré Celsius); mmole (millimole); nmole (nanomole); cm (centimètre); nm (nanomètre); min (minute); ms (milliseconde), Hz (hertz); [α] (pouvoir rotatoire spécifique mesuré à 589 nm, 25°C et à la concentration c; dans la présente invention la dimension deg cm² g⁻¹ est toujours sous entendue) ; les pressions sont données en millibars (mb);
(vi) les abréviations ont la signification suivante : F (point de fusion); Eb (point d'ébullition); AUC (aire sous la courbe);
(vii) par "température ambiante" on entend une température comprise entre 20°C et 25°C.

### Exemple 1 : 3-(R)-Amino-3,4-dihydro-2H-1,5-benzoxathiépine (Ia-1)

### • Etape 1: 2-(R)-tert-Butoxycarbonylamino-3-(2-hydroxy-phénylthio)-propan-1-ol (IXa-1)

Dans un ballon maintenu sous atmosphère inerte, on introduit 74,45 g (0,237 mole) de N-Boc-(2-hydroxyphényl)-L-cystéine (VIII) et 300 ml de tétrahydrofurane distillé. On refroidit le mélange à -10°C puis on additionne goutte à goutte 26 ml (0,236 mole) de N-méthyl-morpholine. Après 15 minutes d'agitation à -10°C. On introduit goutte à goutte 22,7 ml (0,237 mole) de chloroformate d'éthyle. On agite 30 minutes à -10°C puis on filtre à froid le précipité formé. Le filtrat est récupéré directement dans un ballon et refroidi à -10°C. On introduit alors 13,45 g (0,35 mole) de borohydrure de sodium en solution dans 50 ml d'eau, de telle manière que la température du mélange ne dépasse pas -10°C. En fin d'addition. Le mélange est réchauffé à température ambiante et agité pendant 12 heures. Le mélange est concentré sous pression réduite. Acidifié au moyen de 250 ml d'une solution aqueuse d'hydrogénosulfate de potassium (2N) et extrait au dichlorométhane. Les phases organiques combinées sont lavées à la saumure, séchées sur sulfate de sodium, filtrées et concentrées sous pression réduite. On obtient 61,41 g (0,205 mole) du composé du titre (IXa-1) sous forme d'une huile jaune, que l'on engage sans autre purification dans l'étape suivante.
Rendement brut : 86%
[α] = - 44,2 (c = 0,371, méthanol)
¹H RMN (DMSOd₆) δ: 1,38 (s, 9H); 2,80 (dd, 1H); 3,03 (dd, 1H); 3,35 (1s, 1H); 3,49 (m, 2H); 4,78 (m, 1H); 6,74 (m, 3H); 7,02 (m, 1H); 7,23 (m, 1H); 9,72 (1s, 1H).

### • Etape 2: 3-(R)-tert-Butoxycarbonylamino-3,4-dihydro-2H-1,5-benzoxathiépine (Xa-1)

Dans un ballon maintenu sous atmosphère inerte, on introduit (0,205 mole) de 2-tert-Butoxycarbonylamino-3-(2-hydroxy-phénylthio)-propan-1-ol (IXa-1), 300 ml de tétrahydrofurane distillé et 53,80 g (0,205 mole) de triphénylphosphine. Le mélange est refroidi à 0°C puis on additionne goutte à goutte 31,9 ml (0,205 mole) de diéthyl azodicarboxylate. Le mélange est agité à température ambiante pendant 24 heures. Le tétrahydrofurane est évaporé sous pression réduite puis le résidu repris dans l'éther éthylique. Le précipité formé est éliminé par filtration et le filtrat concentré sous pression réduite. Le résidu est purifié par chromatographie flash sur gel de silice (éluant: dichlorométhane/cyclohexane = 80:20). On récupère 48 g (0,170 mole) du composé du titre (Xa-1) sous la forme d'une huile rosée.
Rendement: 83%
[α] = + 15,2 (c = 0,493, méthanol)
¹H RMN (CDCl₃) δ: 1,47 (s, 9H); 2,93 (dd, 1H); 3,07 (dd, 1H); 3,95 (d, 1H); 4,27 (1s, 1H); 4,34 (d, 1H); 5,64 (1d, 1H); 7,00 (m, 2H); 7,18 (td, 1H); 7,41 (d, 1H)
HPLC (Chiracel OD, hexane/isopropanol (92:8), 0,5 ml/min): composé (Xa-1), temps de rétention = 12,71 min; composé (Xa-2), temps de rétention = 14,05 min; rapport des AUC (Xa-1) / (Xa-2) = 98:2.

### • Etape 3 : 3-(R)-Amino-3,4-dihydro-2H-1,5-benzoxathiépine (Ia-1)

Dans un ballon muni d'un réfrigérant, on introduit 48 g (0,170 mole) de 3-(R)-*tert*-Butoxycarbonylamino-3,4-dihydro-2H-1,5-benzoxathiépine (Xa-1) et 120 ml d'acide chlorhydrique (2N) dans l'éthanol. On porte le mélange à 80°C pendant 2 à 3 heures. Le mélange est refroidi, concentré sous pression réduite puis dilué avec de l'éther éthylique. Le précipité formé est filtré. Lavé à l'éther éthylique et essoré. On récupère ainsi 19 g (0,087 mole) de chlorhydrate du composé du titre (Ia-1) sous la forme d'un solide blanc.
Rendement : 50%
F : 235°C
[α] = + 48,9 (c = 0,350, méthanol)

| Analyse C₉H₁₂ClNOS : | | | |
|---|---|---|---|
| Calc.% | C 49,65 | H 5,56 | N 6,43 |
| Tr. | C 49,59 | H 5,63 | N 6,32 |

¹H RMN (DMSOd₆) δ: 3,12 (dd, 1H); 3,21 (dd, 1H); 3,81 (m, 1H); 4,21 (dd, 1H); 4,31 (dd, 1H); 7,09 (m, 2H); 7,28 (td, 1H); 7,45 (dd, 1H); 8,64 (1s, échangeables)
HPLC (Chiralpack AD, hexane/éthanol/diéthylamine (95 : 4,95 : 0,05), 1 ml/min) : composé (Ia-1), temps de rétention = 25,26 min; composé (Ia-2), temps de rétention = 23,48 min; rapport des AUC (Ia- 1) / (Ia-2) = 98:2.

### Exemple 2 : 3-(S)-amino-3,4-dihydro-2H-1,5-benzoxathiépine (Ia-2)

Le chlorhydrate du composé du titre (Ia-2) est préparé selon une séquence réactionnelle identique à celle mise en oeuvre pour la synthèse de la 3-(R)-amino-3,4-dihydro-2H-1,5-benzoxathiépine (Ia-1) mais utilisant, comme produit de départ. La N-Boc-(2-hydroxyphényl)-D-cystéine à la place de la N-Boc-(2-hydroxyphényl)-L-cystéine.
F : 210°C (sublimation)
[α] = - 44,8 (c = 0,402, méthanol)

| Analyse C₉H₁₂ClNOS : | | | |
|---|---|---|---|
| Calc.% | C 49,65 | H 5,56 | N 6,43 |
| Tr. | C 49,68 | H 5,57 | N 6,50 |

¹H RMN (DMSOd₆) δ : 3,12 (dd, 1H) ; 3,21 (dd, 1H) ; 3,80 (1s, 1H) ; 4,20 (d, 1H) ; 4,31 (dd, 1H) ; 7,09 (m, 2H) ; 7,28 (m, 1H) ; 7,45 (d, 1H) ; 8,63 (1s, échangeables).
HPLC (Chiralpack AD, hexaneléthanol/diéthylamine (95 : 4,95 : 0,05), 1 ml/min) : composé (Ia-2), temps de rétention = 23,07 min ; composé (Ia-1), temps de rétention = 24,99 min ; rapport des AUC (Ia-2) / (Ia-1) = 96 : 4.

### Exemple 3 : 3-(R)-amino-7-méthyl-3,4-dihydro-2H-1,5-benzoxathiépine (Ib)

Le composé du titre (Ib) est préparé selon une séquence réactionnelle identique à celle mise en oeuvre pour la synthèse de la 3-(R)-amino-3,4-dihydro-2H-1,5-benzoxathiépine (Ia-1) mais utilisant, comme produit de départ. Le 2-hydroxy-5-méthyl-thiophénol (IVb) à la place du 2-hydroxy-thiophénol. Le composé du titre (Ib) est obtenu sous la forme d'une huile jaune.
Rendement : 60%
[α] = + 46 (c = 0,106, méthanol)
¹H RMN (CDCl₃)δ: 1,65 (1s, H échangeables) ; 2,26 (s, 3H) ; 2,76 (dd, 1H) ; 3,15 (dd, 1H) ; 3,42 (m, 1H) ; 4,05 (m, 2H) ; 6,92 (m, 2H) ; 7,19 (d, 1H).

### Exemple 4 : 3-(R)-amino-6-méthyl-3,4-dihydro-2H-1,5-benzoxathiépine (Ic)

### • Etape 1 : 2-Hydroxy-6-méthyl-thiophénol (IVc)

Dans un ballon contenant 14 g de glace, 14 ml d'acide chlorhydrique à 36 % et 8,62 g (0,07 mole) de 2-amino-*m-*cresol on additionne goutte à goutte 5,3 g (0,077 mole) de nitrite de sodium en solution dans 12 ml d'eau. Le mélange, maintenu à 0°C, est ensuite versé lentement dans une solution de 15 g (0,093 mole) d'éthylxanthate de potassium dans 20 ml d'eau, maintenue à 40°C. Le bain chauffant est enlevé et le mélange agité pendant 3 heures puis extrait à l'éther éthylique. Les phases organiques combinées sont lavées à l'eau salée, séchées sur sulfate de magnésium, filtrées et concentrées sous pression réduite. Le résidu est purifié par une rapide chromatographie sur gel de silice (éluant: cyclohexane/dichlorométhane = 65:35). L'huile obtenue est reprise dans 20 ml d'éthanol et le mélange chauffé à 100°C. On additionne alors goutte à goutte 20 ml d'une solution éthanolique d'hydroxyde de potassium (7N). Après 4 heures à 100°C. Le mélange est refroidi, concentré sous pression réduite. Acidifié au moyen d'acide chlorhydrique (2N), puis extrait à l'éther éthylique. Les phases organiques combinées sont séchées sur sulfate de magnésium, filtrées et concentrées sous pression réduite pour donner une huile jaune qui n'est pas isolée mais engagée directement dans l'étape suivante.

### • Etape 2: 3-(R)-amino-6-méthyl-3,4-dihydro-2H-1,5-benzoxathiépine (Ic)

Le chlorhydrate du composé du titre (Ic) est préparé selon une séquence réactionnelle similaire à celle mise en oeuvre pour la synthèse de la 3-(R)-amino-3,4-dihydro-2H-1,5-benzoxathiépine (Ia-1) mais utilisant, comme produit de départ. Le 2-Hydroxy-6-méthyl-thiophénol (IVc) à la place du 2-hydroxy-thiophénol.
F : > 250 °C
[α] = + 101,4 (c = 0,313, méthanol)

| Analyse C₁₀H₁₄ClNOS : | | | |
|---|---|---|---|
| Calc.% | C 51,83 | H 6,09 | N 6,04 |
| Tr. | C 51,64 | H 6,12 | N 5,89 |

¹H RMN (DMSOd₆)δ: 2,37 (s, 3H) ; 3,16 (m, 2H) ; 3,79 (m, 1H) ; 4,20 (1d, 1H) ; 4,30 (dd, 1H) ; 6,94 (d, 1H); 7,03 (d, 1H); 7,14 (m, 1H); 8,59 (1s, 3H échangeables).

### Exemple 5 : 2-(S)-méthoxy-3-(2-méthoxy-phénylthio)-propionaldéhyde (IIa-1)

### • Etape 1: 1-(3-Triphénylméthyloxy-2-(S)-hydroxy-propylthio)-2-méthoxy-benzène (XII-1)

Dans un ballon maintenu sous atmosphère inerte, on introduit 19,3 g (0,09 mole) de 1-(3-hydroxy-2-(S)-hydroxy-propylthio)-2-méthoxy-benzène (XI-1), 150 ml d'acétonitrile, 11 ml (0,136 mole) de pyridine et 27,5 g (0,098 mole) de chlorure de triphénylméthyle. La solution est agitée à température ambiante pendant 5 heures. Le mélange est concentré sous pression réduite et la pyridine résiduelle entraînée par distillation azéotropique avec du toluène. Le résidu est repris dans l'eau puis extrait au dichlorométhane. Les phases organiques combinées sont séchées sur sulfate de sodium, filtrées, évaporées et le résidu purifié par chromatographie flash sur gel de silice (éluant: cyclohexane/dichlorométhane = 30:70). On récupère 34,8 g (0,076 mole) du composé du titre (XII-1) sous la forme d'une huile jaune.
Rendement : 85%
[α] = - 7,1 (c = 0,225, méthanol)
¹H RMN (CDCl₃) δ: 2,80 (d, 1H) ; 2,93 (dd, 1H) ; 3,14 (dd, 1H) ; 3,23 (d, 2H) ; 3,77 (m, 1H) ; 3,87 (s, 3H) ; 6,88 (m, 2H) ; 7,25 (m, 10H) ; 7,34 (dd, 1H) ; 7,41 (d, 6H).

### • Etape 2: 1-(3-Triphénylméthyloxy-2-(S)-méthoxy-propylthio)-2-méthoxy-benzène (XDIII-1)

Dans un ballon maintenu sous atmosphère inerte, contenant une suspension de 3,5 g d'hydrure de sodium (0,087 mole) dans 30 ml de tétrahydrofurane distillé, on introduit goutte à goutte 34,8 g (0,076 mole) de 1-(3-Triphénylméthyloxy-2-(S)-hydroxy-propylthio)-2-méthoxy-benzène (XII-1) en solution dans 50 ml de tétrahydrofurane distillé. Le mélange est agité à température ambiante pendant 3 heures puis on additionne 5,1 ml (0,082 mole) d'iodure de méthyle. Après 2 heures d'agitation à température ambiante. Le mélange est concentré sous pression réduite puis repris dans du dichlorométhane. La solution obtenue est refroidie puis diluée à l'eau glacée. Les phases sont séparées et la phase aqueuse extraite au dichlorométhane. Les phases organiques combinées sont lavées à l'eau, à la saumure, séchées sur du sulfate de magnésium, filtrées, concentrées sous pression réduite. Le résidu est purifié par chromatographie flash sur gel de silice (éluant: dichlorométhane/cyclohexane = 70:30), pour donner 35,8 g (0,076 mole) du composé du titre (XIII-1) sous la forme d'une huile.
Rendement : 100%
¹H RMN (CDCl₃) δ: 3,01 (dd, 1H) ; 3,16 (dd, 1H) ; 3,25 (dd, 2H) ; 3,36 (s, 3H) ; 3,43 (m, 1H) ; 3,85 (s, 3H) ; 6,86 (m, 2H) ; 7,41 (m, 10H), 7,35 (m, 1H) ; 7,43 (d, 6H).

### • Etape 3 : 2-(S)-Méthoxy-3-(2-méthoxy-phénylthio)-propan-1-ol (XIV-1)

Dans un ballon maintenu sous atmosphère inerte, on introduit 35,8 g (0,076 mole) de 1-(3-triphénylméthyloxy-2-(S)-méthoxy-propylthio)-2-methoxy-benzène (XIII-1) et 150 ml d'une solution d'acide chlorhydrique (2,5N) dans l'éthanol. Le mélange est agité à température ambiante pendant 4 heures. Le précipité blanc formé est éliminé par filtration et le filtrat concentré sous pression réduite. Le résidu est purifié par chromatographie flash sur gel de silice (éluant: dichlorométhane/acétate d'éthyle = 90:10). On récupère 13,4 g (0,058 mole) du composé du titre (XIV-1) sous la forme d'une huile orange.
Rendement : 77%
[α] = -15,5 (c = 0,078, méthanol)
¹H RMN (CDCl₃)δ: 1,99 (t, 1H); 2,97 (dd, 1H) ; 3,15 (dd, 1H) ; 3,42 (s, 3H) ; 3,44 (m, 1H) ; 3,65 (m, 1H) ; 3,83 (m, 1H) ; 3,90 (s, 3H) ; 6,87 (d, 1H) ; 6,93 (t, 1H) ; 7,23 (td, 1H) ; 7,35 (dd, 1H).

### • Etape 4 : 2-(S)-méthoxy-3-(2-méthoxy-phénylthio)-propionaldéhyde (IIa-1)

Dans un ballon maintenu sous atmosphère inerte, on introduit 4 g (0,017 mole) 2-(S)-méthoxy-3-(2-méthoxy-phénylthio)-propan-1-ol (XIV-1), 100 ml de dichlorométhane et 11,13 g (0,026 mole) de réactif de Dess-Martin. Le mélange est agité à température ambiante pendant 3 heures. On ajoute ensuite 190 ml d'une solution aqueuse saturée de thiosulfate de sodium puis 190 ml d'une solution aqueuse saturée d'hydrogénocarbonate de sodium et le mélange est extrait au dichlorométhane. Les phases organiques combinées sont lavées à l'eau, à la saumure, séchées sur du sulfate de magnésium, filtrées et concentrées sous pression réduite. Le résidu est purifié par chromatographie flash sur gel de silice (éluant : dichlorométhane/acétate d'éthyle = 95: 5). On récupère 1,2 g (0,005 mole) du composé du titre (IIa-1) sous la forme d'une huile jaune.
Rendement : 31%
¹H RMN (CDCl₃) δ: 3,13 (dd, 1H) ; 3,23 (dd, 1H) ; 3,47 (s, 3H); 3,70 (m, 1H); 3,90 (s, 3H) ; 6,88 (dd, 1H) ; 6,92 (td, 1H) ; 7,26 (td, 1H) ; 7,38 (dd, 1H) ; 9,68 (d, 1H).

### Exemple 6 : 2-(R)-méthoxy-3-(2-méthoxy-phénylthio)-propionaldéhyde (IIa-2)

En procédant comme dans l'exemple 5 mais en remplaçant, dans l'étape 1. Le 1-(3-hydroxy-2-(S)-hydroxy-propylthio)-2-méthoxy-benzène (XI-1) par le 1-(3-hydroxy-2-(R)-hydroxy-propylthio)-2-méthoxy-benzène (XI-2), on obtient le composé du titre (IIa-2).
Rendement : 90%
¹H RMN (CDCl₃) δ: 3,12 (dd, 1H) ; 3,23 (dd, 1H); 3,47 (s, 3H) ; 3,70 (m, 1H) ; 3,90 (s, 3H) ; 6,89 (m, 2H) ; 7,26 (m, 1H) ; 7,38 (dd, 1H) ; 9,67 (d, 1H).

### Exemple 7 : 2-(S)-méthyl-3-(2-méthoxy-phénylthio)-propionaldéhyde (IIb-1)

### • Etape 1 : 2-(S)-Méthyl-3-(2-méthoxy-phénylthio)-propan-1-ol (XXb-1)

Dans un ballon maintenu sous atmosphère inerte, on introduit 2,2 ml (0,021 mole) de 3-bromo-2-(S)-méthyl-1-propanol. On ajoute goutte à goutte 20 ml d'une solution aqueuse de soude (1N) puis 2,3 ml (0,019 mole) de 2-méthoxy-thiophénol. Le mélange est porté à 90°C pendant 4 heures puis refroidi à température ambiante. On ajoute alors 50 ml d'eau et le mélange est extrait au dichlorométhane. Les phases organiques combinées sont lavées à la saumure, séchées sur sulfate de sodium, filtrées et concentrées sous pression réduite. Le résidu est purifié par chromatographie flash sur gel de silice (éluant: dichlorométhane/méthanol = 99:1). On récupère 4 g (0,019 mole) du composé du titre (XXb-1) sous la forme d'une huile incolore.
Rendement : 100%
[α] = + 22,2 (c = 0,982, méthanol)
¹H RMN (DMSOd₆) δ: 0,95 (d, 3H) ; 1,74 (m, 1H) ; 2,61 (dd, 1H) ; 3,01 (dd, 1H) ; 3,34 (m, 2H) ; 3,80 (s, 3H) ; 4,61 (t, 1H échangeable) ; 6,94 (m, 2H) ; 7,14 (td, 1H) ; 7,23 (dd, 1H).

### • Etape 2 : 2-(S)-méthyl-3-(2-méthoxy-phénylthio)-propionaldéhyde (IIb-1)

Dans un ballon maintenu sous atmosphère inerte, on introduit 0,61 ml (0,007 mole) de chlorure d'oxalyle et 20 ml de dichlorométhane. Le mélange est refroidi à -78°C puis on introduit 1 ml (0,014 mole) de diméthylsulfoxide. Après 15 minutes d'agitation à -78°C. On ajoute goutte à goutte 1,5 g (0,007 mole) de 2-(S)-méthyl-3-(2-méthoxy-phénylthio)-propan-1-ol (XXb-1) en solution dans 15 ml de dichlorométhane. Le mélange est agité 1 heure à -78°C puis on additionne 2 ml (0,014 mole) de triéthylamine. Après 15 minutes à -78°C. Le mélange est réchauffé à -10°C et agité pendant 45 minutes à cette température. A ce stade. L'aldéhyde n'est pas isolé mais engagé *in situ* dans la réaction suivante d'amination réductrice.

### Exemple 8 : 2-(R)-méthyl-3-(2-méthoxy-phénylthio)-propionaldéhyde (IIb-2)

En procédant comme dans l'exemple 7 mais en utilisant, dans l'étape 1. Le 3-bromo-2-(R)-méthyl-1-propanol à la place du 3-Bromo-2-(S)-méthyl-1-propanol, on obtient le composé du titre (IIb-2) qui, comme l'aldéhyde (IIb-1), n'est pas isolé mais engagé *in situ* dans la réaction suivante d'amination réductrice.

### Exemple 9 : 2-(S)-méthyl-3-(2,3-dihydro-benzofuran-7-thio)-propionaldéhyde (IIc)

### • Etape 1 : 2,3-Dihydro-benzofuran-7-thiol (IVd)

Dans un ballon maintenu sous atmosphère inerte, on introduit 3 ml (0,026 mole) de 2,3-dihydrobenzofurane et 50 ml d'éther éthylique. Le mélange est refroidi à 0°C puis on ajoute goutte à goutte 11,5 ml (0,029 mole) d'une solution de n-butyl-lithium (2,5M) dans l'hexane. En fin d'addition. Le mélange est porté à reflux pendant 24 heures puis refroidi à 0°C avant d'ajouter, par fractions, 0,92 g (0,029 mole) de soufre sublimé. Le mélange est chauffé au reflux pendant 2 heures puis à nouveau refroidi à 0°C avant d'ajouter 6 ml d'acide chlorhydrique (10N). Les phases sont séparées et la phase aqueuse extraite à l'éther éthylique. Les phases organiques combinées sont lavées par une solution aqueuse d'acide chlorhydrique (1N), à l'eau puis extraites au moyen d'une solution aqueuse de soude (1N). Les phases alcalines combinées sont lavées à l'éther éthylique. Acidifiées et extraites à l'éther éthylique. Les phases éthérées combinées sont séchées sur sulfate de sodium, filtrées et concentrées sous pression réduite. Le résidu est purifié par chromatographie flash sur gel de silice (éluant: cyclohexane/éther = 90:10) pour donner 0,4 g (0,0026 mole) du composé du titre (IVd).
Rendement : 10%
¹H RMN (DMSOd₆) δ: 3,20 (t, 2H) ; 4,55 (m, 2H) ; 4,79 (1s, 1H échangeable) ; 6,72 (m, 1H) ; 7,01 (m, 2H).

### • Etape 2: 2-(S)-méthyl-3-(2,3-dihydro-benzofuran-7-thio)-propionaldéhyde (IIc)

En procédant comme dans l'exemple 7 mais en utilisant, dans l'étape 1. Le 2,3-dihydro-benzofuran-7-thiol (IVd) à la place du 2-méthoxy-thiophénol, on obtient le composé du titre (IIc) qui, comme l'aldéhyde (IIb-1), n'est pas isolé mais engagé *in situ* dans la réaction suivante d'amination réductrice.

### Exemple 10 : 2-(S)éthyl-3-(2-méthoxy-phénylthio)-propionaldéhyde (IId-1)

### • Etape 1 : 2-(R)-Éthyl-3-benzyloxy-propan-1-ol (XVIId-1)

Dans un ballon maintenu sous atmosphère inerte, on introduit 3,08 g (0,0084 mole) de 4-(R)-benzyl-3-(2-(R)-benzyloxyméthyl-butyryl)-oxazolidin-2-one (XVId-1), 70 ml d'éther éthylique et 0,17 ml (0,0092 mole) d'eau. Le mélange est refroidi à 0°C puis on additionne goutte à goutte 4,6 ml (0,0092 mole) de borohydrure de lithium (2N) dans le tétrahydrofurane. Le mélange est agité 1 heure à 0°C puis on introduit une solution aqueuse d'hydroxyde de sodium (1N) en quantité suffisante pour que les phases deviennent limpides. Les phases sont séparées et la phase aqueuse extraite à l'éther éthylique. Les phases organiques combinées sont lavées à l'eau, à la saumure, séchées sur sulfate de sodium, filtrées, concentrées sous pression réduite. Le résidu est purifié par chromatographie flash sur gel de silice (éluant : cyclohexane/acétate d'éthyle = 70 : 30). On obtient 1,34 g (0,0069 mole) du composé du titre (XVITd-1) sous la forme d'une huile incolore.
Rendement : 82%
[α] = + 21 (c = 0,634, CDCl₃)
¹H RMN (CDCl₃) δ 0,92 (t, 3H) ; 1,31 (m, 2H) ; 1,80 (m, 1H) ; 2,59 (m, 1H) ; 3,48 (t, 1H) ; 3,63 (m, 2H) ; 3,73 (m, 1H) ; 4,52 (s, 1H) ; 4,53 (s, 1H) ; 7,32 (m, 5H).

### • Etape 2 : 2-(S)-éthyl-3-benzyloxy-propyl p-toluènesulfonate (XVIIId-1)

Dans un ballon maintenu sous atmosphère inerte, on introduit 1,34 g (0,0069 mole) de 2-(R)-éthyl-3-benzyloxy-propan-1-ol (XVIId-1), 12 ml de dichlorométhane, 1,31 g (0,0069 mole) de chlorure de tosyle et 0,084 g (0,0007 mole) de 4-diméthylaminopyridine. Le mélange est refroidi à 0°C puis on ajoute goutte à goutte 0,89 ml (0,011 mole) de pyridine. Après une nuit au réfrigérateur le mélange est hydrolysé au moyen d'une solution aqueuse à 10% d'acide citrique. Les phases sont séparées et la phase aqueuse extraite à l'éther éthylique. Les phases organiques combinées sont lavées à l'eau, à la saumure, séchées sur du sulfate de magnésium, filtrées et concentrées sous pression réduite. Le résidu est purifié par chromatographie flash sur gel de silice (éluant: dichlorométhane/cyclohexane = 750:25). On obtient 1,54 g (0,0044 mole) du composé du titre (XVIIId-1).
Rendement : 64%
[α] = + 4 (c = 0,326, CDCl₃)
¹H RMN (CDCl₃) δ: 0,85 (t, 3H) ; 1,37 (m, 2H) ; 1,84 (m, 1H) ; 2,41 (s, 3H) ; 3,38 (m, 2H) ; 4,07 (s, 1H) ; 4,08 (s, 1H) ; 4,38 (s, 2H) ; 7,28 (m, 7H) ; 7,78 (d, 2H).

### • Etape 3 : 2-(S)-éthyl-3-hydroxy-propyl p-toluènesulfonate (XIXd-1)

Dans un ballon de 100 ml, on introduit 1,5 g (0,0043 mole) de 2-(S)-éthyl-3-benzyloxy-propyl p-toluènesulfonate (XVIIId-1), 12 ml d'éthanol et 0,29 g de palladium hydroxyde à 20%. Le mélange est agité vigoureusement à température ambiante sous une faible pression d'hydrogène. Après une heure de réaction. Le mélange est filtré sur celite et le solide lavé à l'éthanol. Le filtrat est concentré sous pression réduite et le résidu purifié par chromatographie flash sur gel de silice (éluant: dichlorométhane/cyclohexane = 80:20). On récupère 1,02 g (0,0039 mole) du composé du titre (XIXd-1) sous la forme d'une huile incolore.
Rendement : 92%
[α] = - 6,2 (c = 0,423, CDCl₃)
¹H RMN (CDCl₃) δ: 0,88 (t, 3H) ; 1,33 (m, 2H) ; 1,52 (t, 1H) ; 1,74 (m, 1H) ; 2,45 (s, 3H) ; 3,57 (m, 1H) ; 3,65 (m, 1H) ; 4,05 (dd, 1H) ; 4,12 (dd, 1H) ; 7,35 (d, 2H) ; 7,80 (d, 2H).

### • Etape 4 : 2-(S)-éthyl-3-(2-méthoxy-phénylthio)-propan-1-ol (XXd-1)

Dans un ballon maintenu sous atmosphère inerte, contenant 0,19 g (0,0047 mole) d'hydrure de sodium en suspension dans 10 ml de diméthylformamide refroidi à 0°C, on ajoute goutte à goutte 0,47 ml (0,0038 mole) de 2-méthoxy-thiophénol en solution dans 5 ml de diméthylformamide. Le mélange est réchauffé à température ambiante et agité pendant 1 heure puis on additionne 0,99 g (0,0038 mole) de 2-(S)-Éthyl-3-hydroxy-propyl p-toluènesulfonate (XIXd-1) en solution dans 10 ml de diméthylformamide. Après 2 heures d'agitation à température ambiante. Le diméthylformamide est évaporé sous vide poussé et le résidu repris dans du dichlorométhane. La phase organique est lavée à l'eau, à la saumure, séchée sur sulfate de sodium, filtrée et concentrée sous pression réduite. Le résidu est purifié par chromatographie flash sur gel de silice (éluant : dichlorométhane/méthanol = 98 : 2). On récupère 0,87 g (0,0038 mole) du composé du titre (XXd-1) sous forme d'huile.
Rendement : 100%
¹H RMN (CDCl₃) δ: 0,94 (t, 3H) ; 1,49 (m, 2H) ; 1,74 (m, 1H) ; 1,80 (m, 1H) ; 2,98 (m, 2H) ; 3,68 (m, 1H) ; 3,76 (m, 1H) ; 3,90 (s, 3H) ; 6,86 (d, 1H) ; 6,93 (td, 1H) ; 7,19 (td, 1H) ; 7,32 (dd, 1H).
HPLC (Chiracel OD, hexane/isepropanol (90: 10), 1 ml/min) : composé (XXd-1), temps de rétention = 11,44 min; composé (XXd-2), temps de rétention = 13,23 min ; rapport des AUC (XXd-1) / (XXd-2) = 99,9: 0,1.

### • Etape 5 : 2-(S)-éthyl-3-(2-méthoxy-phénylthio)-propionaldéhyde (IId-1)

En procédant comme dans l'exemple 7 mais en remplaçant, dans l'étape 2. Le 2-(S)-méthyl-3-(2-méthoxy-phénylthio)-propan-1-ol (XXb-1) par le 2-(S)-éthyl-3-(2-méthoxy-phénylthio)-propan-1-ol (XXd-1), on obtient le composé du titre (IId-1) qui, comme l'aldéhyde (IIb-1), n'est pas isolé mais engagé *in situ* dans la réaction suivante d'amination réductrice.

### Exemple 11 : 2-(R)-Éthyl-3-(2-méthoxy-phénylthio)-propionaldéhyde (IId-2)

En procédant comme dans l'exemple 10 mais en remplaçant, dans l'étape 1. Le 4-(R)-benzyl-3-(2-(R)-benzyloxyméthyl-butyryl)-oxazolidin-2-one (XVId-1) par le 4-(S)-benzyl-3-(2-(S)-benzyloxyméthyl-butyryl)-oxazolidin-2-one (XVId-2), on obtient le composé du titre (IId-2) qui, comme l'aldéhyde (IId-1), n'est pas isolé mais engagé *in situ* dans la réaction suivante d'amination réductrice.

### Exemple 12: 2-(S)-n-propyl-3-(2-méthoxy-phénylthio)-propionaldéhyde (IIe-1)

En procédant comme dans l'exemple 10 mais en utilisant, dans l'étape 1. Le 4-(R)-benzyl-3-(2-(R)-benzyloxyméthyl-pentanoyl)-oxazolidin-2-one (XVIe-1) à la place du 4-(R)-benzyl-3-(2-(R)-benzyloxyméthyl-butyryl)-oxazolidin-2-one (XVId-1), on obtient le composé du titre (IIe-1) qui, comme l'aldéhyde (IId-1), n'est pas isolé mais engagé *in situ* dans la réaction suivante d'amination réductrice.

### Exemple 13 : 2-(R)-n-propyl-3-(2-méthoxy-phénylthio)-propionaldéhyde (IIe-2)

En procédant comme dans l'exemple 10 mais en utilisant, dans l'étape 1 le 4-(S)-Benzyl-3-(2-(S)-benzyloxyméthyl-pentanoyl)-oxazolidin-2-one (XVIe-2) à la place du 4-(R)-Benzyl-3-(2-(R)-benzyloxyméthyl-butyryl)-oxazolidin-2-one (XVId-1) on obtient le composé du titre (IIe-2) qui, comme l'aldéhyde (IId-1), n'est pas isolé mais engagé *in situ* dans la réaction suivante d'amination réductrice.

### Exemple 14 : 2-(S)-iso-Propyl-3-(2-méthoxy-phénylthio)-propionaldéhyde (IIf)

En procédant comme dans l'exemple 10 mais en utilisant, dans l'étape 1 le 4-(R)-Benzyl-3-(2-(R) benzyloxyméthyl-3-méthyl-butyryl)-oxazolidin-2-one (XVIf) à la place du 4-(R)-Benzyl-3-(2-(R)-benzyloxyméthyl-butyryl)-oxazolidin-2-one (XVId-1), on obtient le composé du titre (IIf) qui, comme l'aldéhyde (IId-1), n'est pas isolé mais engagé *in situ* dans la réaction suivante d'amination réductrice.

### Exemple 15 :2-(S)-Méthyl-3-(2,3-diméthoxy-phényIthio)-propionaldéhyde (IIg)

### • Etape 1 : 2-Mercapto-6-méthoxy-phénol (IVg)

Le composé (IVg) est préparé à partir du gaïacol, selon la méthode rapportée par Tanabe (Heterocycles 1999, 50(2), 681). Le brut réactionnel est repris dans un mélange tétrahydrofurane/eau (1 : 1) et traité par 1 équivalent de triphénylphosphine à 60°C pendant 2 à 3 heures puis avec une solution aqueuse de soude (1N). Le mélange est lavé au pentane puis au dichlorométhane. La phase aqueuse est acidifiée avec une solution aqueuse d'acide chlorhydrique (1N) et extraite à l'acétate d'éthyle. La phase acétate d'éthyle est séchée sur sulfate de sodium, filtrée, concentrée sous pression réduite pour donner une huile jaune qui est utilisée sans autre purification dans létape suivante.
¹H RMN (DMSOd₆) δ: 3,77 (s, 3H) ; 4,58 (s, 1H) ; 6,67 (m, 1H) ; 6,74 (dd, 1H) ; 6,81 (dd, 1H) ; 9,12 (s, 1H).

### • Etape 2 : 2-(S)-Méthyl-3-(2-hydroxy-3-méthoxy-phénylthio)-propan-1-ol (XXg)

Dans un ballon maintenu sous atmosphère inerte, contenant 0,13 g (0,0032 mole) d'hydrure de sodium et 5 ml de diméthylformamide, on introduit goutte à goutte 0,57 g de 2-Mercapto-6-méthoxy-phénol (IVg) en solution dans 5 ml de diméthylfoimamide. Après 30 minutes, on additionne 0,34 ml (0,0033 mole) de Bromo-2-(S)-méthyl-1-propanol et le mélange est agité pendant 5 heures à température ambiante. Le mélange est concentré sous pression réduite; repris par une solution aqueuse d'acide chlorhydrique (1N) et extrait au dichlorométhane. Les phases organiques combinées sont séchées sur sulfate de sodium, filtrées, concentrées et le résidu purifié par chromatographie flash sur gel de silice (éluant : dichlorométhane). On récupère 0,615 g (0,0027 mole) du composé du titre (XXg) sous la forme d'une huile jaune.
Rendement : 84%
¹H RMN (DMSOd₆) δ: 0,93 (d, 3H) ; 1,69 (m, 1H) ; 2,58 (dd, 1H); 2,97 (dd, 1H) ; 3,32 (m, 2H) ; 3,78 (s, 3H) ; 4,58 (1s, 1H) ; 6,77 (m, 3H) ; 8,87 (1s, 1H).

### • Etape 3 : 2-(S)-Méthyl-3-(2,3-diméthoxy-phénylthio)-propan-1-ol (XXIb)

Dans un ballon maintenu sous atmosphère inerte, on introduit 0,43 g (0,0019 mole) de 2-(S)-Méthyl-3-(2-hydroxy-3-méthoxy-phénylthio)-propan-1-ol (XXg), 10 ml d'acétone et 0,26 g (0,0019 mole) de carbonate de potassium. Après 15 minutes, on introduit 0,12 ml (0,0019 mole) d'iodure de méthyle et le mélange est chauffé à 60°C pendant 8 heures. Le mélange est concentré sous pression réduite. Le résidu est repris dans l'eau et la phase aqueuse extraite au dichlorométhane. Les phases organiques combinées sont séchées sur sulfate de magnésium, filtrées et concentrées. Le résidu obtenu est utilisé sans autre purification dans l'étape suivante.
¹H RMN (DMSOd₆) δ: 0,95 (d, 3H) ; 1,75 (m, 1H) ; 2,62 (dd, 1H) ; 3,02 (dd, 1H) ; 3,37 (m, 2H) ; 3,70 (s, 3H) ; 3,79 (s, 3H) ; 4,63 (t, 1H) ; 6,85 (m, 2H) ; 7,04 (m, 1H).

### • Etape 4 : 2-(S)-Méthyl-3-(2,3-diméthoxy-phénylthio)-propionaldéhyde (IIg)

En procédant comme dans l'exemple 7 mais en utilisant, dans l'étape 2. Le 2-(S)-Méthyl-3-(2,3-diméthoxy-phénylthio)-propan-1-ol (XXIb) à la place du 2-(S)-Méthyl-3-(2-méthoxy phénylthio)-propan-1-ol (XXb-1), on obtient le composé du titre (IIg) qui, comme l'aldéhyde (IIb-1), n'est pas isolé mais engagé *in situ* dans la réaction suivante d'amination réductrice.

### Exemple 16: 3-(R)-([(S)-3,4-Epoxypropyl]amino)-3,4-dihydro-2H-1,5-benzoxathiépine (III-1)

Dans un ballon maintenu sous atmosphère inerte, on introduit 0,85 g (0,0047 mole) de 3-(R)-amino-3,4-dihydro-2H-1,5-benzoxathiépine (Ia-1), 15 ml de propanol-2 et 0,41 ml (0,0052 mole) d'Epichlorhydrine-(S). Le mélange est porté à 60°C pendant 12 heures puis refroidi à température ambiante. On ajoute alors 0,37 g (0,0066 mole) de potassium hydroxyde broyé et après 3 heures d'agitation à température ambiante le solvant est évaporé sous pression réduite. Le résidu est repris dans du dichlorométhane. La solution obtenue est lavée à l'eau, à la saumure, séchée sur sulfate de magnésium, filtrée et concentrée sous pression réduite. Le résidu est purifié par chromatographie flash sur gel de silice (éluant : dichlorométhane/méthanol = 98 : 2). On obtient 0,69 g (0,0029 mole) du composé du titre (III-1).
Rendement : 62%
[α] = + 22,1 (c = 0,227, méthanol)
¹H RMN (DMSOd₆) δ: 2,17 (1s, 1H) ; 2,56 (dd, 1H) ; 2,65 (m, 2H) ; 2,84 (m, 2H) ; 3,00 (m, 1H) ; 3,11 (m, 1H) ; 3,17 (1s, 1H) ; 3,93 (dd, 1H) ; 4,16 (dd, 1H) ; 6,98 (m, 2H) ; 7,18 (td, 1H) ; 7,34 (d, 1H)
HPLC (Chiralpack AD, hexane/éthanol (90: 10), 1 ml/min) : composé (III-1), temps de rétention = 24,04 min ; composé (III-2), temps de rétention = 29,81 min ; rapport des AUC, (III-1) / (III-2) = 97 : 3.

### Exemple 17 : 3-(R)-([(R)-3,4-Epoxypropyl]amino)-3,4-dihydro-2H-1,5-benzoxathiépine (III-2)

En procédant comme dans l'exemple 16 mais en remplaçant l'Epichlorhydrine-(S) par l'Epichlorhydrine-(R), on obtient le composé du titre (III-2) sous la forme d'une huile blanche,
Rendement : 55%
[α] = + 56 (c = 0,256, méthanol)
¹H RMN (CDCl₃) δ: 2,08 (1s, 1H) ; 2,65 (m, 1H) ; 2,75 (dd, 1H) ; 2,80 (m, 1H) ; 2,97 (dd, 1H) ; 3,05 (dd, 1H) ; 3,12 (m, 2H) ; 3,28 (m, 1H) ; 4,09 (dd, 1H) ; 4,29 (dd, 1H) ; 6,96 (m, 2H) ; 7,14 (td, 1H) ; 7,35 (dd, 1H).

### Exemple 18 : 2-(S)-Méthyl-3-(2-méthoxyméthoxy-phénylthio)-propionaldéhyde (Vb)

### • Etape 1 : 2-(S)-Méthyl-3-(2-hydroxy-phénylthio)-propan-1-ol (XXh)

En procédant comme dans l'exemple 7 mais en remplaçant, dans l'étape 1. Le 2-Méthoxy-thiophénol par le 2-Hydroxy-thiophénol, on obtient le composé du titre (XXh),
Rendement: 100%
¹H RMN (DMSOd₆) δ: 0,94 (d,3H) ; 1,71 (m, 1H) ; 2,58 (dd, 1H) ; 2,98 (dd, 1H) ; 3,34 (m, 2H); 4,59 (1s, 1H échangeable) ; 6,77 (m, 2H) ; 7,00 (m, 1H) ; 7,18 (m, 1H) ; 9,68 (1s, 1H échangeable).

### • Etape 2 : 2-(S)-Méthyl-3-(2-méthoxyméthoxy-phénylthio)-propan-1-ol (XXIa)

Dans un ballon de 100 ml, on introduit 2,47 g (0,012 mole) de 2-(S)-Méthyl-3-(2-hydroxy-phénylthio)-propan-1-ol (XXh), 25 ml de dichlorométhane, 12,5 ml (0,024 mole) d'une solution aqueuse de soude (2N), 0,55 ml (0,0012 mole) d'aliquat 336 et 0,9 ml (0,012 mole) de chlorométhylméthyl éther. Le mélange est agité à température ambiante pendant 24 heures puis les phases sont séparées. La phase organique est lavée successivement par une solution aqueuse d'acide chlorhydrique (1N), une solution aqueuse d'hydroxyde de sodium (1N), de l'eau, de la saumure puis séchée sur sulfate de sodium, filtrée et concentrée sous pression réduite. Le résidu est purifié par chromatographie flash sur gel de silice (éluant: dichlorométhane/acétone = 96:4). On récupère 1,1 g (0,0045 mole) du composé du titre (XXIa) sous forme d'une huile blanche.
Rendement: 38%
¹H RMN (DMSOd₆) δ: 0,96 (d, 3H) ; 1,74 (m, 1H) ; 2,63 (dd, 1H) ; 3,04 (dd, 1H) ; 3,36 (t, 2H) ; 3,40 (s, 3H) ; 4,62 (t, 1H échangeable) ; 5,23 (s, 2H) ; 6,98 (td, 1H) ; 7,05 (dd, 1H) ; 7,12 (td, 1H) ; 7,25 (dd, 1H).

### • Etape 3 : 2-(S)-Méthyl-3-(2-méthoxyméthoxy-phénylthio)-propionaldéhyde (Vb)

En procédant comme dans l'exemple 7 mais en utilisant, dans l'étape 2. Le 2-(S)-Méthyl-3-(2-méthoxyméthoxy-phénylthio)-propan-1-ol (XXIa) à la place du 2-(S)-Méthyl-3-(2-méthoxy-phénylthio)-propan-1-ol (XXb-1), on obtient le composé du titre (Vb) qui, comme l'aldéhyde (IIb-1), n'est pas isolé mais engagé *in situ* dans la réaction suivante d'amination réductrice.

### Exemple 19 : 2-(S)-Méthyl-3-(2-méthoxyméthoxy-3-méthyl-phénylthio)-propionaldéhyde (Vc)

En procédant comme dans l'exemple 18 mais en utilisant, dans l'étape 1. Le 2-Hydroxy-3-méthyl-thiophénol (IVa) à la place du 2-Hydroxy-thiophénol, on obtient le composé du titre (Vc) qui, comme l'aldéhyde (Vb), n'est pas isolé mais engagé *in situ* dans la réaction suivante d'amination réductrice.

### Exemple 20 : 2-(S)-Méthyl-3-(2-méthoxyméthoxy-3-éthyl-phénylthio)-propionaldéhyde (Vd)

En procédant comme dans l'exemple 18 mais en utilisant, dans l'étape 1, le 2-Hydroxy-3-éthyl-thiophénol (IVe) à la place 2-Hydroxy-thiophénol, on obtient le composé du titre (Vd) qui, comme l'aldéhyde (Vb), n'est pas isolé mais engagé *in situ* dans la réaction suivante d'amination réductrice.

### Exemple 21 : 2-(S)-Éthyl-3-(2-méthoxyméthoxy-3-méthyl-phénylthio)-propionaldéhyde (Ve)

### • Etape 1 : 2-(S)-Éthyl-3-(2-hydroxy-3-méthyl-phénylthio)-propan-1-ol (XXj)

En procédant comme dans l'exemple 10 mais en remplaçant, dans l'étape 4. Le 2-Méthoxy-thiophénol par le 2-Hydroxy-3-méthyl-thiophénol (IVa), on obtient 0,78 g (0,0034 mole) du composé du titre (XXj) sous forme d'une huile orangée.
Rendement : 79%
¹H RMN (DMSOd₆) δ: 0,84 (t, 3H) ; 1,40 (m, 2H) ; 1,51 (m, 1H) ; 2,16 (s, 3H) ; 2,71 (dd, 1H) ; 2,86 (dd, 1H) ; 3,39 (dd, 1H) ; 3,46 (dd, 1H) ; 4,55 (1s, 1H échangeable) ; 6,73 (t, 1H) ; 6,96 (d, 1H) ; 7,10 (d, 1H) ; 8,48 (1s, 1H échangeable).

### • Etape 2: 2-(S)-Éthyl-3-(2-méthoxyméthoxy-3-méthyl-phénylthio)-propionaldéhyde (Ve)

En procédant comme dans l'exemple 18 mais en utilisant, dans l'étape 2, le 2-(S)-Éthyl-3-(2-hydroxy-3-méthyl-phénylthio)-propan-1-ol (XXj) à la place du 2-(S)-Méthyl-3-(2-hydroxy-phénylthio)-propan-1-ol (XXh), on obtient le composé du titre (Ve) qui, comme l'aldéhyde (Vb), n'est pas isolé mais engagé *in situ* dans la réaction suivante d'amination réductrice.

### Exemnle 22 : 2-(S)-iso-Propyl-3-(2-méthoxyméthoxy-3-méthyl-phénylthio)-propionaldéhyde (Vf)

En procédant comme dans l'exemple 21 mais en utilisant, dans l'étape 1, le 2-(S)-iso-Propyl-3-hydroxy-propyl p-toluènesulfonate (XIXf) à la place du 2-(S)-Éthyl-3-hydroxy-propyl p-toluènesulfonate (XIXd-1), on obtient le composé du titre (Vf) qui, comme l'aldéhyde (Vb), n'est pas isolé mais engagé *in situ* dans la réaction suivante d'amination réductrice.

### Exemple 23 : 2-(S)-Méthyl-3-(2-méthoxyméthoxy-3-méthoxy-phénylthio)-propionaldéhyde (Vg)

En procédant comme dans l'exemple 18 mais en utilisant, dans l'étape 1, le 2-Mercapto-6-méthoxy-phénol (IVg) à la place du 2-Hydroxy-thiophénol, on obtient le composé du titre (Vg) qui, comme l'aldéhyde (Vb), n'est pas isolé mais engagé *in situ* dans la réaction suivante d'amination réductrice.

### Exemple 24 : 2-(S)-Méthyl-3-(2-méthoxyméthoxy 3-iso-propyl-phénylthio)-propionaldéhyde (Vh)

### • Etape 1 : 2-Hydroxy-3-iso-propyl-thiophénol (IVh)

En procédant comme dans l'exemple 15 mais en utilisant, dans l'étape 1, le 2-Isopropyl-phénol à la place du gaïacol, on obtient le composé du titre (IVh) sous la forme d'une huile jaune, qui est utilise brute pour l'étape suivante de S-alkylation conduisant à l'intermédiaire (XXh).
¹H RMN (DMSOd₆) δ: 1,14 (d, 6H) ; 3,27(m, 1H) ; 4,64 (1s, 1H) ; 6,73 (m, 1H) ; 6,96 (m, 1H) ; 7,10 (m, 1H) ; 8,54 (1s, 1H).

### • Etape 2 : 2-(S)-Méthyl-3-(2-méthoxyméthoxy-3-iso-propyl-phénylthio)-propionaldéhyde (Vh)

En procédant comme dans l'exemple 18 mais en utilisant, dans l'étape 1, le 2-Hydroxy-3-iso-propyl-thiophénol (IVh) à la place 2-Hydroxy-thiophénol, on obtient le composé du titre (Vh) qui, comme l'aldéhyde (Vb), n'est pas isolé mais engagé *in situ* dans la réaction suivante d'amination réductrice.

### Exemple 25 : 2-(S)-Méthyl-3-(2-méthoxyméthoxy-6-méthyl-phénylthio)-propionaldéhyde (Vj)

En procédant comme dans l'exemple 18 mais en utilisant, dans l'étape 1, le 2-Hydroxy-6-méthyl-phénol (IVc) à la place 2-Hydroxy-thiophénol, on obtient le composé du titre (Vj) qui, comme l'aldéhyde (Vb), n'est pas isolé mais engagé *in situ* dans la réaction suivante d'amination réductrice.

### Exemple 26 : 3-(R)-[3-(2-Méthoxyméthoxy-3-méthyl-phénylthio)-2-(S)-éthyl-propyl]amino-3,4-dihydro-2H-1,5-benzoxathiépine (VIa)

Les quantités de l'amine de formule (I) et de l'agent réducteur engagées dans la réaction d'amination réductrice sont calculées sur la base d'une réaction d'oxydation quantitative de l'alcool de formule (XXI) en l'aldéhyde de formule (V). Dans une solution de l'aldéhyde (Ve), 0,0023 mole théorique, constituée par le milieu réactionnel de la réaction d'oxydation de l'alcool (XXIj) en l'aldéhyde (Ve), maintenue à -10°C. On additionne 0,43 g (0,0024 mole) de 3-(R)-Amino-3,4-dihydro-2H-1,5-benzoxathiépine (Ia-1) en solution dans 5 ml de dichlorométhane. Après 10 minutes d'agitation à -10°C, on ajoute 0,75 g (0,0035 mole) de triacetoxyborohydrure de sodium et le mélange agité 1 heure 30 à -10°C puis hydrolysé au moyen d' une solution aqueuse à 10% de carbonate de sodium. Les phases sont séparées et la phase aqueuse extraite au dichlorométhane. Les phases organiques combinées sont lavées à l'eau, à la saumure, sécher sur sulfate de magnésium, filtrées et concentrées sous pression réduite. Le résidu est purifié par chromatographie flash sur gel de silice (éluant = cyclohexane/acétate d'éthyle = 70 : 30). On récupère 0,42 g (0,0097 mole) du composé du titre (VIa), sous forme d'une huile.
Rendement : 41 %
¹H RMN (DMSOd₆) : 0,88 (t, 3H) ; 1,45 (m, 2H) ; 1,60 (m,1H) ; 1,98 (m, 1H) ; 2,24 (s, 3H) ; 2,64 (1s, 2H) ; 2,84 (m, 2H) ; 3,05 (m, 3H) ; 3,53 (s, 3H) ; 3,93 (dd, 1H) ; 4,14 (dd, 1 H) ; 4,99 (s, 2H) ; 7,00 (m, 4H) ; 7,18 (m, 2H) ; 7,46 (d, 1H).

### Exemple 27: 3-(R)-[3-(2-Méthoxyméthoxy-3-méthyl-phénylthio)-2-(S)-méthylpropyl]amino-3,4-dihydro-2H-1,5-benzoxathiépine (VIb)

En procédant comme dans l'exemple 26 mais en utilisant le 2-(S)-Méthyl-3-(2-méthoxyméthoxy-3-méthyl-phénylthio)-propionaldéhyde (Vc) à la place du 2-(S)-Éthyl-3-(2-méthoxyméthoxy-3-méthyl-phénylthio)-propionaldéhyde (Ve), on obtient le composé du titre (VIb).
Rendement : 70%
¹H RMN (DMSOd₆) δ: 1,00 (d, 3H) ; 1,76 (m, 1H) ; 2,02 (1s, 1H échangeable) ; 2,24 (s, 3H) ; 2,60 (m, 2H) ; 2,70 (dd, 1H) ; 2,81 (dd, 1H) ; 3,09 (m, 3H) ; 3,53 (s, 3H) ; 3,92 (dd, 1H) ; 4,16 (dd, 1H) ; 4,99 (s, 2H) ; 7,00 (m, 4H) ; 7,17 (m, 2H) ; 7,33 (d, 1H).

### Exemple 28 : 3-(R)-[3-(2-Méthoxyméthoxy-3-méthyl-phénylthio)-2-(S)-iso-propyl-propyl]amino-3,4-dihydro-2H-1,5-benzoxathiépine (VIc)

En procédant comme dans l'exemple 26 mais en utilisant le 2-(S)-iso-Propyl-3-(2-méthoxyméthoxy-3-méthyl-phénylthio)-propionaldéhyde (Vf) à la place du 2-(S)-Éthyl-3-(2-méthoxyméthoxy-3-méthyl-phénylthio)-propionaldéhyde (Ve), on obtient le composé du titre (VIc) qui est utilisé sans autre purification dans l'étape suivante.
Rendement: 18%

### Exemple 29 : 3-(R)-[3-(2-Méthoxyméthoxy-phénylthio)-2-(S)-méthyl-propyl]amino-3,4-dihydro-2H-1,5-benzoxathiépine (VId)

En procédant comme dans l'exemple 26 mais en utilisant le 2-(S)-Méthyl-3-(2-méthoxyméthoxy-phénylthio)-propionaldéhyde (Vb) à la place du 2-(S)-Éthyl-3-(2-méthoxyméthoxy-3-méthyl-phénylthio)-propionaldéhyde (Ve), on obtient le composé du titre (VId).
Rendement : 77%
¹H RMN (DMSOd₆) δ: 1,01,(d, 3H) ; 1,77 (m, 1H) ; 2,02 (1s, 1H échangeable) ; 2,60 (m, 2H) ; 2,70 (dd, 1H) ; 2,81 (dd, 1H) ; 3,10 (m, 3H) ; 3,40 (s, 3H) ; 3,92 (dd, 1H) ; 4,16 (dd, 1H) ; 5,23 (s, 2H) ; 7,10 (m, 6H) ; 7,32 (m, 2H).

### Exemple 30 : 3-(R)-[3-(2-Méthoxyméthoxy-3-éthyl-phénylthio)-2-(S)-méthyl-propyl]amino-3,4-dihydro-2H-1,5-benzoxathiépine (VIe)

En procédant comme dans l'exemple 26 mais en utilisant le 2-(S)-Méthyl-3-(2-méthoxyméthoxy-3-éthyl-phénylthio)-propionaldéhyde (Vd) à la place du 2-(S)-Éthyl-3-(2-méthoxyméthoxy-3-méthyl-phénylthio)-propionaldéhyde (Ve), on obtient le composé du titre (VIe).
Rendement : 23%
¹H RMN (CDCl₃) δ: 1,09 (d, 3H) ; 1,22 (t, 3H) ; 1,83 (1s, 1H) ; 1,93 (m, 1H) ; 2,72 (m, 5H) ; 2,95 (dd, 1H) ; 3,09 (m, 3H) ;3,64 (s, 3H) ; 5,08 (s, 2H) ; 4,06 (dd, 1H) ; 4,25 (dd, 1H) ; 6,99 (m, 4H) ; 7,14 (m, 2H) ; 7,35 (dd, 1H).

### Exemple 31 : 3-(R)-[3-(2-Méthoxyméthoxy-3-iso-propyl-phénylthio)-2-(S)-méthyl-propyl)amino-3,4-dihydro-2H-1,5-benzoxathiépine (VIh)

En procédant comme dans l'exemple 26 mais en utilisant le 2-(S)-Méthyl-3-(2-méthoxyméthoxy-3-iso-propyl-phénylthio)-propionaldéhyde (Vh) à la place du 2-(S)-Éthyl-3-(2-méthoxyméthoxy-3-méthyl-phénylthio)-propionaldéhyde (Ve), on obtient le composé du titre (VIh).
Rendement : 30%
¹H RMN (CDCl₃) δ: 1,10 (d, 3H) ; 1,21 (d, 6H) ; 1,70 (1s, 1H) ; 1,92 (m, 1H) ; 2,63 (dd, 1H) ; 2,77 (m, 2H) ; 2,94 (dd, 1H); 3,10 (m, 3H); 3,42 (m, 1H); 3,64 (s, 3H); 4,06 (dd, 1H); 4,24 (dd, 1H); 5,07 (s, 2H); 6,96 (m, 2H); 7,11 (m, 4H); 7,35 (d, 1H).

### Exemple 32 : 3-(R)-[3-(2-Méthoxyméthoxy-3-méthyl-phénylthio)-2-(S)-méthyl-propyl]amino-6-méthyl-3,4-dihydro-2H-1,5-benzoxathiépine (VIg)

En procédant comme dans l'exemple 26 mais en utilisant le 2-(S)-Méthyl-3-(2-méthoxyméthoxy-3-méthyl-phénylthio)-propionaldéhyde (Vc) à la place du 2-(S)-Éthyl-3-(2-méthoxyméthoxy-3-méthyl-phénylthio)-propionaldéhyde (Ve) et la 3-(R)-Amino-6-méthyl-3,4-dihydro-2H-1,5-benzoxathiépine (Ic), à la place de la 3-(R)-Amino-3,4-dihydro-2H-1,5-benzoxathiépine (Ia-1), on obtient le composé du titre (VIg).
Rendement : 52%
¹H RMN (DMSOd₆) δ: 1,00 (d, 3H) ; 1,78 (1s, 1H) ; 2,24 (s, 3H) ; 2,31 (s, 3H) ; 2,55 (1s, 1H) ; 2,69 (m, 2H) ; 2,86 (1s, 1H) ; 3,10 (m, 3H) ; 3,53 (s, 3H) ; 3,94 (1s, 1H) ; 4,18 (1d, 1H) ; 4,99 (s, 2H) ; 6,81 (d, 1H) ; 6,92 (d, 1H) ; 7,03 (m, 3H) ; 7,18 (m, 1H).

### Exemple 33 : 3-(R)-[3-(2-Méthoxyméthoxy-3-méthoxy-phénylthio)-2-(S)-méthylpropyl]amino-3,4-dihydro-2H-1,5-benzoxathiépine (VIi)

En procédant comme dans l'exemple 26 mais en utilisant le 2-(S)-Méthyl-3-(2-méthoxyméthoxy-3-méthoxy-phénylthie)-propionaldéhyde (Vg) à la place du 2-(S)-Éthyl-3-(2-méthoxyméthoxy-3-méthyl-phénylthio)-propionaldéhyde (Ve), on obtient le composé du titre (VIi).
Rendement : 76%
¹H RMN (DMSOd₆) δ: 1,00 (d, 3H) ; 1,77 (m, 1H) ; 2,02 (m, 1H) ; 2,55 (m, 1H); 2,64 (m, 1H); 2,70 (dd, 1H); 2,81 (dd, 1H); 3,09 (m, 3H); 3,54 (s, 3H); 3,77 (s, 3H); 3,92 (dd, 1H); 4,16 (dd, 1H); 5,05 (s, 2H); 6,96 (m, 5H); 7,17 (m, 1H); 7,33 (d, 1H).

### Exemple 34 : 3-(R)-[3-(2-Méthoxyméthoxy-6-méthyl-phénylthio)-2-(S)-méthyl-propyl]amino-3,4-dihydro-2H-1,5-benzoxathiépine (VIj)

En procédant comme dans l'exemple 26 mais en utilisant le 2-(S)-Méthyl-3-(2-méthoxyméthoxy-6-méthyl-phénylthio)-propionaldéhyde (Vj) à la place du 2-(S)-Éfhyl-3-(2-méthoxyméthoxy-3-méthyl-phénylthio)-propionaldéhyde (Ve), on obtient le composé du titre (VIj).
Rendement : 74%
¹H RMN (DMSOd₆) δ: 0,96 (d, 3H) ; 1,56 (m, 1H) ; 1,88 (1s, 1H) ; 2,45 (s, 3H) ; 2,59 (m, 3H) ; 2,75 (dd, 1H) ; 2,89 (m, 3H) ; 3,42 (s, 3H) ; 3,86 (dd, 1H) ; 4,09 (dd, 1H) ; 5,24 (s, 2H) ; 6,95 (m, 4H) ; 7,16 (m, 2H) ; 7,33 (dd, 1H).

### Exemple de référence 1 : 3-(R)-[3-(2-Méthoxy-phénylthio)-2-(S)-méthoxypropyl]amino-3,4-dihydro-2H-1,5-benzoxathiépine (1-1)

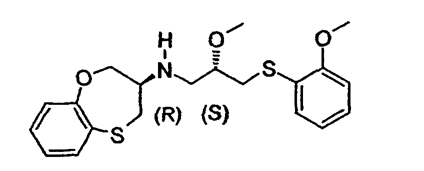

Dans un ballon maintenu sous atmosphère inerte, on introduit 1,43 g (0,0063 mole) de 2-(S)-Méthoxy-3-(2-méthoxy-phénylthio)-propionaldéhyde (IIa-1) et 5 ml de dichloro-1,2-éthane. On ajoute goutte à goutte 0,60 g (0,0063 mole) de 3-(R)-Amino-3,4-dihydro-2H-1,5-benzoxathiépine (Ia-1) en solution dans 5 ml de dichloro-1,2-éthane. Le mélange est refroidi à 0°C puis on introduit 1 g (0,0047 mole) de triacetoxyborohydrure de sodium. La solution est agitée à température ambiante pendant 5 heures puis hydrolysée au moyen d'une solution aqueuse à 10% de bicarbonate de sodium. Les phases sont séparées et la phase aqueuse extraite au dichlorométhane. Les phases organiques combinées sont lavées à la saumure, séchées sur du sulfate de magnésium, filtrées et concentrées sous pression réduite. Le résidu est purifié par chromatographie flash sur gel de silice (éluant : dichlorométhane/acétate d'éthyle = 90: 10). On récupère 0,88 g (0,0022 mole) du composé (1-1) sous la forme d'une huile jaune.
Rendement : 36%
¹H RMN (CDCl₃) δ: 1,96 (1s, 1H échangeable) ; 2,81 (dd, 1H) ; 2,98 (m, 2H) ; 3,12 (m, 4H) ; 3,41 (s, 3H) ; 3,49 (m, 1H) ; 3,90 (s, 3H) ; 4,16 (m, 2H) ; 6,86 (d, 1H) ; 6,94 (m, 3H) ; 7,12 (t, 1H) ; 7,20 (t, 1H) ; 7,35 (m, 2H).

On dissout 0,88 g (0,0022 mole) du produit (1-1) dans 3 ml de méthanol, puis on ajoute 0,23 g (0,0020 mole) d'acide maléique solubilisé dans 2 ml de méthanol. La solution obtenue est concentrée puis on ajoute de l'éther éthylique. Le précipité formé est filtré. Lavé à l'éther éthylique et séché sous vide à 50°C. On obtient 0,90 g (0,0018 mole) du maléate du composé (1-1) sous la forme d'un solide blanc.
F : 122°C
[α] = - 15,3 (c = 0,300, méthanol)

| Analyse C₂₄H₂₉NO₇S₂ : | | | |
|---|---|---|---|
| Calc.% | C 56,79 | H 5,76 | N 2,76 |
| Tr. | C 56,42 | H 5,81 | N 2,96 |

¹H RMN (DMSOd₆) δ: 3,22 (m, 8H) ; 3,36 (s, 3H) ; 3,78 (1s, 1H) ; 3,84 (s, 3H) ; 4,30 (1d, 1H) ; 4,46 (1d, 1H); 6,04 (s, 2H); 7,00 (m, 4H); 7,24 (m, 2H) ; 7,38 (m, 2H), 8,90 (1s, 2H échangeables)
HPLC (Chiracel OD, hexane/isopropanol (90: 10), 1 ml/min) : composé (1-1), temps de rétention = 25,40 min ; composé (1-2), temps de rétention = 21,99 min; rapport des AUC, (1-1) / (1-2) = 95: 5.

### Exemple de référence 2 : 3-(R)[3-(2-Méthoxy-phénylthio)-2-(R)-méthoxypropyl]amino-3,4-dihydro-2H-1,5-benzoxathiépine (1-2)

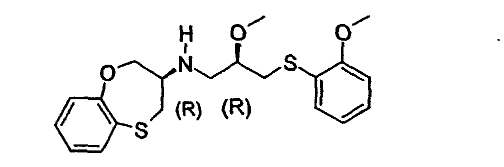

En procédant comme dans l'exemple de référence 1 mais en utilisant le 2-(R)-Méthoxy-3-(2-méthoxy-phénylthio)-propionaldéhyde (IIa-2) à la place du 2-(S)-Méthoxy-3-(2-méthoxy-phénylthio)-propionaldéhyde (IIa-1), on obtient le composé (1-2).
Rendement : 33%
¹H RMN (CDCl₃) δ: 1,95 (1s, 1H échangeable) ; 3,03 (m, 7H) ; 3,41 (s, 3H) ; 3,50 (m, 1H) ; 3,90 (s, 3H) ; 4,16 (m, 2H) ; 6,86 (d, 1H) ; 6,93 (m, 3H) ; 7,12 (td, 1H) ; 7,21 (td, 1H) ; 7,34 (m, 2H).

On dissout 0,79 g (0,002 mole) du produit (1-2) dans 3 ml de méthanol, puis on ajouté 0,21 g (0,0018 mole) d'acide maléique solubilisé dans 2 ml de méthanol. On concentre la solution obtenue puis on ajoute de l'éther éthylique. Le précipité formé est filtré. Lavé à l'éther éthylique et séché sous vide à 50°C. On obtient 0,90 g (0,0018 mole) du maléate du composé (1-2) sous la forme d'un solide blanc.
F : 116°C
[α] = + 60,5 (c = 0,228, méthanol)

| Analyse C₂₄H₂₉NO₇S₂ : | | | |
|---|---|---|---|
| Calc.% | C 56,79 | H 5,76 | N 2,76 |
| Tr. | C 56.55 | H 5.69 | N 2,92 |

¹H RMN (DMSOd₆) δ: 3,21 (m, 8H) ; 3,36 (s, 3H) ; 3,81 (1s, 1H) ; 3,84 (s, 3H) ; 4,26 (1d, 1H) ; 4,43 (1d, 1H) ; 6,04 (s, 2H) ; 7,03 (m, 4H) ; 7,24 (m, 2H) ; 7,35 (d, 1H) ; 7,42 (d, 1H) ; 8,83 (1s, 2H échangeables)
HPLC (Chiracel OD, hexane/isopropanol (90 : 10), 1 ml/min) : composé (1-2), temps de rétention = 20,75 min ; composé (1-1), temps de rétention = 25,47 min ; rapport des AUC, (1-2) / (1-1) = 86 : 14.

### Exemple de référence 3 : 3-(R)-[3-(2-Hydroxy-phénylthio)-2-(S)-hydroxypropyl]amino-3,4-dihydro-2H-1,5-benzoxathiépine (1-3)

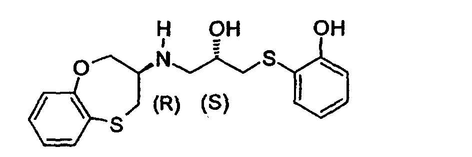

Dans un ballon maintenu sous atmosphère inerte, on introduit 0,5 g (0,0021 mole) de 3-(R)-([(S)-3,4-epoxypropyl]amino)-3,4-dihydro-2H-1,5-benzoxathiépine (III-1) et 15 ml d'éthanol. On ajoute ensuite goutte à goutte 0,22 ml (0,0021 mole) de 2-Hydroxy-thiophénol puis après 15 minutes d'agitation à température ambiante 0,45 g (0,0042 mole) de carbonate de sodium. Le mélange est agité à température ambiante pendant 12 heures puis concentré sous pression réduite. Le résidu est repris dans du dichlorométhane et la solution obtenue lavée à l'eau puis séchée sur du sulfate de sodium, filtrée, concentrée sous vide. Le résidu est purifié par cbromatographie flash sur gel de silice (éluant : dichlorométhane/méthanol = 96: 4). On récupère 0,58 g (0,0016 mole) du composé (1-3) sous forme d'une huile jaune pâle.
Rendement : 76%
¹H RMN (DMSOd₆) δ: 2,60 (m, 1H) ; 2,93 (m, 6H) ; 3,63 (m, 1H) ; 3,97 (dd, 1H) ; 4,12 (dd, 1H); 6,78 (m, 2H) ; 7,00 (m, 3H) ; 7,17 (td, 1H) ; 7,25 (dd, 1H) ; 7,33 (dd, 1H).

On dissout 0,57 g (0,0016 mole) du produit (1-3) dans 3 ml de méthanol, puis on ajoute 0,13 g (0,0014 mole) d'acide oxalique solubilisé dans 2 ml de méthanol. On concentre la solution obtenue puis on ajoute de l'éther éthylique. Le précipité formé est filtré. Lavé à l'éther éthylique et séché sous vide à 50°C. On obtient 0,52 g (0,0011 mole) de l'oxalate du composé (1-3) sous la forme d'un solide blanc.
F : 176-7°C
[α] = - 5.2 (c = 0,309, méthanol)

| Analyse C₂₀H₂₃NO₇S₂ : | | | |
|---|---|---|---|
| Calc.% | C 52,96 | H 5,11 | N 3,09 |
| Tr. | C 52,90 | H 5,15 | N 3,26 |

¹H RMN (DMSOd₆) δ: 2,97 (m, 3H) ; 3,22 (m, 3H) ; 3,72 (1s, 1H) ; 3,91 (m, 1H) ; 4,35 (m, 2H) ; 6,79 (td, 1H) ; 6,85 (dd, 1H) ; 7,06 (m, 3H) ; 7,24 (m, 2H) ; 7,40 (dd, 1H)
HPLC (Chiralpack AD, hexane/éthanol (50 : 50), 1 ml/min) : composé (1-3), temps de rétention = 23,08 min ; composé (1-4), temps de rétention = 19,40 min ; rapport des AUC, (1-3) / (1-4) = 99:1.

### Exemple de référence 4 : 3-(R)-[3-(2-Hydroxy-phénylthio)-2-(R)-hydroxy-propyl]amino-3,4-dihydro-2H-1,5 benzoxathiépine (1-4)

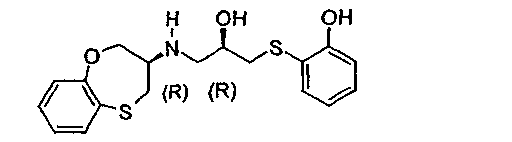

En procédant comme dans l'exemple de référence 3 mais en utilisant la 3-(R)-([(R)-3,4-Epoxypropyl]amino)-3,4-dihydro-2H-1,5-benzoxathiépine (III-2) à la place de la 3-(R)-([(S)-3,4-Epoxypropyl]amino)-3,4-dihydro-2H-1,5-benzoxathiépine (III-1), on obtient le composé (1-4).
Rendement : 95%
¹H RMN (DMSOd₆) δ: 2,68 (m, 2H) ; 2,89 (m, 3H) ; 3,10 (m, 2H) ; 3,63 (m, 1H), 3,93 (dd, 1H) ; 4,12 (dd, 1H) ; 6,79 (m, 2H) ; 7,00 (m, 3H) ; 7,17 (td, 1H) ; 7,25 (dd, 1H) ; 7,34 (dd, 1H).

On dissout 0,50 g (0,0014 mole) du produit (1-4) dans 3 ml de méthanol, puis on ajoute 0,12 g (0,0013 mole) d'acide oxalique solubilisé dans 2 ml de méthanol. On concentre la solution obtenue. Le précipité formé est filtré, lavé à l'éther éthylique et séché sous vide à 50°C. On obtient 0,53 g (0,0011 mole) de l'oxalate du composé (1-4) sous la forme d'un solide blanc.
F : 135°C
[α] = + 60,48 (c = 0,248, méthanol)

| Analyse C₂₀H₂₃NO₇S₂ : | | | |
|---|---|---|---|
| Cale.% | C 52,96 | H5,11 | N 3,09 |
| Tr. | C 53,33 | H 5,12 | N 3,15 |

¹H RMN (DMSOd₆) δ: 2,96 (m, 3H) ; 3,23 (m, 3H) ; 3,68 (1s, 1H) ; 3,90 (m, 1H) ; 4,46 (m, 2H) ; 6,79 (t, 1H) ; 6,85 (d, 1H) ; 7,05 (m, 3H) ; 7,25 (m, 2H); 7,40 (dd, 1H),
HPLC (Chiralpack AD, hexane/éthanol (50 : 50), 1 ml/min) : composé (1-4), temps de rétention = 18,70 min ; composé (1-3), temps de rétention = 22,71 min; rapport des AUC, (1-4) / (1-3) = 96 : 4.

### Exemple de référence 5 : 3-(R)-[3-(2-Méthoxy-phénylthio)-2-(S)-hydroxy-propyl]amino-3,4-dihydro-2H-1,5-benzoxathiépine (1-5)

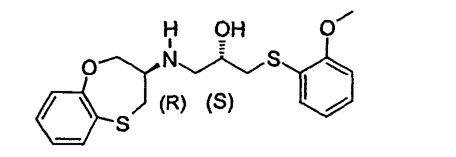

En procédant comme dans l'exemple de référence 3 mais en utilisant le 2-Méthoxy-thiophénol à la place du 2-Hydroxy-thiophénol, on obtient le composé (1-5).
Rendement : 38%
¹H RMN (DMSOd₆) δ: 2,12 (1s, 1H échangeable) ; 2,64 (m, 1H) ; 2,81 (m, 2H); 2,89 (dd, 1H) ; 3,06 (m, 3H) ; 3,65 (m, 1H) ; 3,81 (s, 3H) ; 3,97 (dd, 1H) ; 4,13 (dd, 1H) ; 5,07 (d, 1H échangeable) ; 6,95 (m, 4H) ; 7,16 (m,2H) ; 7,31 (m, 2H).

On dissout 0,20 g (0,0005 mole) du produit (1-5) dans 3 ml de méthanol, puis on ajoute 0,06 g (0,0005 mole) d'acide maléique solubilisé dans 2 ml de méthanol. On concentre la solution obtenue puis on ajoute de l'éther éthylique. Le précipité est filtré. Lavé à l'éther éthylique et séché sous vide à 50°C. On obtient 0,14 g (0,0003 mole) du maléate du composé (1-5) sous la forme d'un solide blanc;
F : 133-5°C
[α] = - 3,2 (c = 0,436, méthanol)

| Analyse C₂₃H₂₇NO₇S₂ : | | | |
|---|---|---|---|
| Calc.% | C 55,97 | H 5,51 | N 2,84 |
| Tr. | C 55,83 | H 5,40 | N 2,93 |

¹H RMN (DMSOd₆) δ:: 3,03 (m, 3H) ; 3,25 (d, 2H) ; 3,30 (1s, 1H); 3,83 (s, 3H) ; 3,86 (1s, 1H) ; 3,99 (1s, 1H) ; 4,32 (1d, 1H); 4,47 (1d, 1H) ; 5,85 (1s, 1H) ; 6,03 (s, 2H) ; 7,00 (m, 4H) ; 7,22 (m, 2H); 7,32 (dd, 1H) ; 7,41 (dd, 1H)
HPLC (Chiracel OD, hexane/éthanol (80 : 20), 1 ml/min): composé (1-5), temps de rétention = 20,04 min; composé (1-6), temps de rétention = 16,29 min; rapport des AUC, (1-5) / (1-6) = 95 : 5.

### Exemple de référence 6 : 3-(R)-[3-(2-Méthoxy-phénylthio)-2-(R)-hydroxy-propyl]amino-3,4-dihydro-2H-1,5-benzoxathiépine (1-6)

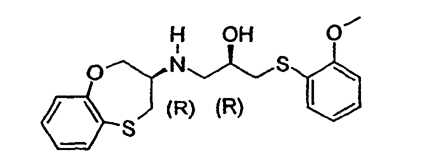

En procédant comme dans l'exemple de référence 5 mais en utilisant la 3-(R)-([(R)-3,4-Epoxypropyl]amino)-3,4-dihydro-2H-1,5-benzoxathiépine (III-2) à la place de la 3-(R)-([(S)-3,4-Epoxypropyl]amino)-3,4-dihydro-2H-1,5-benzoxathiépine (III-1), on obtient le composé (1-6).
Rendement : 70%
¹H RMN (DMSOd₆) δ: 2,13 (1s, 1H échangeable) ; 2,68 (m, 2H); 2,88 (m, 2H) ; 2,99 (dd, 1H) ; 3,10 (m, 2H) ; 3,65 (m, 1H) ; 3,81 (s, 3H) ; 3,93 (dd, 1H) ; 4,12 (dd, 1H) ; 5,07 (d, 1H échangeable) ; 6,95 (m,4H) ; 7,16 (m, 2H); 7,31 (m, 2H).

On dissout 0,52 g (0,0014 mole) du produit (1-6) dans 3 ml de méthanol, puis on ajoute 0,15 g (0,0013 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,59 g (0,0012 mole) du maléate du composé (1-6) sous la forme d'un solide blanc.
F : 136-8°C
[α] = + 60,3 (c = 0,745, méthanol)

| Analyse C₂₃H₂₇NO₇S₂ : | | | |
|---|---|---|---|
| Cale.% | C 55,97 | H 5,51 | N 2,84 |
| Tr. | C 55,99 | H 5,59 | N 2,96 |

¹H RMN (DMSOd₆) δ: 3,03 (m, 3H) ; 3,26 (d, 2H) ; 3,30 (1s, 1H) ; 3,83 (s, 3H) ; 3,86 (1s, 1H) ; 4,00 (1s, 1H) ; 4,26 (1d, 1H); 4,44 (1d, 1H) ; 5,85 (1s, 1H) ; 6,03 (s, 2H) ; 7,04 (m, 4H) ; 7,22 (m, 2H) ; 7,33 (dd, 1H) ; 7,43 (dd, 1H)
HPLC (Chiracel OD, hexane/éthanol (80: 20), 1 ml/min) : composé (1-6), temps de rétention = 16,29 min; composé (1-5), temps de rétention = 20,04 min ; rapport des AUC, (1-6) / (1-5) = 97 : 3.

### Exemple de référence 7 : 3-(R)-[3-(2-Méthoxy-phénylthio)-2-(S)-méthyl-propyl]amino-3,4-dihydro-2H-1,5-benzoxathiépine (1-7)

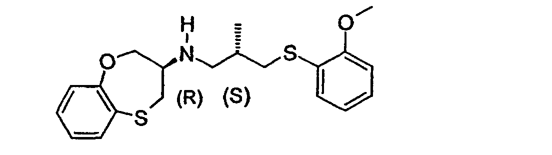

En procédant comme dans l'exemple 26, mais à partir du mélange réactionnel contenant le 2-(S)-Méthyl-3-(2-méthoxy-phénylthio)-propionaldéhyde (IIb-1) à la place du 2-(S)-Éthyl-3-(2-méthoxyméthoxy-3-méthyl-phénylthio)-propionaldéhyde (Ve), on obtient le composé (1-7).
Rendement : 58%
¹H RMN (CDCl₃) δ: 1,10 (d, 3H) ; 1,68 (1s, 1H échangeable) ; 1,91 (m, 1H) ; 2,63 (dd, 1H) ; 2,78 (m, 2H) ; 2,93 (dd, 1H) ; 3,10 (m, 3H) ; 3,90 (s, 3H) ; 4,07 (dd, 1H); 4,23 (dd, 1H) ; 6,84 (d, 1H) ; 6,91 (m, 3H) ; 7,14 (m, 2H) ; 7,33 (m, 2H).

On dissout 2,85 g (0,0076 mole) du produit (1-7) dans 5 ml de méthanol, puis on ajoute 0,84 g (0,0072 mole) d'acide fumarique solubilisé dans 3 ml de méthanol. On concentre la solution obtenue puis on ajoute de l'éther isopropylique. Le précipité formé est filtré, lavé à l'éther isopropylique et séché sous vide à 50°C. On obtient 3,36 g (0,0068 mole) du fumarate du composé (1-7) sous la forme d'un solide blanc.
F : 133-4°C
[α] = - 1,2 (c = 0,446, méthanol)

| Analyse C₂₄H₂₉NO₆S₂ : | | | |
|---|---|---|---|
| Calc.% | C 58,63 | H 5,95 | N 2,85 |
| Tr. | C 58,53 | H 5,89 | N 2,74 |

¹H RMN (DMSOd₆) δ: 1,01 (d, 3H) ; 1,80 (m, 1H) ; 2,59 (dd, 1H) ; 2,69 (m, 2H) ; 2,87 (dd, 1H) ; 3,11 (m, 3H) ; 3,81 (s, 3H) ; 3,98 (dd, 1H) ; 4,19 (dd, 1H) ; 6,61 (s, 2H) ; 6,96 (m, 4H) ; 7,16 (m, 2H) ; 7,27 (d, 1H) ; 7,34 (d, 1H)
HPLC (Chiracel OD, hexane/isopropanol (80 : 20), 1 ml/min) : composé (1-7), temps de rétention = 13,09 min ; composé (1 -8), temps de rétention = 9,15 min ; rapport des AUC, (1 -7) / (1-8) = 99 : 1.

### Exemple de référence 8 : 3-(R)-[3-(2-Méthoxy-phénylthio)-2-(R)-méthyl-propyl]amino-3,4-dihydro-2H-1,5 benzoxathiépine (1-8)

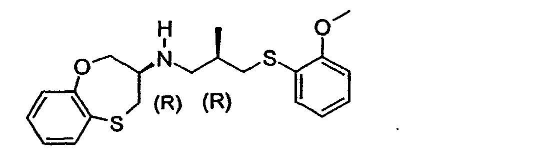

En procédant comme dans l'exemple 26, mais à partir du mélange réactionnel contenant le 2-(R)-Méthyl-3-(2-méthoxy-phénylthio)-propionaldéhyde (IIb-2) à la place du 2-(S)-Éthyl-3-(2-méthoxyméthoxy-3 méthyl-phénylthio)-propionaldéhyde (Ve), on obtient le composé (1-8).
Rendement : 58%
¹H RMN (CDCl₃) δ: 1,10 (d, 3H) ; 1,68 (1s, 1H échangeable) ; 1,91 (m, 1H) ; 2,63 (dd, 1H) ; 2,78 (m, 2H) ; 2,93 (dd, 1H) ; 3,10 (m, 3H) ; 3,90 (s, 3H) ; 4,07 (dd, 1H) ; 4,23 (dd, 1H) ; 6,84 (d, 1H) ; 6,91 (m, 3H); 7,14 (m, 2H) ; 7,33 (m, 2H).

On dissout 0,60 g (0,0016 mole) du produit (1-8) dans 3 ml de méthanol, puis on ajoute 0,18 g (0,0015 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,72 g (0,0014 mole) du maléate du composé (1-8) sous la forme d'un solide blanc.
F : 140°C
[α] = + 52,4 (c = 0,254, méthanol)

| Analyse C₂₄H₂₉NO₆S₂ : | | | |
|---|---|---|---|
| Calc.% | C 58,63 | H 5,95 | N 2,85 |
| Tr. | C 58,48 | H 5,99 | N 3,13 |

¹H RMN (DMSOd₆) δ: 1,11 (d, 3H) ; 2,13 (m, 1H) ; 2,83 (dd, 1H) ; 2,98 (m, 1H) ; 3,06 (dd, 1H) ; 3,24 (m, 4H) ; 3,82 (s, 3H) ; 4,33 (1d, 1H) ; 4,44 (1d, 1H) ; 6,03 (s, 2H) ; 7,02 (m, 4H) ; 7,24 (m, 3H) ; 7,40 (d, 1H)
HPLC (Chiralpack AS, méthanol, 1 ml/min) : composé (1-7), temps de rétention = 10,67 min ; composé (1-8), temps de rétention = 8,81 min; rapport des AUC, (1-8) / (1-7) = 87 : 13.

### Exemple de référence 9 : 3-(R)-[3-(2-Méthoxy-phénylthio)-2-(S)-éthyl-propyl]amino-3,4-dihydro-2H-1,5-benzoxathiépine (1-9)

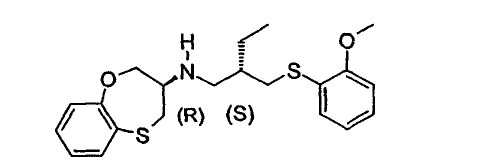

En procédant comme dans l'exemple 26, mais à partir du mélange réactionnel contenant le 2-(S)-Éthyl-3-(2-méthoxy-phénylthio)-propionaldéhyde (IId-1) à la place du 2-(S)-Éthyl-3-(2-méthoxyméthoxy-3-méthyl-phénylthio)-propionaldéhyde (Ve), on obtient le composé (1-9).
Rendement : 93%
¹H RMN (DMSOd₆) δ: 0,87 (t, 3H) ; 1,44 (m, 2H); 1,59 (m, 1H) ; 1,98 (1s, 1H) ; 2,64 (m, 2H) ; 2,82 (m, 2H) ; 3,04 (m, 3H) ; 3,80 (s, 3H) ; 3,93 (dd, 1H) ; 4,14 (dd, 1H) ; 6,96 (m, 4H) ; 7,15 (m, 2H) ; 7,28 (dd, 1H) ; 7,33 (dd, 1H).

On dissout 1,1 g (0,0028 mole) du produit (1-9) dans 5 ml de méthanol, puis on ajoute 0,29 g (0,0025 mole) d'acide fumarique solubilisé dans 3 ml de méthanol. On concentre la solution obtenue puis on ajoute du pentane. Le précipité formé est filtré, lavé au pentane et séché sous vide à 50°C. On obtient 1,19 g (0,0023 mole) du fumarate du composé (1-9) sous la forme d'un solide blanc.
F : 86-8°C
[α] = - 8 (c = 0,512, méthanol)

| Analyse C₂₅H₃₁NO₆S₂ : | | | |
|---|---|---|---|
| Calc.% | C 59,38 | H 6,18 | N 2,77 |
| Tr. | C 59,32 | H 6,18 | N 2,98 |

¹H RMN (DMSOd₆) δ: 0,87 (t, 3H) ; 1,44 (m, 2H) ; 1,63 (m, 1H) ; 2,68 (m, 2H) ; 2,85 (m, 2H) ; 3,02 (dd, 1H) ; 3,11 (m, 2H) ; 3,80 (s, 3H) ; 3,97 (dd, 1H) ; 4,16 (dd, 1H) ; 6,61 (s, 2H) ; 6,96 (m, 4H); 7,16 (m, 2H) ; 7,29 (dd, 1H) ; 7,34 (m, 1H)
HPLC (Chiracel OD, hexanelisopropanol (95 : 5), 1 ml/min) : composé (1-9), temps de rétention = 16,62 min ; composé (1-10), temps de rétention = 14,69 min ; rapport des AUC, (1-9) / (1-10) = 97: 3.

### Exemple de référence 10 : 3-(R)-[3-(2-Méthoxy-phénylthio)-2-(R)-éthyl-propyl]amino-3,4-dihydro-2H-1,5-benzoxathiépine (1-10)

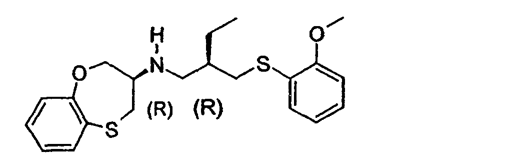

En procédant comme dans l'exemple 26, mais à partir du mélange réactionnel contenant le 2-(R)-Éthyl-3-(2-méthoxy-phénylthio)-propionaldéhyde (IId-2) à la place du 2-(S)-Éthyl-3-(2-méthoxyméthoxy-3-méthyl-phénylthio)-propionaldéhyde (Ve), on obtient le composé (1-10).
Rendement : 49%
¹H RMN (DMSOd₆) δ: 0,86 (t, 3H); 1,43 (m, 2H) ; 1,56 (m, 1H) ; 2,89 (m, 7H) ; 3,80 (s, 3H) ; 3,91 (dd, 1H) ; 4,14 (dd, 1H) ; 6,95 (m, 4H) ; 7,24 (m, 4H).

On dissout 0,17 g (0,0044 mole) du produit (1-10) dans 3 ml de méthanol, puis on ajoute 0,05 g (0,0043 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,15 g (0,0030 mole) du maléate du composé (1-10) sous la forme d'un solide blanc.
F : 140°C
[α] = + 71,7 (c = 0,318, méthanol)

| Analyse C₂₅H₃₁NO₆S₂ : | | | |
|---|---|---|---|
| Calc.% | C 59,38 | H 6,18 | N 2,77 |
| Tr. | C 59,20 | H 6,07 | N 2,93 |

¹H RMN (DMSOd₆) δ: 0,90 (t, 3H) ; 1,52 (m, 2H) ; 1,99 (m, 1H) ; 3,16 (m, 7H) ; 3,82 (s, 3H) ; 4,33 (1d, 1H) ; 4,44 (m, 1H) ; 6,04 (s, 2H) ; 7,02 (m, 4H); 7,24 (m, 3H) ; 7,40(d, 1H)
HPLC (Chiracel OD, hexane/isopropanol (95 : 5), 1 ml/min) : composé (1-10), temps de rétention = 14,01 min; composé (1-9), temps de rétention = 16,47 min ; rapport des AUC, (1-10) / (1-9) = 98 : 2.

### Exemple de référence 11 : 3-(R)-[3-(2-Méthoxy-phénylthio)-2-(S)-n-propyl-propyl]amino-3,4-dihydro-2H-1,5-benzoxathiépine (1-11)

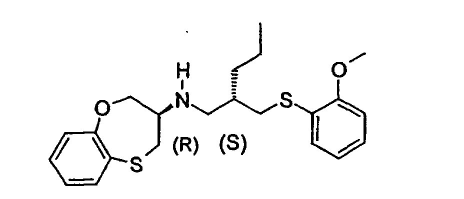

En procédant comme dans l'exemple 26, mais à partir du mélange réactionnel contenant le 2-(S)-n-Propyl-3-(2-méthoxy-phénylthio)-propionaldéhyde (IIe-1) à la place du 2-(S)-Éthyl-3-(2-méthoxyméthoxy-3-méthyl-phénylthio)-propionaldéhyde (Ve), on obtient le composé (1-11),
Rendement : 80%
¹H RMN (DMSOd₆) δ: 0,85 (t, 3H) ; 1,33 (m, 4H) ; 1,65 (m , 1H) ; 1,96 (m, 1H) ; 2,63 (m, 2H); 2,82 (m, 2H) ; 3,03 (m, 3H) ; 3,80 (s, 3H); 3,92 (dd, 1H) ; 4,14 (dd, 1H) ; 6,95 (m, 4H) ; 7,16 (m, 2H) ; 7,28 (d, 1H) ; 7,34 (d, 1H).

On dissout 0,31 g (0,0077 mole) du produit (1-11) dans 3 ml de méthanol, puis on ajoute 0,08 g (0,0070 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,31 g (0,0060 mole) du maléate du composé (1-11) sous la forme d'un solide blanc.
F : 132-3°C
[α] = - 12,7 (c = 0,434, méthanol)

| Analyse C₂₆H₃₃NO₆S₂ : | | | |
|---|---|---|---|
| Cale.% | C 60,09 | H 6,40 | N 2,70 |
| Tr. | C 60,13 | H 6,29 | N 2,87 |

¹H RMN (DMSOd₆) δ: 0,87 (t, 3H) ; 1,32 (m, 2H) ; 1,45 (m, 2H) ; 2,05 (1s, 1H) ; 3,13 (m, 8H) ; 3,82 (s, 3H) ; 4,33 (1d, 1H) ; 4,43 (1d, 1H) ; 6,04 (s, 2H) ; 7,02 (m, 4H); 7,24 (m, 3H) ; 7,40 (d, 1H).
HPLC (Chiralpack AD, hexane/éthanol (97: 3), 1 ml/min) : composé (1-11), temps de rétention = 8,65 min ; composé (1-12), temps de rétention = 9,16 min ; rapport des AUC, (1-11) / (1-12) = 93 : 7.

### Exemple de référence 12 : 3-(R)-[3-(2-Méthoxy-phénylthio)-2-(R)-n-propyl-propyl]amino-3,4-dihydro-2H-1,5-benzoxathiépine (1-12)

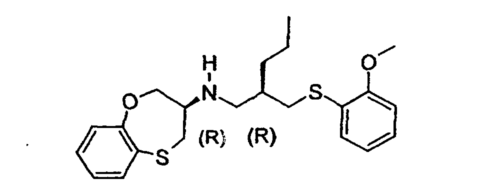

En procédant comme dans l'exemple 26, mais à partir du mélange réactionnel contenant le 2-(R)-n-Propyl-3-(2-méthoxy-phénylthio)-propionaldéhyde (IIe-2) à la place du 2-(S)-Éthyl-3-(2-méthoxyméthoxy-3-méthyl-phénylthio)-propionaldéhyde (Ve), on obtient le composé (1-12), Rendement : 47%
¹H RMN (CDCl₃) δ: 0,90 (t, 3H) ; 1,37 (m, 2H) ; 1,46 (m, 2H) ; 1,81 (m, 1H) ; 2,74 (m, 2H) ; 3,03 (m, 5H) ; 3,90 (s, 3H) ; 4,08 (dd, 1H) ; 4,20 (dd, 1H) ; 6,84 (d, 1H) ; 6,94 (m, 3H) ; 7,14 (m, 2H) ; 7,34 (m, 2H).

On dissout 0,16 g (0,0039 mole) du produit (1-12) dans 3 ml de méthanol, puis on ajoute 0,04 g (0,0034 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,12 g (0,0023 mole) du maléate du composé (1-12) sous la forme d'un solide blanc.
F : 131-3°C
[α] = + 63,3 (c = 0,216, méthanol)

| Analyse C₂₆H₃₃NO₆S₂ : | | | |
|---|---|---|---|
| Calc.% | C 60,09 | H 6,40 | N 2,70 |
| Tr. | C 60,15 | H 6,56 | N 2,79 |

¹H RMN (DMSOd₆) δ: 0,87 (t, 3H) ; 1,32 (m, 2H) ; 1,46 (m, 2H) ; 2,05 (1s, 1H) ; 3,16 (m, 8H) ; 3,82 (s, 3H) ; 4,33 (1d, 1H) ; 4,43 (1s, 1H); 6,04 (s, 2H) ; 7,02 (m, 4H); 7,24 (m, 3H) ; 7,40(d, 1H)
HPLC (Chiralpack AD, héxane/éthanol (97: 3), 1 ml/min) : composé (1-12), temps de rétention = 9,15 min ; composé (1-11), temps de rétention = 8,66 min ; rapport des AUC, (1-12) / (1-11) = 94: 6.

### Exemple de référence 13 : 3-(R)-[3-(2-Méthoxy-phénylthio)-2-(S)-iso-propyl-propyl]amino-3,4-dihydro-2H-1,5-benzoxathiépine (1-13)

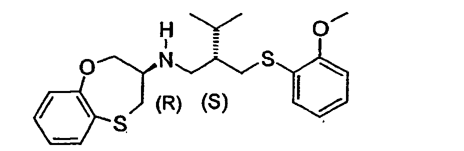

En procédant comme dans l'exemple 26, mais à partir du mélange réactionnel contenant le 2-(S)-iso-Propyl-3-(2-méthoxy-phénylthio)-propionaldéhyde (IIf) à la place du 2-(S)-Éthyl-3-(2-méthoxyméthoxy-3-méthyl-phénylthio)-propionaldéhyde (Ve), on obtient le composé (1-13).
Rendement : 70%
¹H RMN (DMSOd₆) δ: 0,88 (d, 3H); 0,90 (d, 3H) ; 1,46 (m, 1H) ; 1,92 (m, 2H) ; 2,65 (m, 2H) ; 2,82 (dd, 1H) ; 2,91 (m, 2H) ; 3,08 (m, 2H) ; 3,80 (s, 3H) ; 3,94 (dd, 1H) ; 4,13 (dd, 1H) ; 6,95 (m, 4H) ; 7,15 (m, 2H) ; 7,32 (m, 2H).

On dissout 0,31 g (0,0077 mole) du produit (1-13) dans 3 ml de méthanol, puis on ajoute 0,08 g (0,0069 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,32 g (0,0061 mole) du maléate du composé (1-13) sous la forme d'un solide blanc.
F : 114-5°C
[α] = - 29,6 (c = 0,361, méthanol)

| Analyse C₂₆H₃₃NO₆S₂ : | | | |
|---|---|---|---|
| Calc.% | C 60,09 | H 6,40 | N 2,70 |
| Tr. | C 60,03 | H 6,61 | N 2,83 |

¹H RMN (DMSOd₆) δ: 0,89 (d, 3H) ; 0,93 (d, 3H) ; 1,90 (1s, 1H) ; 2,02 (m, 1H) ; 2,94 (dd, 1H) ; 3,06 (m, 3H); 3,30 (m, 3H) ; 3,81 (s, 3H), 4,32 (1d, 1H) ; 4,44 (1s, 1H) ; 6,04 (s, 3H); 6,97 (m, 2H) ; 7,08 (m, 2H) ; 7,22 (m, 2H); 7,31 (dd, 1H) ; 7,41 (dd, 1H).

### Exemple de référence 14 : 3-(R)-[3-(2-Méthoxy-phénylthio)-2-(S)-méthyl-propyl]amino-7-méthyl-3,4-dihydro-2H-1,5-benzoxathiépine (1-14)

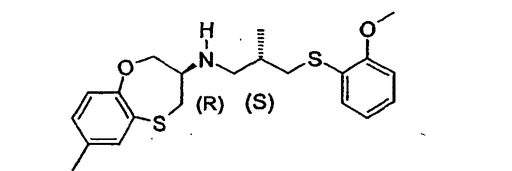

En procédant comme dans l'exemple 26, mais à partir du mélange réactionnel contenant le 2-(S)-Méthyl-3-(2-méthoxy-phénylthio)-propionaldéhyde (IIb-1) à la place du 2-(S)-Éthyl-3-(2-méthoxyméthoxy-3-méthyl-phénylthio)-propionaldéhyde (Ve) et en utilisant la 3-(R)-Amino-7-méthyl-3,4-dihydro-2H-1,5-benzoxathiépine (Ib) à la place de la 3-(R)-Amino-3,4-dihydro-2H-1,5-benzoxathiépine (Ia-1), on obtient le composé (1-14) sous la forme d'une huile incolore.
Rendement : 36%
¹H RMN (CDCl₃) δ: 1,10 (d, 3H) ; 1,68 (1s, 1H) ; 1,91 (m, 1H) ; 2,25 (s, 3H); 2,62 (dd, 1H) ; 2,77 (m, 2H) ; 2,92 (dd, 1H) ; 3,08 (m, 3H) ; 3,90 (s, 3H) ; 4,00 (dd, 1H) ; 4,20 (dd, 1H) ; 6,88 (m, 4H) ; 7,15 (m, 2H) ; 7,31 (dd, 1H).

On dissout 0,22 g (0,0056 mole) du produit (1-14) dans 3 ml de méthanol, puis on ajoute 0,065 g (0,0056 mole) d'acide maléique solubilisé dans 2 ml de méthanol. On concentre la solution obtenue, un précipité blanc se forme; il est filtré et séché sous vide à 50°C. On obtient 0,25 g (0,0049 mole) du maléate du composé (1-14) sous la forme d'un solide blanc. F : 148°C
[α] = - 12,2 (c = 0,302, méthanol)

| Analyse C₂₅H₃₁NO₆S₂ : | | | |
|---|---|---|---|
| Calc.% | C 59,38 | H 6,18 | N 2,77 |
| Tr. | C 59,63 | H 6,20 | N 2,95 |

¹H RMN (DMSOd₆) δ: 1,10 (d, 3H) ; 2,13 (m, 1H) ; 2,23 (s, 3H) ; 2,81 (dd, 1H) ; 2,97 (m, 1H) ; 3,09 (dd, 1H) ; 3,25 (m, 4H) ; 3,78 (1s, 1H) ; 3,82 (s, 3H) ; 4,24 (1d, 1H) ; 4,41 (1d, 1H) ; 6,04 (s, 2H) ; 6,99 (m, 4H); 7,19 (m, 2H) ; 7,28 (d,1H).

### Exemple de référence 15 : 3-(R)-[3-(2-Méthoxy-phénylthio)-2-(S)-méthyl-propyl]amino-6-méthyl-3,4-dihydro-2H-1,5-benzoxathiépine (1-15)

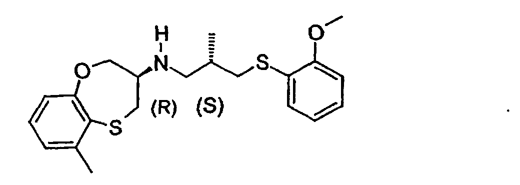

En procédant comme dans l'exemple 26, mais à partir du mélange réactionnel contenant le 2-(S)-Méthyl-3-(2-méthoxy-phénylthio)-propionaldéhyde (IIb-1) à la place du 2-(S)-Éthyl-3-(2-méthoxyméthoxy-3-méthyl-phénylthio)-propionaldéhyde (Ve) et en utilisant la 3-(R)-Amino-6-méthyl-3,4-dihydro-2H-1,5-benzoxathiépine (Ic) à la place de la 3-(R)-Amino-3,4-dihydro-2H-1,5-benzoxathiépine (Ia-1), on obtient le composé (1-15) sous la forme d'une huile incolore.
Rendement : 23%
¹H RMN (DMSOd₆) δ: 1,00 (d, 3H) ; 1,75 (m, 1H) ; 1,99 (1s, 1H) ; 2,31 (s, 3H) ; 2,53 (m, 1H) ; 2,66 (m, 2H) ; 2,84 (dd, 1H) ; 3,09 (m, 3H) ; 3,80 (s, 3H) ; 3,92 (dd, 1H) ; 4,17 (1d, 1H) ; 6,81 (d, 1H) ; 6,94 (m, 3H) ; 7,02 (t, 1H) ; 7,15 (td, 1H) ; 7,27 (d, 1H).

On dissout 0,10 g (0,0026 mole) du produit (1-15) dans 3 ml de méthanol, puis on ajoute 0,03 g (0,0026 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,11 g (0,0022 mole) du maléate du composé (1-15) sous la forme d'un solide blanc.
F : 111-3°C
[α] = + 16,3 (c = 0,214, méthanol)

| Analyse C₂₅H₃₁NO₆S₂ : | | | |
|---|---|---|---|
| Calc.% : | C 59,38 | H 6,18 | N 2,77 |
| Tr. : | C 58,74 | H 6,20 | N 3,01 |

¹H RMN (DMSOd₆) δ: 1,10 (d, 3H) ; 2,12 (1s, 1H) ; 2,33 (s, 3H) ; 2,81 (dd, 1H) ; 2,99 (1s, 1H) ; 3,09 (dd, 1H) ; 3,28 (1m, 5H) ; 3,82 (s, 3H) ; 4,39 (1m, 2H) ; 6,04 (s, 2H) ; 6,90 (d, 1H) ; 6,97 (m, 3H) ; 7,10 (t, 1H) ; 7,20 (td, 1H) ; 7,28 (dd, 1H).

### Exemple de référence 16 : 3-(R)-[3-(2,3-Dihydro-benzofuran-7-thio)-2-(S)-méthyl-propyl]amino-3,4-dihydro-2H-1,5-benzaxathiépine (1-16)

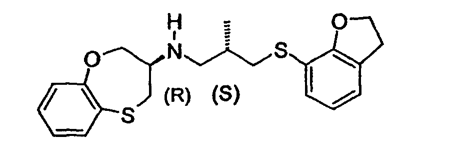

En procédant comme dans l'exemple 26, mais à partir du mélange réactionnel contenant le 2-(S)-Méthyl-3-(2,3-dihydro-benzofuran-7-thio)-propionaldéhyde (IIc) à la place du 2-(S)-Éthyl-3-(2-méthoxyméthoxy-3-méthyl-phénylthio)-propionaldéhyde (Ve), on obtient le composé (1-16), sous la forme d'une huile incolore.
Rendement : 40%
¹H RMN (DMSOd₆) δ: 0,96 (d, 3H) ; 1,70 (m, 1H) ; 1,97 (1s, 1H) ; 2,67 (m, 4H) ; 3,07 (m, 3H) ; 3,19 (t, 2H) ; 3,32 (s, 3H) ; 3,89 (dd, 1H) ; 4,14 (1d, 1H) ; 4,54 (t, 2H) ; 6,79 (t, 1H) ; 6,97 (m, 2H) ; 7,08 (m, 2H) ; 7,17 (m, 1H) ; 7,33 (d, 1H).

On dissout 0,20 g (0,0005 mole) du produit (1-16) dans 3 ml de méthanol, puis on ajoute 0,055 g (0,0005 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,15 g (0,0003 mole) du maléate du composé (1-16) sous la forme d'un solide blanc.
F : 116-8°C
[α] = + 8,2 (c = 0,291, méthanol)

| Analyse C₂₅H₂₉NO₆S₂ : | | | |
|---|---|---|---|
| Calc.% | C 59,62 | H 5,80 | N 2,78 |
| Tr. | C 59,51 | H 5,70 | N 3,06 |

¹H RMN (DMSOd₆) δ: 1,07 (d, 3H) ; 2,06 (1s, 1H) ; 2,83 (m, 1H) ; 2,95 (1s, 1H) ; 3,08 (m, 2H) ; 3,21 (t, 2H) ; 3,25 (m, 2H); 3,79 (1s, 1H) ; 4,33 (1d, 1H) ; 4,42 (1s, 1H); 4,56 (t, 2H) ; 6,04 (s, 2H) ; 6,82 (t, 1H) ; 7,09 (m, 4H) ; 7,24 (td, 1H) ; 7,40 (d, 1H),

### Exemple de référence 17 : 3-(R)-[3-(2-Hydroxy-3-méthyl-phénylthio)-2-(S)-méthyl-propyl]amino-3,4-dihydro-2H- 1,5-benzoxathiépine (1-17)

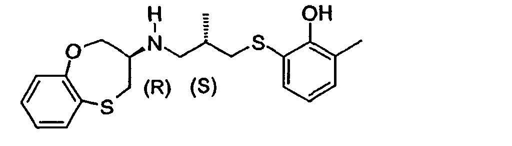

Dans un ballon de 100 ml, on introduit 0,69 g (0,0016 mole) de 3-(R)-[3-(2-Méthoxyméthoxy-3-méthyl-phényIthio)-2-(S)-méthyl-propyl]amino-3,4-dihydro-2H-1,5-benzoxathiépine (VIb), 10 ml de méthanol et 3 ml d'acide chlorhydrique (5N). Le mélange est porté à 50°C pendant 12 heures. Le méthanol est évaporé sous pression réduite puis on ajoute 3 ml d'une solution aqueuse de soude (5N) et le mélange est extrait au dichlorométhane. Les phases organiques combinées sont séchées sur sulfate de sodium, filtrées et concentrées sous pression réduite. Le résidu est purifié par chromatographie flash sur gel de silice (éluant: dichlorométhane/méthanol = 99 : 1). On récupère 0,31 g (0,0008 mole) du composé (1-17) sous la forme d'une huile incolore.
Rendement : 37%
¹H RMN (DMSOd₆) δ: 0,98 (d, 3H) ; 1,73 (m, 1H) ; 2,16 (s, 3H) ; 2,61 (m, 3H) ; 2,81 (dd, 1H) ; 2,96 (dd, 1H) ; 3,07 (m, 2H) ; 3,92 (dd, 1H) ; 4,15 (dd, 1H) ; 6,72 (t, 1H) ; 6,98 (m, 3H) ; 7,16 (m, 2H) ; 7,34 (d, 1H).

On dissout 0,31 g (0,0008 mole) du produit (1-17) dans 3 ml de méthanol, puis on ajoute 0,09 g (0,0008 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,25 g (0,0005 mole) du maléate du composé (1-17) sous la forme d'un solide blanc.
F : 124-6°C
[α] = -1,2 (c = 0,255, méthanol)

| | | | |
|---|---|---|---|
| Analyse C₂₄H₂₉NO₆S₂ : | | | |
| Calc.% : | C 59,63 | H 5,94 | N 2,85 |
| Tr. : | C 59,23 | H 5,78 | N 2,80 |

¹H RMN (DMSOd₆) δ: 1,09 (d, 3H) ; 2,07 (1s, 1H) ; 2,17 (s, 3H) ; 2,75 (dd, 1H) ; 2,98 (m, 2H) ; 3,16 (1s, 1H) ; 3,26 (m, 2H) ; 3,80 (1s, 1H) ; 4,35 (1d, 1H) ; 4,45 (1d, 1H) ; 6,04 (s, 2H); 6,76 (t, 1H) ; 7,00 (d, 1H) ; 7,06 (m, 2H); 7,16 (d, 1H) ; 7,25 (td, 1H) ; 7,39 (dd, 1H).

### Exemple de référence 18 : 3-(R)-[3-(2-Hydroxy-phénylthio)-2-(S)-méthyl-propyl]amino-3,4-dihydro-2H-1,5-benzoxathiépine (1-18)

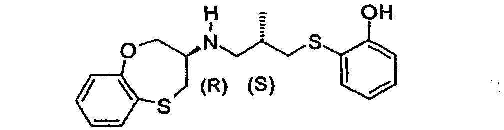

En procédant comme dans l'exemple de référence 17, mais en remplaçant le 3-(R)-[3-(2-Méthoxyméthoxy-3-métbyl-phényIthio)-2-(S)-méthyl-propyllamino-3,4-dihydro-2H-1,5-benzoxathiépine (VIb) par le 3-(R)-[3-(2-Méthoxyméthoxy-phénylthio)-2-(S)-méthyl-propyl]aminc>-3,4-dihydro-2H-1,5-benzoxathiépine (VId) on obtient le composé (1-18) sous la forme d'une huile jaune pâle.
Rendement : 95%
¹H RMN (DMSOd₆) δ: 0,99 (d, 3H) ; 1,73 (m, 1H) ; 2,59 (m, 3H) ; 2,80 (m, 1H) ; 3,05 (m, 3H) ; 3,91 (dd, 1H) ; 4,15 (1d, 1H) ; 6,78 (m, 2H) ; 6,99 (m, 3H) ; 7,19 (m, 2H) ; 7,33 (m, 1H).

On dissout 1,2 g (0,0033 mole) du produit (1-18) dans 5 ml de méthanol, puis on ajoute 0,3 g (0,0026 mole) d'acide maléique solubilisé dans 3 ml de méthanol. On concentre la solution obtenue puis on ajoute de l'éther éthylique. Le précipité formé est filtré, lavé à l'éther éthylique et séché sous vide à 50°C. On obtient 0,88 g (0,0018 mole) du maléate du composé (1-18) sous la forme d'un solide blanc.
F : 119-21°C
[α] = - 15,3 (c = 0,416, méthanol)

| Analyse C₂₃H₂₇NO₆S₂ : | | | |
|---|---|---|---|
| Calc.% : | C 57,84 | H 5,70 | N 2,93 |
| Tr. : | C 57,51 | H 5,82 | N 2,80 |

¹H RMN (DMSOd₆) δ: 1,09 (d, 3H) ; 2,1 (m, 1H) ; 2,79 (dd, 1H) ; 2,95 (m, 1H) ; 3,05 (dd, 1H) ; 3,18 (m, 1H) ; 3,29 (m, 3H) ; 3,81 (1s, 1H) ; 4,35 (1d, 1H) ; 4,45 (1d, 1H) ; 6,04 (s, 2H) ; 6,82 (m, 2H) ; 7,06 (m, 3H) ; 7,24 (m, 2H) ; 7,39 (dd, 1H).

### Exemple de référence 19 : 3-(R)-[3-(2-Hydroxy-3-éthyl-phénylthio)-2-(S)-méthyl-propyl]amino-3,4-dihydro-2H-1,5-benzoxathiépine (1-19)

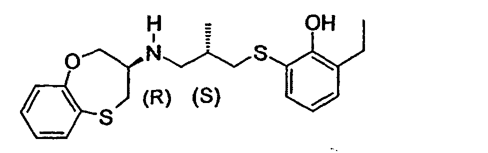

En procédant comme dans l'exemple de référence 17, mais en remplaçant le 3-(R)-[3-(2-Méthoxyméthoxy-3-méthyl-phénylthio)-2-(S)-méthyl-propyl]amino-3,4-dihydro-2H-1,5-benzoxathiépine (VIb), par le 3-(R)-[3-(2-Méthoxyméthoxy-3-éthyl-phénylthio)-2-(S)-méthyl-propyl]amino-3,4-dihydro-2H-1,5-benzoxathiépine (VIe), on obtient le composé (1-19) sous la forme d'une huile incolore,
Rendement : 33%
¹H RMN (CDCl₃) δ : 1,04 (d, 3H) ; 1,22 (t, 3H) ; 1,91 (m, 1H) ; 2,67 (q, 2H) ; 2,79 (m, 4H) ; 3,05 (m, 2H) ; 3,15 (m 1H) ; 4,02 (dd, 1H) ; 4,34 (dd, 1H) ; 6,77 (t, 1H) ; 6,98 (m, 2H) ; 7,13 (m, 2H) ; 7,32 (dd, 1H) ; 7,37 (dd, 1H).

On dissout 0,11 g (0,0003 mole) du produit (1-19) dans 3 ml de méthanol, puis on ajoute 0,033 g (0,00028 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,075 g (0,00015 mole) du maléate du composé (1-19) sous la forme d'un solide blanc.
F : 120°C [α] = + 1,4 (c = 0,280, méthanol)

| Analyse C₂₅H₃₁NO₆S₂ : | | | |
|---|---|---|---|
| Calc.% | C 59,38 | H 6,18 | N 2,77 |
| Tr. | C 59,18 | H 6,28 | N 2,68 |

¹H RMN (DMSOd₆) δ: 1,11 (m, 6H) ; 2,07 (1s, 1H) ; 2,58 (q, 2H); 2,74 (dd, 1H) ; 2,99 (m, 2H) ; 3,21 (m, 3H) ; 3,79 (1s, 1H) ; 4,34 (1d, 1H) ; 4,42 (1s, 1H) ; 6,04 (s, 2H) ; 6,80 (t, 1H) ; 7,05 (m, 3H) ; 7,17 (d, 1H); 7,24 (td, 1H) ; 7,39 (d, 1H) ; 8,56 (1s, 1H échangeable).

### Exemple de référence 20 : 3-(R)-[3-(2-Hydroxy-3-méthyl-phénylthio)-2-(S)-éthyl-propyl]amino-3,4-dihydro-2H-1,5-benzoxathiépine (1-20)

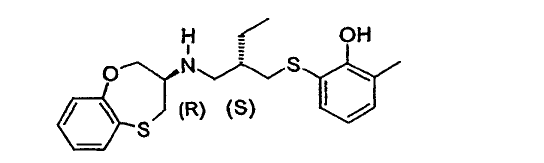

En procédant comme dans l'exemple de référence 17, mais en remplaçant le 3-(R)-[3-(2-Méthoxyméthoxy-3-méthyl-phénylthio)-2-(S)-méthyl-propyl]amino-3,4-dihydro-2H-1,5-benzoxathiépine (VIb), par le 3-(R)-[3-(2-Méthoxyméthoxy-3-méthyl-phénylthio)-2-(S)-éthyl-propyl]amino-3,4-dihydro-2H-1,5-benzoxathiépine (VIa) on obtient le composé (1-20) sous la forme d'une huile incolore.
Rendement : 91%
¹H RMN (DMSOd₆) δ : 0,85 (t, 3H) ; 1,42 (m, 2H) ; 1,57 (m, 1H); 2,16 (s, 3H) ; 2,66 (m, 2H) ; 2,85 (m, 3H) ; 3,07 (m, 2H) ; 3,94 (dd, 1H) ; 4,14 (dd, 1H) ; 6,72 (t, 1H) ; 6,98 (m, 3H) ; 7,17 (m, 2H) ; 7,34 (d, 1H).

On dissout 0,32 g (0,0008 mole) du produit (1-20) dans 3 ml de méthanol, puis on ajoute 0,09 g (0,0008 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,31 g ( 0,0006 mole) du maléate du composé (1-20) sous la forme d'un solide blanc.
F : 111-2°C
[α] = - 7,8 (c = 0,332, méthanol)

| Analyse C₂₅H₃₁NO₆S₂ : | | | |
|---|---|---|---|
| Calc.% | C 59,38 | H 6,18 | N 2,77 |
| Tr. | C 59,04 | H 6,28 | N 2,84 |

¹H RMN (DMSOd₆) δ: 0,87 (t, 3H); 1,52 (m, 2H); 1,94 (1s, 1H); 2,17 (s, 3H); 3,07 (m, 6H); 3,81 (1s, 1H); 4,34 (1d, 1H); 4,43 (1d, 1H); 6,04 (s, 2H); 6,76 (t, 1H); 7,00 (d, 1H); 7,06 (m, 2H); 7,16 (d, 1H); 7,25 (td, 1H); 7,40 (d, 1H); 8,64 (1s, H échangeables)

### Exemple de référence 21 : 3-(R)-[3-(2-Hydroxy-3-méthyl-phénylthio)-2-(S)-iso-propyl-propyl]amino-3,4-dihydro-2H-1,5-benizoxathiépine (1-21)

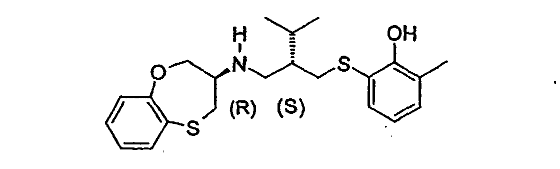

En procédant comme dans l'exemple de référence 17, mais en remplaçant le 3-(R)-[3-(2-Méthoxyméthoxy-3-méthyl-phénylthio)-2-(S)-méthyl-propyl]amino-3,4-dihydro-2H-1,5-benzoxathiépine (VIb), par le 3-(R)-[3-(2-Méthoxyméthoxy-3-méthyl-phénylthio)-2-(S)-iso-propyl-propyl]amino-3,4-dihydro-2H-1,5-benzoxathiépine (VIc) on obtient le composé (1-21) qui n'est pas purifié à ce stade mais salifié directement.
Rendement brut : 84%

On dissout 0,083 g (0,0002 mole) du produit (1-21) dans 3 ml de méthanol, puis on ajoute 0,024 g (0,0002 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,08 g ( 0,00015 mole) du maléate du composé (1-21) sous la forme d'un solide blanc.
F : 127°C
[α] = - 29,4 (c = 0,211, méthanol)

| Analyse C₂₆H₃₃NO₆S₂ : | | | |
|---|---|---|---|
| Calc.% | C 60,09 | H 6,40 | N 2,70 |
| Tr. | C 59,85 | H 6,43 | N 2,77 |

¹H RMN (DMSOd₆) δ: 0,85 (d, 3H) ; 0,90 (d, 3H) ; 1,85 (1s, 1H) ; 2,01 (1s, 1H) ; 2,17 (s, 3H) ; 2,86 (dd, 1H) ; 2,96 (dd, 1H) ; 3,10 (1s, 1H) ; 3,29 (1s, 3H) ; 3,83 (1s, 1H) ; 4,34 (d, 1H) ; 4,44 (1s, 1H) ; 6,04 (s, 2H) ; 6,76 (t, 1H) ; 7,00 (d, 1H) ; 7,07 (m, 2H) ; 7,17 (d, 1H) ; 7,25 (t, 1H) ; 7,40 (d, 1H) ; 8,65 (1s, H échangeables).

### Exemple de référence 22 : 3-(R)-[3-(2-Hydroxy-3-méthyl-phénylthio)-2-(S)-méthyl-propyl]amino-6-méthyl-3,4-dihydro-2H-1,5-benzoxathiépine (1-22)

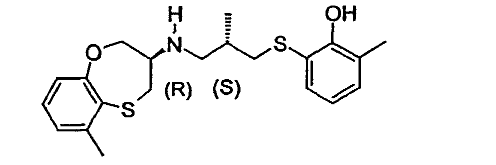

En procédant comme dans l'exemple de référence 17, mais en remplaçant le 3-(R)-[3-(2-Méthoxyméthoxy 3-méthyl-phénylthio)-2-(S)-méthyl-propyl]amino-3,4-dihydro-2H-1,5-benzoxathiépine (VIb), par le 3-(R)-[3-(2-Méthoxyméthoxy-3-mëthyl-phénylthio)-2-(S)-méthyl-propyl]amino-6-méthyl-3,4-dihydro-2H-1,5-benzoxathiépine (VIg) on obtient le composé (1-22) sous la forme d'une huile incolore.
Rendement : 95%
¹H RMN (DMSOd₆) δ : 0,98 (d, 3H) ; 1,74 (m, 1H) ; 2,16 (s, 3H) ; 2,31 (s, 3H) ; 2,55 (m, 1H) ; 2,41 (m, 2H) ; 2,86 (m, 1H) ; 2,96 (dd, 1H) ; 3,11 (m, 2H) ; 3,94 (1s, 1H) ; 4,17 (1d, 1H) ; 6,72 (t, 1H) ; 6,81 (d, 1H) ; 6,92 (d, 1H) ; 6,97 (d, 1H) ; 7,03 (t, 1H) ; 7,14 (d, 1H).

On dissout 0,315 g (0,0008 mole) du produit (1-22) dans 3 ml de méthanol, puis on ajoute 0,084 g (0,0007 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,35 g ( 0,0007 mole) du maléate du composé (1-22) sous la forme d'un solide blanc.
F : 108-9°C
[α] = + 13,4 (c = 0,209, méthanol)

| Analyse C₂₅H₃₁NO₆S₂ : | | | |
|---|---|---|---|
| Calc.% | C 59,38 | H 6,18 | N 2,77 |
| Tr. | C 59,38 | H 6,26 | N 3,00 |

¹H RMN (DMSOd₆) δ: 1,09 (d, 3H) ; 2,06 (m, 1H) ; 2,17 (s, 3H) ; 2,33 (s, 3H) ; 2,75 (dd, 1H) ; 2,99 (m, 2H) ; 3,16 (1s, 1H) ; 3,27 (m, 3H) ; 3,79 (1s, 1H) ; 4,39 (m, 2H) ; 6,04 (s, 2H) ; 6,76 (t, 1H) ; 6,90 (d, 1H) ; 7,00 (m, 2H) ; 7,10 (t, 1H) ; 7,16 (d, 1H).

### Exemple de référence 23 : 3-(R)-[3-(2-Hydroxy-3-méthoxy-phénylthio)-2-(S)-méthyl-propyl]amino-3,4-dihydro-2H-1,5-benzoxathiépine (1-23)

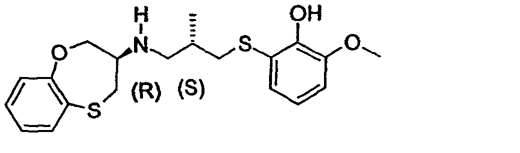

En procédant comme dans l'exemple de référence 17, mais en remplaçant le 3-(R)-[3-(2-Méthoxyméthoxy-3-méthyl-phénylthio)-2-(S)-méthyl-propyl]amino-3,4-dihydro-2H-1,5-benzoxathiépine (VIb), par le 3-(R)-[3-(2-Méthoxyméthoxy-3-méthoxy-phénylthio)-2-(S)-méthyl-propyl]amino-3,4-dihydro-2H-1,5-benzoxathiépine (Vli). On obtient le composé (1-23) qui n'est pas purifié à ce stade mais salifié directement.
Rendement brut: 73%

On dissout 0,13 g (0,0003 mole) du produit (1-23) dans 3 ml de méthanol, puis on ajoute 0,035 g (0,0003 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,103 g ( 0,0002 mole) du maléate du composé (1-23) sous la forme d'un solide blanc.
F : 137-9°C
[α] = - 11,6 (c = 0,268, méthanol)

| Analyse C₂₄H₂₉NO₇S₂ : | | | |
|---|---|---|---|
| Calc.% | C 56,78 | H 5,76 | N 2,76 |
| Tr. | C 56,82 | H 5,85 | N 2,89 |

¹H RMN (DMSOd₆) δ: 1,08 (d, 3H) ; 2,07 (m, 1H) ; 2,79 (dd, 1H) ; 2,96 (m, 1H) ; 3,04 (dd, 1H) ; 3,17 (m, 1H) ; 3,27 (m, 3H) ; 3,79 (s, 3H) ; 4,35 (1d, 1H); 4,44 (1d, 1H) ; 6,04 (s, 2H) ; 6,77 (m, 1H); 6,86 (m, 2H) ; 7,06 (m, 2H); 7,24 (m, 1H) ; 7,39 (d, 1H) ; 8,79 (1s, échangeable) ; 9,02 (1s, échangeable).

### Exemple de référence 24 : 3-(R)-[3-(2,3-Diméthoxy-phénylthio)-2-(S)-méthyl-propyl]amino-3,4-dihydro-2H-1,5-benzoxathiépine (1-24)

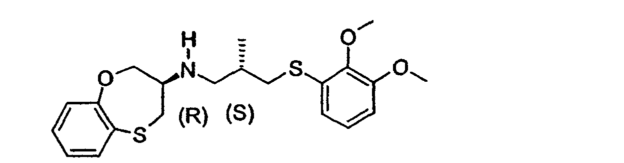

En procédant comme dans l'exemple 26, mais à partir du mélange réactionnel contenant le 2-(S)-Méthyl-3-(2,3-diméthoxy-phénylthio)-propionaldéhyde (IIg) à la place du 2-(S)-Éthyl-3-(2-méthoxyméthoxy-3-méthyl-phénylthio )-propionaldéhyde (Ve), on obtient le composé (1-24),
Rendement : 37%
¹H RMN (CDCl₃) δ: 1,00 (d, 3H) ; 1,78 (m, 1H) ; 2,02 (1s, 1H) ; 2,62 (m, 3H); 2,81 (dd, 1H) ; 3,08 (m, 3H) ; 3,70 (s, 3H) ; 3,78 (s, 3H) ; 3,92 (dd, 1H) ; 4,17 (dd, 1H) ; 6,86 (m, 2H) ; 7,01 (m, 3H) ; 7,17 (m, 1H) ; 7,33 (d, 1H).

On dissout 0,22 g (0,0005 mole) du produit (1-24) dans 3 ml de méthanol, puis on ajoute 0,057 g (0,0005 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,175 g ( 0,0003 mole) du maléate du composé (1-24) sous la forme d'un solide blanc.
F : 117-9°C
[α] = + 1 (c = 0,270, méthanol)

| Analyse C₂₅H₃₁NO₇S₂ : | | | |
|---|---|---|---|
| Calc.% | C 57,56 | H 5,99 | N 2,68 |
| Tr. | C 57,16 | H 5,82 | N 2,92 |

¹H RMN (DMSOd₆) δ: 1,11 (d, 3H) ; 2,14 (1s, 1H) ; 2,82 (dd, 1H) ; 2,98 (1s, 1H) ; 3,11 (dd, 1H) ; 3,17 (1s, 1H) ; 3,30 (m, 3H) ; 3,72 (s, 3H) ; 3,79 (s, 3H) ; 4,34 (1d, 1H) ; 4,40 (1s, 1H) ; 6,89 (m, 2H) ; 7,06 (m, 3H) ; 7,25 (m, 1H) ; 7,40 (dd, 1H) ; 8,78 (1s, H échangeables).

### Exemple de référence 25 : 3-(R)-[3-(2-Hydroxy-3-iso-propyl-phénylthio)-2-(S)-méthyl-propyl]amino-3,4-dihydro-2H-1,5-benzoxathiépine (1-25)

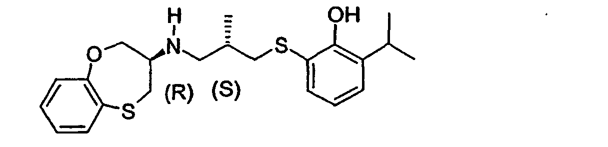

En procédant comme dans l'exemple de référence 17, mais en remplaçant le 3-(R)-[3-(2-Méthoxyméthoxy-3-méthyl-phénylthio)-2-(S)-méthyl-propyl]amino-3,4-dihydro-2H-1,5-benzoxathiépine (VIb), par le 3-(R)-[3-(2-Méthoxyméthoxy-3-iso-propyl-phénylthio)-2-(S)-méthyl-propyl]amino-3,4-dihydro-2H-1,5-benzoxathiépine (VIh), on obtient le composé (1-25) sous la forme d'une huile incolore.
Rendement : 68%
¹H RMN (CDCl₃) δ : 1,05 (d, 3H) ; 1,23 (d, 6H) ; 1,55 (1s, H échangeables) ; 1,91 (m, 1H); 2,76 (m, 3H); 2,86 (dd, 1H); 3,04 (m, 2H); 3,14 (1s, 1H); 3,32 (m, 1H); 4,02 (dd, 1H); 4,33 (dd, 1H); 6,80 (m, 1H); 6,97 (m, 2H); 7,15 (m, 2H); 7,34 (m, 2H).

On dissout 0,124 g (0,0003 mole) du produit (1-25) dans 3 ml de méthanol, puis on ajoute 0,036 g (0,0003 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,155 g ( 0,0003 mole) du maléate du composé (1-25) sous la forme d'un solide blanc.
F : 126°C
[α] = + 4,1 (c = 0,245, méthanol)

| Analyse C₂₆H₃₃NO₆S₂ : | | | |
|---|---|---|---|
| Calc.% | C 60,09 | H 6,40 | N 2,69 |
| Tr. | C 59,70 | H 6,33 | N 2,72 |

¹H RMN (DMSOd₆) δ: 1,09 (d, 3H) ; 1,15 (d, 6H) ; 2,08 (m, 1H) ; 2,75 (dd, 1H) ; 2,98 (m, 2H) ; 3,16 (1s, 1H) ; 3,27 (m, 4H) ; 3,78 (1s, 1H); 4,34 (1d, 1H) ; 4,43 (1d, 1H) ; 6,04 (s, 2H) ; 6,84 (t, 1H) ; 7,06 (m, 3H) ; 7,18 (d, 1H) ; 7,24 (t, 1H) ; 7,39 (d, 1H) ; 8,52 (1s, échangeable) ; 8,76 (1s, échangeable).

### Exemple de référence 26 : 3-(R)-[3-(2-Hydroxy-6-méthyl-phénylthio)-2-(S)-méthyl-propyl]amino-3,4-dihydro-2H-1,5-benzoxathiépine (1-26)

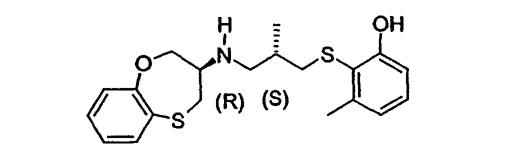

En procédant comme dans l'exemple de référence 17, mais en remplaçant le 3-(R)-[3-(2-Méthoxyméthoxy-3-méthyl-phénylthio)-2-(S)-méthyl-propyl]amino-3,4-dihydro-2H-1,5 benzoxathiépine (VIb), par le 3-(R)-[3-(2-Méthoxyméthoxy-6-méthyl-phénylthio)-2-(S)-méthyl-propyl]amino-3,4-dihydro-2H-1,5-benzoxathiépine (VIj), on obtient le composé (1-26) sous la forme d'une huile incolore.
Rendement : 88%
¹H RMN (DMSOd₆) δ : 0,95 (d, 3H) ; 1,61 (m,1H) ; 2,40 (s, 3H) ; 2,60 (m, 2H) ; 2,77 (dd, 1H) ; 2,88 (dd, 1H) ; 3,00 (m, ZH) ; 3,88 (dd, 1H) ; 4,11 (dd,1H) ; 6,71 (m, 2H) ; 6,99 (m, 3H) ; 7,17 (m, 1H) ; 7,33 (dd, 1H).

On dissout 0,39 g (0,001 mole) du produit (1-26) dans 3 ml de méthanol, puis on ajoute 0,105 g (0,0009 mole) d'acide maléique solubilisé dans 2 ml de méthanol. On concentre la solution obtenue puis on ajoute de l'éther éthylique. Le précipité formé est filtré, lavé à l'éther éthylique et séché sous vide à 50°C. On obtient 0,37 g ( 0,0007 mole) du maléate du composé (1-26) sous la forme d'un solide blanc.
F : 153-4°C
[α] = 0 (c = 0,2, méthanol)

| Analyse C₂₄H₂₉NO₆S₂ : | | | |
|---|---|---|---|
| Calc.% | C 58,63 | H 5,95 | N 2,85 |
| Tr. | C 58,53 | H 5,97 | N 3,04 |

¹H RMN (DMSOd₆) δ: 1,06 (d, 3H) ; 1,96 (m, 1H) ; 2,42 (s, 3H) ; 2,71 (dd, 1H) ; 2,91 (dd, 2H) ; 3,15 (m, 1H) ; 3,24 (1s, 2H) ; 3,76 (1s, 1H) ; 4,36 (m, 2H) ; 6,04 (s, 2H) ; 6,74 (d, 2H) ; 7,05 (m, 3H) ; 7,24 (m, 1H) ; 7,39 (dd, 1H).

Les composés de formule (1) ainsi que leurs sels thérapeutiquement acceptables présentent des propriétés pharmacologiques intéressantes, notamment des propriétés cytoprotectrices cardiaques.

En effet, ils sont actifs au niveau du cardiomyocite en inhibant la contracture de l'oreillette gauche isolée du rat induite par la vératrine. Il est, en effet, admis que la vératrine retarde l'inactivation du canal sodique et engendre un courant sodique persistant qui reproduit la surcharge sodique observée lors de l'ischémie. Ce test pharmacologique est réalisé selon la technique décrite dans Naunyn-Schmiedeberg's Arch. Pharmacol. 1993, 348, 184 selon le protocole qui suit.

On utilise des rats mâles Wistar (OFA, Iffa Credo, France) pesant 400-450 g. Les animaux sont placés en quarantaine de 4 à 8 jours avec un accès libre à la nourriture standardisée de laboratoire avant leur utilisation dans les expérimentations. Les animaux sont hébergés individuellement 24 heures avant les tests. Grâce à un distributeur automatique, de l'eau filtrée à 0,22 µm, est disponible à volonté. La zone de quarantaine et le laboratoire d'expérimentation sont climatisés (température : 20 ± 3°C; degré hygrométrique: 55 ± 5%) et éclairés de 7 à 19 heures. Tous les rats sont traités suivant l'éthique des animaux de laboratoire (Guide for the Care and Use of Laboratory Animals, U.S. Department of Agriculture. Public Health Service. National Institutes of Health publication N° 85-23, Revised 1985), et le protocole (n°31) est réalisé en accord avec les recommandations du comité d'éthique local des animaux de recherche.

Les animaux sont sacrifiés au moyen d'une dose létale de pentobarbital sodium (50 mg/kg) administrée par voie intrapéritonéale. Le thorax est ouvert et l'oreillette gauche rapidement excisée et montée, en position verticale, dans une cuve à organe contenant 20 ml de liquide de Krebs (NaCl, 119 mmole ; KCl, 5,6 mmole ; MgSO₄, 1.17 mmole ; CaCl₂, 2,1 mmole ; NaH₂PO₄, 1 mmole ; NaHCO₃, 25 mmole ; glucose, 10 mmole ; pH = 7,4). Le bain est maintenu à une température constante de 34°C avec bullage en continu d'un mélange O₂ / CO₂ (95:5). L'oreillette est stimulée au moyen d'un courant électrique d'une fréquence de 4 Hz (durée de l'impulsion 1 ms) à l'aide de deux électrodes (Campden, Stimulator 915, Phymep, Paris, France). La force de contraction est mesurée à l'aide d'un capteur (Statham ; UC2). L'amplificateur est connecté à une interface MP 100, Biopac Systems, Goleta, CA, USA et le signal analogique est numérisé simultanément et analysé (Acknowledge III, Biopac Systems). Après 30 min de retour à l'équilibre, une concentration du produit à tester ou du véhicule est introduite dans la cuve à organe. Quinze minutes après l'introduction du produit ou du véhicule, la vératrine (100 µg/ml) est ajoutée. La tension systolique développée est mesurée avant l'introduction du produit ou du véhicule et juste avant l'addition de vératrine, de manière à détecter tout effet inotrope négatif ou positif du produit ou du véhicule. L'amplitude maximum de la contracture induite par la vératrine est mesurée indépendamment du temps. Le produit à tester est dissous dans du DMSO en quantité suffisante pour obtenir une solution mère de concentration égale à 10 mmole. Cette solution mère est ensuite diluée jusqu'à la concentration désirée en produit à tester au moyen du liquide de Krebs. La plus forte concentration en DMSO *in fine* est de 0,1%.

L'analyse statistique des résultats intergroupes (produit versus véhicule) est conduite par une analyse de variance ANOVA suivie par un test de Dunett.

L'activité cytoprotectrice des composés de l'invention a également été mise en évicence *in vivo* dans un modèle d'occlusion-reperfusion chez l'animal anesthésié.

Ainsi, les composés de l'invention sont capables de normaliser les perturbations électriques de l'ECG provoquées par une ischémie régionale suivie d'une reperfusion et cela, sans effet notable sur les paramètres hémodynamiques. Le test en question est réalisé selon la technique décrite dans J. Cardiovasc. Pharmacol. 1995, 25, 126 selon la protocole qui suit.

On utilise des lapins mâles New-Zealand (élevage des Dombes, Romans, Chatillon sur Chalaronne, France) pesant 2,2 à 2,7 kg. Les animaux sont placés en quarantaine de 4 à 8 jours avec un accès libre à la nourriture standardisée de laboratoire avant leur utilisation dans les expérimentations. Les animaux sont hébergés individuellement. Grâce à un distributeur automatique, de l'eau filtrée à 0,22 µm, est disponible à volonté. La zone de quarantaine et le laboratoire d'expérimentation sont climatisés (température: 20 ± 3°C ; degré hygrométrique: 55 ± 5%) et éclairés de 7 heures à 19 heures. Tous les animaux sont traités suivant l'éthique des animaux de laboratoire (Guide for the Care and Use of Laboratory Animals, U.S. Department of Agriculture. Public Health Service. National Institutes of Health publication N° 85-23, Revised 1985), et le protocole (n°28) est réalisé en accord avec les recommandations du comité d'éthique local des animaux de recherche. Les animaux sont anesthésiés au moyen de pentobarbital sodium (60 mg/kg) administré par voie intraveineuse (i.v.), par l'intermédiaire d'un cathéter disposé au niveau de la veine de l'oreille. Les animaux sont instrumentés sous assistance respiratoire (683 rodent/small animal ventilator, Havard Apparatus, Les Ulis, France). Le mélange gazeux inhalé est enrichi en oxygène. Le rythme respiratoire, le volume courant ainsi que le pourcentage d'oxygène dans le mélange gazeux sont ajustés de manière à maintenir les gaz dans le sang dans des limites physiologiques. Un cathéter en polyéthylène, introduit dans l'artère carotide, est utilisé à la fois pour les mesures de pression artérielle et pour prélever les échantillons destinés à l'analyse des gaz du sang (ABL 510, Radiometer, Copenhagen, Denmark). L'anesthésie est maintenue par injections de pentobarbital sodium en fonction des besoins. La température corporelle des animaux est maintenue pendant toute la durée de l'expérience à 38-39°C au moyen d'une couverture chauffante (Homothermie Blanket, Haverd apparatus). Les différents cathéters sont rincés au moyen d'une solution saline (0,9%), stérile, contenant de l'héparine (150 U.I/ml). L'ECG (dérivation DII) est enregistré afin de mesurer les variations de la fréquence cardiaque (intervalle RR) et de l'amplitude du segment ST. La pression artérielle est numérisée et analysée simultanément (Dataffow®, Crystal Biotech, Northboro, MA). Le thorax de l'animal est ouvert au niveau du quatrième espace intercostal et le péricarde incisé de manière à laisser apparaître l'artère coronaire gauche. Une ligature (Vicryl®, 5/0, Ethicon, Paris, France) est passée sous cette artère. Après examen de l'ECG, pour déceler tous signes de lésions myocardiques (élévation persistante du segment ST au-delà de 0,25 mV), une période de stabilisation de 30 min est systématiquement observée. Tout animal montrant une possible lésion myocardique est exclu de l'étude. Le composé à tester ou le véhicule est administré *per os* (p.o.), en solution dans de la méthylcellulose à 1% à raison de 1 ml/kg, par l'intermédiaire d'un tube gastrique en caoutchouc souple. L'anesthésie est effectuée 6C min après administration du composé à tester ou du véhicule. L'artère coronaire principale est alors ligaturée pendant 10 min, soit 60 min après l'anesthésie, la tension de la ligature est ensuite relâchée totalement pendant 10 min puis rétablie à la fin de la procédure. Le coeur est excisé et perfusé au moyen d'une solution de formaldéhyde (10%). La surface non fixée par le formaldéhyde est considérée comme la surface à risque. Les paramètres mesurés dans l'expérience sont :
- la pression artérielle systolique et diastolique ;
- la fréquence cardiaque (mesurée à partir des intervalles RR) ;
- l'amplitude du segment ST.

Tous les paramètres mentionnés ci-dessus sont mesurés pré-occlusion, 5 min et 10 min post-occlusion puis 5 min, 10 min et 20 min après la reperfusion.

Les résultats obtenus pour certains composés de formule (1), donnés à titre d'exemples non limitatifs, ainsi que ceux obtenus pour le dérivé R 56865 (bloqueur des canaux sodiques non-inactivés), l'Atenolol (β-bloquant) et le Diltiazem (bloqueur des canaux calciques), choisis comme produit de référence, sont rapportés dans le tableau ci-après:

| **Composé ou témoin** | **Contracture à la vératrine Inhibition IC**_{**50**} | **Segment ST % inhibition à 2.5 mg/kg p.o.** | **Pression artérielle % variation** | **Fréquence cardiaque % variation** |
|---|---|---|---|---|
| **1-1** | 0,64 | 85 | 8 | 2 |
| **1-7** | 0,14 | 69 | 5 | 0 |
| R 56865 | 0,25 | 0 | - | - |
| Aténolol | >10 | 49 | -9 | -13 |
| Diltiazem | >10 | 30 | -27 | -5 |

Les résultats des essais montrent donc que les composés de formule (1) :
- s'opposent au courant sodique persistant induit par la vératrine;
- tendent à normaliser les perturbations électriques de l'ECG provoquées par une ischémie régionale suivie d'une reperfusion.

L'activité *in vitro* de ces composés de l'invention est du même ordre de grandeur que celle du produit R 56865, l'Atenolol et le Diltiazem étant inactifs dans ce test.

L'activité *in vivo* de ces composés de l'invention est très supérieure à celle de l'ensemble des produits témoins (R 56865, Atenolol et Diltiazem). De plus, il est à remarquer qu'à la dose de 2,5 mg/kg, administrée par voie orale, ces produits de l'invention inhibent efficacement la surélévation du segment ST sans modifier de façon significative la fréquence cardiaque et la pression artérielle, contrairement aux produits témoins actifs dans ce modèle (Atenolol et Diltiazem).

Les molécules de l'invention s'opposent donc à la surcharge sodique en interagissant spécifiquement au niveau du canal sodique non-inactivé. Ils montrent une activité cardioprotectrice *in vivo* en l'absence d'effet hémodynamique.

De ce fait, les composés de l'invention ainsi que leurs sels thérapeutiquement acceptables sont potentiellement utiles comme médicaments, en particulier dans le domaine de la cardiologie, notamment dans le traitement de certaines pathologies cardio-vasculaires telles que, par exemple, l'ischémie cardiaque, l'angor stable, l'angor instable, l'insuffisance cardiaque, l'infarctus du myocarde, les troubles du rythme cardiaque, le syndrome du QT long d'origine congénitale.

Les composés de l'invention pouvant être également dotés d'une activité modulatrice des canaux sodiques neuronaux suffisante et possédant des propriétés pharmacocinétiques appropriées peuvent être actifs au niveau du système nerveux central et/ou périphérique. En conséquence, certains composés de l'invention sont considérés comme pouvant être également utiles dans le traitement des maladies ou des désordres tels que, par exemple, l'ischémie cérébrale, l'attaque ischémique transitoire, les neuropathies d'ordre traumatique ou ischémique, les maladies neurodégénératives (Trends in Pharmacological Science 1995, 16, 309; Clin. Exp. Pharmacol. Physiol. 2000, 27(8), 569), l'épilepsie et les douleurs d'origine neuropathique (Brain Res. 2000, 871(1), 98).

L'administration des composés de l'invention peut être réalisée par voie orale, nasale, sublinguale, rectale ou parentérale. A titre d'exemples de formulation non limitatifs, deux préparations des composés de l'invention sont données, ci-après. Les ingrédients ainsi que d'autres, thérapeutiquement acceptables, peuvent être introduits en d'autres proportions sans modifier la portée de l'invention. Les termes 'ingrédient actif' utilisés dans les exemples de formulation ci-après font références à un composé de formule (1) ou un sel d'addition ou éventuellement un hydrate d'un sel d'addition du composé de formule (1) avec un acide minéral ou un acide organique pharmaceutiquement acceptable.

### Exemple de formulation 1 : comprimés

| | |
|---|---|
| Ingrédient actif | 100 g |
| Lactose | 570 g |
| Amidon de maïs | 200 g |
| Lauryl sulfate de sodium | 5 g |
| Polyvinylpyrrolidone | 10 g |
| Cellulose microcristalline | 100 g |
| Huile végétale saturée | 15 g |

soit 10 000 comprimés, comprenant chacun 10 mg de l'ingrédient actif

### Exemple de formulation 2 : solution injectable

| | |
|---|---|
| Ingrédient actif | 10 mg |
| Acide acétique | 20 mg |
| Acétate de sodium | 5,9 mg |
| eau distillée stérile q.s.p | 2 ml |
| flacon ou ampoule stérile. | |

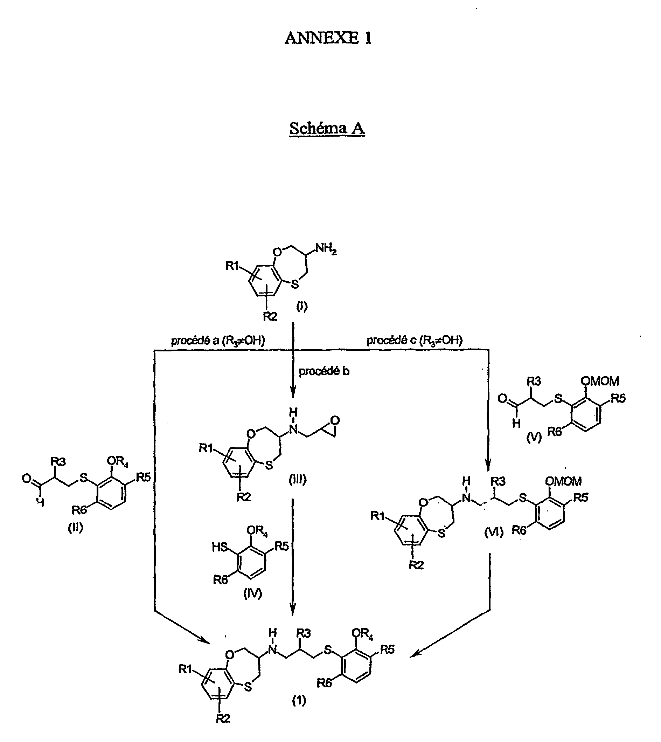

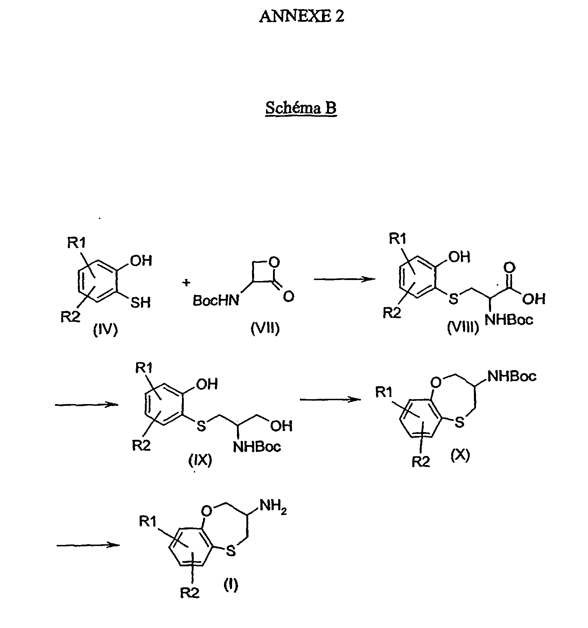

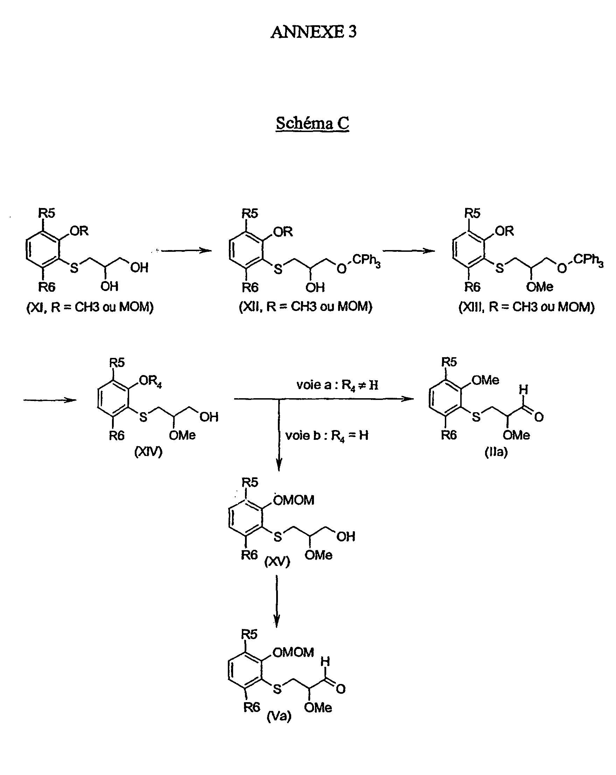

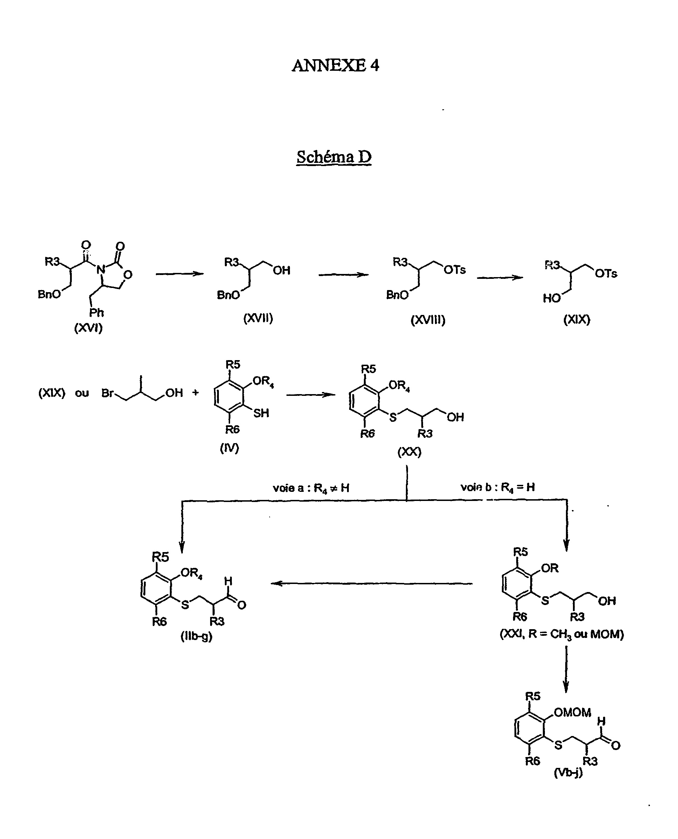

## Revendications

1. Dérivés de la 3-arylthio-propyl-amino-3,4-dihydro-2H-1,5-aryloxathiépine de formule générale (1) 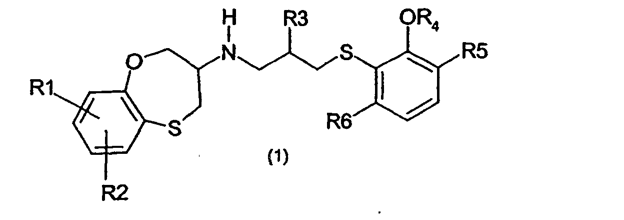 dans laquelle
R₁ et R₂, identiques ou différents, représentent :
- un atome d'hydrogène ;
- un atome de fluor ou un atome de chlore ;
- un groupe hydroxy ;
- un radical alkyle, ramifié ou non, renfermant de 1 à 3 atomes de carbone;
- un radical cyclopropyle ;
- un radical cyclopropoxy ou
- lorsque les groupes R₁ et R₂ occupent des positions adjacentes sur le cycle aromatique, alors ils forment avec les atomes de carbone qui les portent un cycle carboné ou un hétérocycle oxygéné à cinq chaînons non aromatique ;
R₃ représente :
- un radical alkyle, ramifié ou non, renfermant de 1 à 3 atomes de carbone ;
- un groupe hydroxy ou un radical méthoxy ;
R₄ représente :
- un atome d'hydrogène ou un radical méthyle et
R₅ et R₆ identiques ou différents représentent :
- un atome d'hydrogène ;
- un radical alkyle, ramifié ou non, renfermant de 1 à 3 atomes de carbone ;
- un radical alcoxy ramifié ou non, renfermant de 1 à 3 atomes de carbone ;
- un radical alkylthio ramifié ou non, renfermant de 1 à 3 atomes de carbone ;
- un radical alkylamino,
pourvu que lorsque R₄ représente un radical méthyle alors R₅ représente un atome d'hydrogène, un radical alcoxy renfermant de 1 à 3 atomes de carbone, un radical alkylthio ramifié ou non renfermant de 1 à 3 atomes de carbone ou un radical alkylamino, ou
les groupes OR₄ et R₅ forment avec les carbones qui les portent un hétérocycle non aromatique à cinq ou six chaînons contenant au moins un atome d'oxygène, et R₆ est tel que défini précédemment,
leurs sels d'addition et les hydrates de ces sels d'addition avec les acides minéraux ou les acides organiques pharmaceutiquement acceptables,
ainsi que leurs formes tautomères, les énantiomères et les mélanges d'énantiomères, et les stéréoisomères purs ou en mélange racémique ou non.

2. Dérivés selon la revendication 1 **caractérisés en ce que**
R₁ et R₂, identiques ou différents, représentent :
- un atome d'hydrogène ;
- un atome de fluor ou un atome de chlore ;
- un groupe hydroxy ;
- un radical alkyle choisi dans le groupe comprenant les radicaux méthyle, éthyle, propyle et isopropyle ;
- un radical cyclopropyle ;
- un radical alcoxy choisi dans le groupe comprenant les radicaux méthoxy, éthoxy, propoxy et isopropoxy ;
- un radical cyclopropoxy ou
- lorsque les groupes R₁ et R₂ occupent des positions adjacentes sur le cycle aromatique, alors R₁R₂ représentent -CH₂CH₂CH₂-, -OCH₂CH₂-, -OCH₂O- ou -CH₂CH₂O- ;
R₃ représente :
- un radical alkyle choisi dans le groupe comprenant les radicaux méthyle, éthyle, propyle et isopropyle ;
- un groupe hydroxy ou un radical méthoxy ;
R₄ représente :
- un atome d'hydrogène ou un radical méthyle et
R₅ et R₆ identiques ou différents représentent :
- un atome d'hydrogène ;
- un radical alkyle choisi dans le groupe comprenant les radicaux méthyle, éthyle et isopropyle ;
- un radical alcoxy choisi dans le groupe comprenant les radicaux méthoxy, éthoxy, propoxy et isopropoxy ;
- un radical alkylthio choisi dans le groupe comprenant les radicaux méthylthio, éthylthio et isopropylthio ;
- un radical alkylamino choisi dans le groupe comprenant les radicaux N-méthylamino et N,N-diméthylamino; ou
R₄R₅ représente un radical choisi dans le groupe comprenant -CH₂CH₂-, -CH₂O-, -CH₂CH₂O-, -CH₂CH₂S-, et -CH₂CH₂NR₄-, et R₆ est tel que défini précédemment,
leurs sels d'addition et les hydrates de ces sels d'addition avec les acides minéraux ou les acides organiques pharmaceutiquement acceptables,
ainsi que leurs formes tautomères, les énantiomères et les mélanges d'énantiomères, et les stéréoisomères purs ou en mélange racémique ou non.

3. Dérivés selon la revendication 1, **caractérisé en ce qu'**ils sont choisis parmi les composés suivants :
3-[3-(2-Méthoxy-phénylthio)-2-méthoxy-propyl]amino-3,4-dihydro-2H-1,5-benzoxathiépine ;
3-[3-(2-Méthoxy-phénylthio)-2-hydroxy-propyl] amino-3,4-dihydro-2H-1,5-benzoxathiépine ;
3-[3-(2-Hydroxy-phénylthio)-2-hydroxy-propyl] amino-3,4-dihydro-2H-1,5-benzoxathiépine;
3-[3-(2-Méthoxy-phénylthio)-2-méthyl-propyl]amino-3,4-dihydro-2H-1,5-benzoxathiépine ;
3-[3-(2-Méthoxy phénylthio)-2-éthyl-propyl]amino-3,4-dihydro-2H-1,5-benzoxathiépine ;
3-[3-(2-Méthoxy phénylthio)-2-n-propyl-propyl]amino-3,4-dihydro-2H-1,5-benzoxathiépine ;
3-[3-(2-Méthoxy-phénylthio)-2-iso-propyl-propyl]amino-3,4-dihydro-2H-1,5-benzoxathiépine ;
3-[3-(2-Méthoxy phénylthio)-2-méthyl-propyl]amino-7-méthyl-3,4-dihydro-2H-1,5-benzoxathiépine ;
3-[3-(2-Méthoxy-phénylthio)-2-méthyl-propyl]amino-6-méthyl-3,4-dihydro-2H-1,5-benzoxathiépine ;
3-[3-(2-Hydroxy-3-méthyl-phénylthio)-2-méthyl-propyl]amino-3,4-dihydro-2H-1,5-benzoxathiépine ;
3-[3-(2-Hydroxy phénylthio)-2-méthyl-propyl]amino-3,4-dihydro-2H-1,5-benzoxathiépine ;
3-[3-(2-Hydroxy-3-éthyl-phénylthio)-2-méthyl-propyl]amino-3,4-dihydro-2H-1,5-benzoxathiépine ;
3-[3-(2,3-Dihydro-benzofuran-7-thio)-2-methyl-propyl]amino-3,4-dihydro-2H-1,5-benzoxathiépine ;
3-[3-(2-Hydroxy-3-méthyl-phénylthio)-2-éthy]-propyl]amino-3,4-dihydro-2H-1,5-benzoxathiépine ;
3-[3-(2-Hydroxy-3-méthyl-phénylthio)-2-iso-propyl-propyl]amino-3,4-dihydro-2H-1,5-benzoxathiépine ;
3-[3-(2-Hydroxy-3-méthyl-phénylthio)-2-méthyl-propyl]amino-6-méthyl-3,4-dihydro-2H-1,5-benzoxathiépine ;
3-[3-(2-Hydroxy 3-méthoxy-phénylthio)-2-méthyl-propyl]amino-3,4-dihydro-2H-1,5-benzoxathiépine ;
3-[3-(2,3-Diméthoxy-phénylthio)-2-méthyl-propyl]amino-3,4-dihydro-2H-1,5-benzoxathiépine ;
3-[3-(2-Hydroxy-3-iso-propyl-phénylthio)-2-méthyl-propyl]amino-3,4-dihydro-2H-1,5-benzoxathiépine ;
3-[3-(2-Hydroxy-6-méthyl-phénylthio)-2-méthyl-propyl]amino-3,4-dihydro-2H-1,5-benzoxathiépine,
leurs sels d'addition et les hydrates de ces sels d'addition avec les acides minéraux ou les acides organiques pharmaceutiquement acceptables,
ainsi que leurs formes tautomères, les énantiomères et les mélanges d'énantiomères, et les stéréoisomères purs ou en mélange racémique ou non.

4. Procédé de préparation des composés de formule générale (1) selon l'une quelconque des revendications 1 à 3 dans lesquels R₃ représente un radical alkyle ramifié renfermant de 1 à 3 atomes de carbone ou un radical méthoxy et R₄ représente un radical méthyle, **caractérisé en ce que** l'on fait réagir une amine de formule (I) 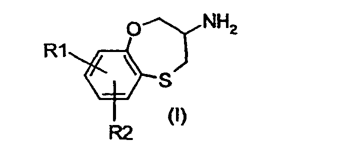 dans laquelle R₁, R₂ sont tels que définis dans la revendication 1, ou un de ces sels, avec un aldéhyde de formule (II) 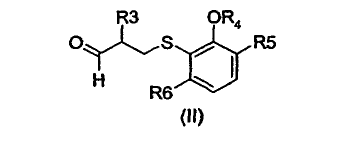 dans laquelle R₃ représente un radical alkyle ramifié renfermant de 1 à 3 atomes de carbone ou un radical méthoxy, R₄ représente un radical méthyle et R₅ et R₆ sont tels que définis dans la revendication 1
en présence d'un agent réducteur et à une température comprise entre -20°C et +25°C,
pour donner un composé de formule (1) .

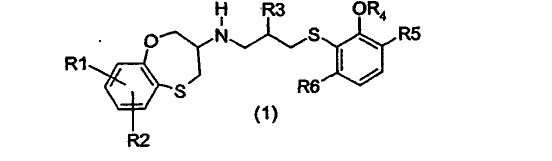

5. Procédé de préparation des composés de formule générale (1) selon l'une quelconque des revendications 1 à 3 dans lesquels R₃ représente un radical alkyle ramifié renfermant de 1 à 3 atomes de carbone ou un radical méthoxy et R₄ représente un atome d'hydrogène, **caractérisé en ce que** l'on fait réagir une amine de formule (I) 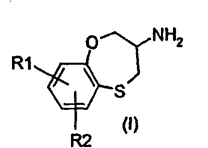 dans laquelle R₁, R₂ sont tels que définis dans la revendication 1, ou un de ces sels, avec un composé de formule (V) 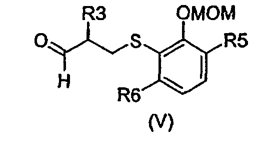 dans laquelle R₃ lesquels R₃ représente un radical alkyle ramifié renfermant de 1 à 3 atomes de carbone ou un radical méthoxy et R₅ et R₆ sont tels que définis dans la revendication 1 en présence d'un agent réducteur et à une température comprise entre - 20°C et +25°C,
pour donner un composé de formule (VI) 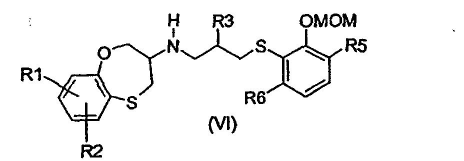 que l'on hydrolyse pour donner un composé de formule (1) 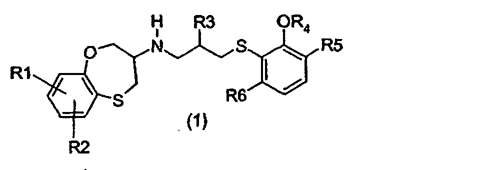 dans laquelle lesquels R₃ représente un radical alkyle ramifié renfermant de 1 à 3 atomes de carbone ou un radical méthoxy et R₄ un atome d'hydrogène.

6. Procédé de préparation des composés de formule générale (1), selon les revendications 1 et 2 dans lesquels R₃ est un groupe hydroxy, **caractérisé en ce que** l'on fait réagir un époxyde de formule (III) 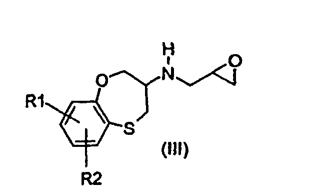 dans laquelle R₁ et R₂ sont tels que définis dans la revendication 1,
avec un arylthiol de formule (IV) 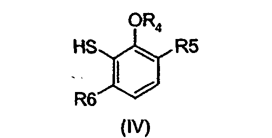 dans laquelle R₄, R₅ et R₆ sont tels que définis dans la revendication 1,
dans un solvant protique, en présence d'une base inorganique et à une température comprise entre 20°C et 70°C,
pour donner un composé de formule (1) 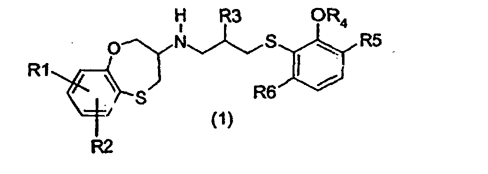 dans laquelle R₁, R₂, R₄, R₅ et R₆ sont tels que définis dans la revendication 1 et R₃ est un groupe hydroxy.

7. Dérivés de formule générale (1), selon l'une quelconque des revendications 1 ou 2, **caractérisés en ce qu'**ils sont de configuration absolue (R) au niveau de l'atome de carbone asymétrique C(3) du fragment 3,4-dihydro-2H-1,5-benoxathièpine et de configuration absolue (S) au niveau de l'atome de carbone asymétrique qui porte le groupe R₃.

8. Dérivé de formule générale (1) selon la revendication 7, **caractérisé en ce qu'**il est choisi parmi les stéréoisomères suivants :
3-(R)-[3-(2-Méthoxy-phénylthio)-2-(S)-méthoxy-propyl]amino-3,4-dihydro-2H-1,5-benzoxathiépine ;
3-(R)-[3-(2-Méthoxy-phénylthio)-2-(S)-hydroxy-propyl]amino-3,4-dihydro-2H-1,5-benzoxathiépine;
3-(R)-[3-(2-Hydroxy-phénylthio)-2-(S)-hydroxy-propyl]amino-3,4-dihydro-2H-1,5-benzoxathiépine ;
3-(R)-[3-(2-Méthoxy-phénylthio)-2-(S)-méthyl-propyl]amino-3,4-dihydro-2H-1,5-benzoxathiépine ;
3-(R)-[3-(2-Méthoxy-phénylthio)-2-(S)-éthyl-propyl]amino-3,4-dihydro-2H-1,5-benzoxathiépine ;
3-(R)-[3-(2-Méthoxy-phénylthio)-2-(S)-n-propyl-propyl]amino-3,4-dihydro-2H-1,5-benzoxathiépine ;
3-(R)-[3-(2-Méthoxy-phénylthio)-2-(S)-iso-propyl-propyl]amino-3,4-dihydro-2H-1,5-benzoxathiépine ;
3-(R)-[3-(2-Methoxy-phénylthio)-2-(S)-méthyl-propyl]amino-7-méthyl-3,4-dihydro-2H-1,5-benzoxathiépine ;
3-(R)-[3-(2-Méthoxy-phénylthio)-2-(S)-méthyl-propyl]amino-6-méthyl-3,4-dihydro-2H- 1,5-benzoxathiépine ;
3-(R)-[3-(2-Hydroxy-3-méthyl-phénylthio)-2-(S)-méthyl-propyl]amino-3,4-dihydro-2H-1,5-benzoxathiépine;
3-(R)-[3-(2-Hydroxy-phénylthio)-2-(S)-méthyl-propyl]amino-3,4-dihydro-2H-1,5-benzoxathiépine ;
3-(R)-[3-(2-Hydroxy-3-éthyl-phénylthio)-2-(S)-méthyl-propyl]amino-3,4-dihydro-2H-1,5-benzoxathiëpine ;
3-(R)-[3-(2,3-Dihydro-benzofuran-7-thio)-2-(S)-méthyl-propyl]amino-3,4-dihydro-2H-1,5-benzoxathiépine ;
3-(R)-[3-(2-Hydroxy-3-méthyl-phénylthio)-2-(S)-éthyl-propyl]amino-3,4-dihydro-2H-1,5-benzoxathiépine ;
3-(R)-[3-(2-Hydroxy-3-méthyl-phénylthio)-2-(S)-iso-propyl -propyl]amino-3,4-dihydro-2H-1,5-benzoxathiépine ;
3-(R)-[3-(2-Hydroxy-3-méthyl-phénylthio)-2-(S)-méthyl-propyl]amino-6-méthyl-3,4-dihydro-2H-1,5-benzoxathiépine ;
3-(R)-[3-(2-Hydroxy-3-méthoxy-phénylthio)-2-(S)-méthyl-propyl]amino-3,4-dihydro-2H-1,5 benzoxathiépine ;
3-(R)-[3-(2,3-Diméthoxy-phénylthio)-2-(S)-méthyl-propyl]amino-3,4-dihydro-2H-1,5-benzoxathiépine ;
3-(R)[3-(2-Hydroxy-3-iso-propyl-phénylthio)-2-(S)-méthyl-propyl]amino-3,4-dihydro-2H-1,5-benzoxathiépine ;
3-(R)-[3-(2-Hydroxy-6-méthyl-phénylthio)-2-(S)-méthyl-propyl]amino-3,4-dihydro-2H-1,5-benzoxathiépine,
leurs sels d'addition et les hydrates de ces sels d'addition avec les acides minéraux ou les acides organiques pharmaceutiquement acceptables,
ainsi que leurs formes tautomères, les énantiomères et les mélanges d'énantiomères, et les stéréoisomères purs ou en mélange racémique ou non.

9. Amines de formule (I) 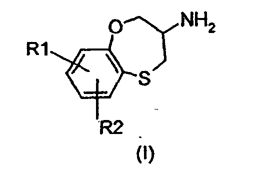 dans laquelle
R₁ et R₂, identiques ou différents, représentent :
- un atome d'hydrogène ;
- un atome de fluor ou un atome de chlore ;
- un groupe hydroxy ;
- un radical alkyle, ramifié ou non, renfermant de 1 à 3 atomes de carbone ;
- un radical cyclopropyle ;
- un radical alcoxy ramifié ou non, renfermant de 1 à 3 atomes de carbone ;
- un radical cyclopropoxy ou
- lorsque les groupes R₁ et R₂ occupent des positions adjacentes sur le cycle aromatique, ils forment avec les atomes de carbone qui les portent un cycle carboné ou un hétérocycle oxygéné à cinq chaînons non aromatique;
comme intermédiaires dans la synthèse des composés de formule (1).

10. Amines selon la revendication 9 dans laquelle l'atome de carbone asymétrique C(3) est de configuration absolue (R).

11. Procédé de préparation des composés de formule (I) selon l'une quelconque des revendications 9 et 10, **caractérisé en ce qu'**on transforme la N-Boc-(2-hydroxyphényl)-cystéine de formule (VIII) 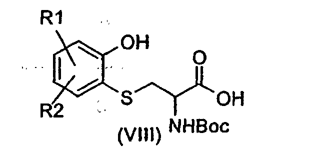 en alcool primaire de formule (IX) 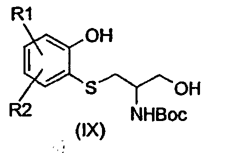 par réduction d'un anhydride mixte intermédiaire, formé *in situ,* au moyen d'un hydrure de bore simple ou complexe selon une technique "*one-pot*",
puis on cyclise ledit composé (IX), pour obtenir le composé cyclique correspondant (X) 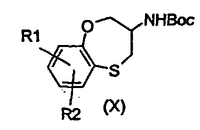 que l'on traite par un acide protique pour obtenir l'amine de formule (I) que l'on salifie si on le souhaite.

12. Aldéhydes de formule (II) 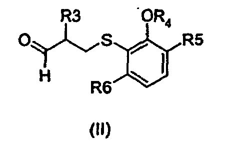 dans laquelle
R₃ représente
- un radical alkyle, ramifié ou non, renfermant de 1 à 3 atomes de carbone ou un radical méthoxy ,
R₄ représente :
- un atome d'hydrogène ou un radical méthyle et
R₅ et R₆ identiques ou différents représentent :
- un atome d'hydrogène ;
- un radical alkyle, ramifié ou non, renfermant de 1 à 3 atomes de carbone ;
- un radical alcoxy ramifié ou non, renfermant de 1 à 3 atomes de carbone ;
- un radical alkylthio ramifié ou non renfermant de 1 à 3 atomes de carbone ;
- un radical alkylamino,
pourvu que lorsque R₄ représente un radical méthyle alors R₅ représente un atome d'hydrogène, un radical alcoxy renfermant de 1 à 3 atomes de carbone, un radical alkylthio ramifié ou non renfermant de 1 à 3 atomes de carbone ou un radical alkylamino, ou
les groupes OR₄ et R₅ forment avec les carbones qui les portent un hétérocycle non aromatique à cinq ou six chaînons contenant au moins un atome d'oxygène, et R₆ est tel que défini précédemment,
utiles comme intermédiaires dans la synthèse des composés de formule (1).

13. Aldéhydes selon la revendication 12 dans lesquels l'atome de carbone asymétrique porteur du groupe R₃ est de configuration absolue (S).

14. Aldéhydes de formule (V) 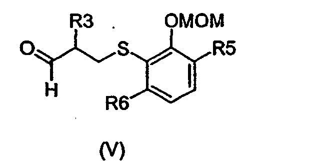 dans laquelle
R₃ représente
- un radical alkyle, ramifié ou non, renfermant de 1 à 3 atomes de carbone ou un radical méthoxy et
R₅ et R₆ identiques ou différents représentent :
- un atome d'hydrogène ;
- un radical alkyle, ramifié ou non, renfermant de 1 à 3 atomes de carbone ;
- un radical alcoxy ramifié ou non, renfermant de 1 à 3 atomes de carbone ;
- un radical alkylthio ramifié ou non renfermant de 1 à 3 atomes de carbone ;
- un radical alkylamino,
utiles comme intermédiaires dans la synthèse des composés de formule (1).

15. Aldéhydes selon la revendication 14 dans lesquels l'atome de carbone asymétrique porteur du groupe R₃ est de configuration absolue (S).

16. Procédé de préparation des composés de formule (IIa) selon la revendication 12 dans laquelle R₃ représente un groupe méthoxy et des composés de formule (Va) selon la revendication 13 dans laquelle R₃ représente un groupe méthoxy, **caractérisé en ce que** la fonction alcool primaire de l'intermédiaire du type 3-arylthio-1,2-propanediol (XI) 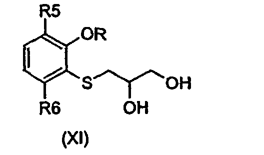 dans laquelle R représente un radical méthyle ou méthoxyméthyle et R₅ et R₆ sont tels que définis dans la revendication 1 est protégée sous forme d'éther tritylique de formule (XII), 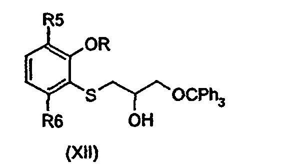 activée sous la forme d'un alcoolate alcalin, puis méthylée au moyen d'un halogénure ou du sulfate de méthyle pour donner le composé de formule (XIII) 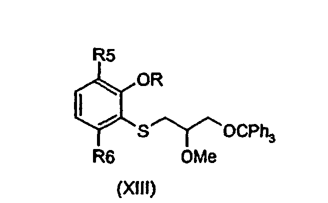 dans laquelle R est tel que défini précédemment et on libère la fonction alcool primaire par hydrolyse du groupement triphénylméthyle en milieu acide protique et on obtient,
- lorsque R est un radical méthyle, un composé de formule (XIVa1) 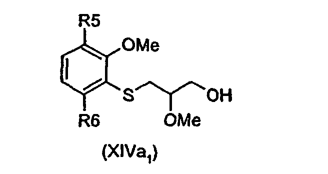 dont on oxyde l'alcool primaire et on obtient un composé de formule (IIa) 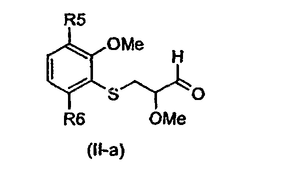
- lorsque R est un radical méthoxyméthyle, on obtient un composé de formule (XIVa2) 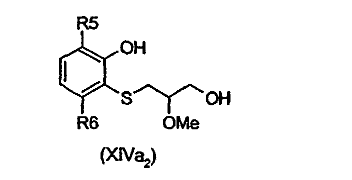 dont on protège la fonction phénol par alkylation chimio-sélective au moyen de chlorométhylméthyl éther et on obtient un composé de formule (XV) 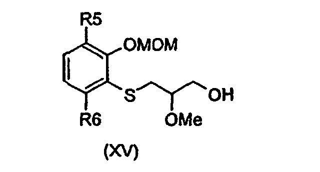 dont on oxyde l'alcool primaire et on obtient un composé de formule (Va)

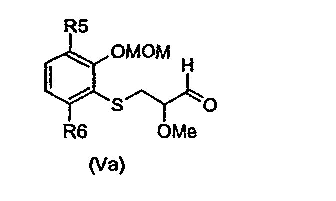

17. Procédé de préparation des composés de formule (In selon la revendication 12 et des composés de formule (V) selon la revendication 13 dans lesquelles R₃ représente un radical alkyle **caractérisé en ce que**
- soit on réduit un composé de formule (XVI) 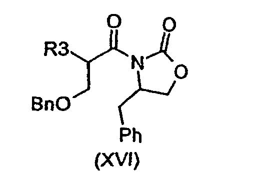
dans laquelle R₃ représente un radical alkyle, ramifié ou non, renfermant de 1 à 3 atomes de carbone,
et on obtient un alcool de formule (XVII) 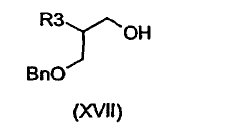 que l'on transforme en un ester de l'acide p-toluènesulfonique (tosylate) de formule (XVIII) 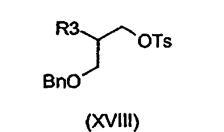 que l'on soumet à une hydrogénolyse en présence d'un catalyseur au palladium pour donner le composé (XIX) 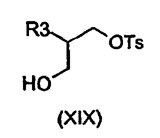 que l'on fait réagir avec un composé de formule (IV), éventuellement sous forme de sel alcalin, 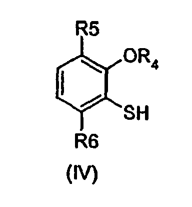 dans laquelle
R₄ représente :
- un atome d'hydrogène ou un radical méthyle et
R₅ et R₆ identiques ou différents représentent :
- un atome d'hydrogène ;
- un radical alkyle, ramifié ou non, renfermant de 1 à 3 atomes de carbone ;
- un radical alcoxy ramifié ou non, renfermant de 1 à 3 atomes de carbone ;
- un radical alkylthio ramifié ou non renfermant de 1 à 3 atomes de carbone ;
- un radical alkylamino,
pourvu que lorsque R₄ représente un radical méthyle alors R₅ représente un atome d'hydrogène, un radical alcoxy renfermant de 1 à 3 atomes de carbone, un radical alkylthio ramifié ou non renfermant de 1 à 3 atomes de carbone ou un radical alkylamino, ou
les groupes OR₄ et R₅ forment avec les carbones qui les portent un hétérocycle non aromatique à cinq ou six chaînons contenant au moins un atome d'oxygène, et R₆ est tel que défini précédemment,
- soit on fait réagir le 3-Bromo-2-méthyl-propan-1-ol avec un composé de formule (IV)
et on obtient un composé de formule (XX) 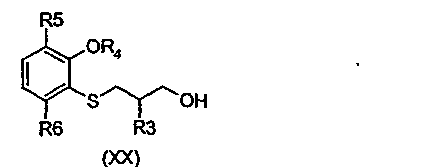 puis, lorsque R₄ est un radical alkyle ou forme avec le groupe R₅ adjacent un hétérocycle, on réalise directement une oxydation du composé de formule (XX) en aldéhyde de formule (II) 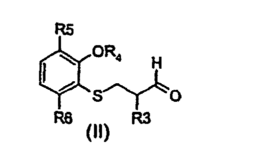 lorsque R₄ est un atome d'hydrogène, alors la fonction phénol du composé de formule (XX) est convertie en composé de formule (XXI) 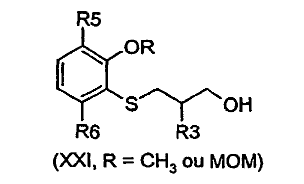 dans laquelle R représente un radical méthoxyméthyle (XXIa, R = MOM), ou un radical méthyle (XXIb, R = CH₃),
puis l'alcool (XXIa) est oxydé en aldéhyde (II) 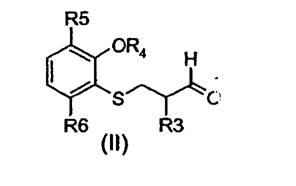 et l'alcool (XXIb) en aldéhyde (V)

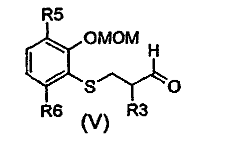

18. Composés selon l'une des revendications 1, 2, 3, 7 et 8 à titre de médicaments.

19. Compositions pharmaceutiques **caractérisées en ce qu'**elles contiennent comme ingrédient actif au moins un composé selon l'une des revendications 1, 2, 3, 7 et 8 associé à un support pharmaceutique inerte ou autres véhicules pharmaceutiquement acceptables et éventuellement à un autre médicament.

20. Compositions pharmaceutiques selon la revendication 19 utiles dans le traitement de l'angor stable, l'angor instable, l'insuffisance cardiaque, le syndrome du QT long d'origine congénitale, l'infarctus du myocarde et les troubles du rythme cardiaque.

21. Compositions pharmaceutiques selon la revendication 19 utiles dans le traitement de l'ischémie cérébrale, l'attaque ischémique transitoire, les neuropathies d'ordre traumatique ou ischémique et l'épilepsie.

22. Compositions pharmaceutiques selon la revendication 19 utiles dans le traitement des douleurs d'origine neuropathique et des maladies neurodégénératives.

## Patentansprüche

1. Derivate von 3-Arylthiopropylamino-3,4-dihydro-2H-1,5-aryloxathiepin mit der allgemeinen Formel (1) 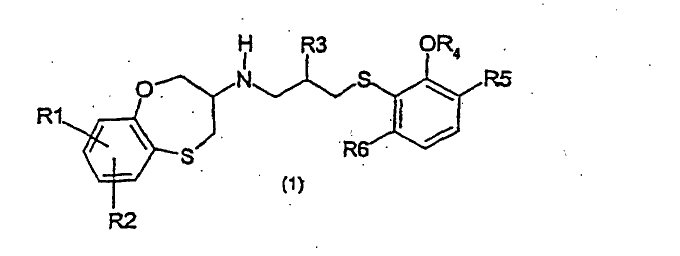 in der
R₁ und R₂, identisch oder verschieden:
- ein Wasserstoffatom;
- ein Fluoratom oder ein Chloratom;
- eine Hydroxygruppe;
- einen Alkylrest, verzweigt oder nicht, der 1 bis 3 Kohlenstoffatome einschließt;
- einen Cyclopropylrest;
- einen Cyclopropoxyrest darstellen oder,
- wenn die Gruppen R₁ und R₂ benachbarte Positionen an dem aromatischen Ring einnehmen, sie dann mit den Kohlenstoffatomen, welche sie tragen, einen Kohlenstoffcyclus oder einen nicht-aromatischen sauerstoffhaltigen Heterocyclus mit fünf Ringgliedem bilden;
R₃:
- einen Alkylrest, verzweigt oder nicht, der 1 bis 3 Kohlenstoffatome einschließt;
- eine Hydroxygruppe oder einen Methoxyrest darstellt;
R₄:
- ein Wasserstoffatom oder einen Methylrest darstellt und
R₅ und R₆, identisch oder verschieden:
- ein Wasserstoffatom;
- einen Alkylrest, verzweigt oder nicht, der 1 bis 3 Kohlenstoffatome einschließt;
- einen Alkoxyrest, verzweigt oder nicht, der 1 bis 3 Kohlenstoffatome einschließt;
- einen Alkylthiorest, verzweigt oder nicht, der 1 bis 3 Kohlenstoffatome einschließt;
- einen Alkylaminorest darstellen,
mit der Maßgabe, dass, wenn R₄ einen Methylrest darstellt, dann R₅ ein Wasserstoffatom, einen Alkoxyrest, der 1 bis 3 Kohlenstoffatome einschließt, einen Alkylthiorest, verzweigt oder nicht, der 1 bis 3 Kohlenstoffatome einschließt, oder einen Alkylaminorest darstellt oder
die Gruppen OR₄ und R₅ mit den Kohlenstoffen, welche sie tragen, einen nicht-aromatischen Heterocyclus mit fünf oder sechs Ringgliedern bilden, der mindestens ein Sauerstoffatom enthält, und R₆ wie vorstehend definiert ist, deren Additionssalze und die Hydrate dieser Additionssalze mit pharmazeutisch annehmbaren Mineralsäuren oder organischen Säuren,
sowie deren tautomere Formen, die Enantiomere und die Mischungen von Enantiomeren und die reinen Stereoisomere entweder in racemischer Mischung oder nicht.

2. Derivate nach Anspruch 1, **dadurch gekennzeichnet, dass**
R₁ und R₂, identisch oder verschieden:
- ein Wasserstoffatom;
- ein Fluoratom oder ein Chloratom;
- eine Hydroxygruppe;
- einen Alkylrest, der aus der Gruppe ausgewählt ist, welche die Reste Methyl, Ethyl, Propyl und Isopropyl umfasst;
- einen Cyclopropylrest;
- einen Alkoxyrest, der aus der Gruppe ausgewählt ist, welche die Reste Methoxy, Ethoxy, Propoxy und Isopropoxy umfasst;
- einen Cyclopropoxyrest darstellen oder,
- wenn die Gruppen R₁ und R₂ benachbarte Positionen an dem aromatischen Ring einnehmen, dann R₁R₂ -CH₂CH₂CH₂-, -OCH₂CH₂-, -OCH₂O- oder -CH₂CH₂O- darstellt;
R₃:
- einen Alkylrest, der aus der Gruppe ausgewählt ist, welche die Reste Methyl, Ethyl, Propyl und lsopropyl umfasst;
- eine Hydroxygruppe oder einen Methoxyrest darstellt;
R₄:
- ein Wasserstoffatom oder einen Methylrest darstellt und
R₅ und R₆, identisch oder verschieden:
- ein Wasserstoffatom;
- einen Alkylrest, der aus der Gruppe ausgewählt ist, welche die Reste Methyl, Ethyl und Isopropyl umfasst;
- einen Alkoxyrest, der aus der Gruppe ausgewählt ist, welche die Reste Methoxy, Ethoxy, Propoxy und lsopropoxy umfasst;
- einen Alkylthiorest, der aus der Gruppe ausgewählt ist, welche die Reste Methylthio, Ethylthio und Isopropylthio umfasst;
- einen Alkylaminorest, der aus der Gruppe ausgewählt ist, welche die Reste N-Methylamino und N,N-Dimethylamino umfasst, darstellen; oder R₄R₅ einen Rest darstellen, der aus der Gruppe ausgewählt ist, welche -CH₂CH₂-, -CH₂O-, -CH₂CH₂O-, -CH₂CH₂S- und -CH₂CH₂NR₄- umfasst, und
R₆ wie vorstehend definiert ist,
deren Additionssalze und die Hydrate dieser Additionssalze mit pharmazeutisch annehmbaren Mineralsäuren oder organischen Säuren,
sowie deren tautomere Formen, die Enantiomere und die Mischungen von Enantiomeren und die reinen Stereoisomere entweder in racemischer Mischung oder nicht.

3. Derivate nach Anspruch 1, **dadurch gekennzeichnet, dass** sie aus den folgenden Verbindungen ausgewählt sind:
3-[3-(2-Methoxyphenylthio )-2-methoxypropyl]amino-3,4-dihydro-2H-1,5-benzoxathiepin;
3-[3-(2-Methoxyphenylthio)-2-hydroxypropyl]amino-3,4-dihydro-2H-1,5-benzoxathiepin;
3-[3-(2-Hydroxyphenylthio)-2-hydroxypropyl]amino-3,4-dihydro-2H-1,5-benzoxathiepin;
3-[3-(2-Methoxyphenylthio)-2-methylpropyl]amino-3,4-dihydro-2H-1,5-benzoxathiepin;
3-[3-(2-Methoxyphenylthio)-2-ethylpropyl]amino-3,4-dihydro-2H-1,5-benzoxathiepin;
3-[3-(2-Methoxyphenylthio)-2-n-propylpropyl]amino-3,4-dihydro-2H-1,5-benzoxathiepin;
3-[3-(2-Methoxyphenylthio)-2-isopropylpropyl]amino-3,4-dihydro-2H-1,5-benzoxathiepin;
3-[3-(2-Methoxyphenylthio)-2-methylpropyl]amino-7-methyl-3,4-dihydro-2H-1,5-benzoxathiepin;
3-[3-(2-Methoxyphenylthio)-2-methylpropyl]amino-6-methyl-3,4-dihydro-2H-1,5-benzoxathiepin;
3-[3-(2-Hydroxy-3-methylphenylthio)-2-methylpropyl]amino-3,4-dihydro-2H-1,5-benzoxathiepin;
3-[3-(2-Hydroxyphenylthio)-2-methylpropyl]amino-3,4-dihydro-2H-1,5-benzoxathiepin;
3-[3-(2-Hydroxy-3-ethylphenylthio)-2-methylpropyl]amino-3,4-dihydro-2H-1,5-benzoxathiepin;
3-[3-(2,3-Dihydrobenzofuran-7-thio)-2-methylpropyl]amino-3,4-dihydro-2H-1,5-benzoxathiepin;
3-[3-(2-Hydroxy-3-methylphenylthio)-2-ethylpropyl]amino-3,4-dihydro-2H-1,5-benzoxathiepin;
3-[3-(2-Hydroxy-3-methylphenylthio)-2-isopropylpropyl]amino-3,4-dihydro-2H-1,5-benzoxathiepin;
3-[3-(2-Hydroxy-3-methylphenylthio)-2-methylpropyl]amino-6-methyl-3,4-dihydro-2H-1,5-benzoxathiepin;
3-[3-(2-Hydroxy-3-methoxyphenylthio)-2-methylpropyl]amino-3,4-dihydro-2H-1,5-benzoxathiepin;
3-[3-(2,3-Dimethoxyphenylthio)-2-methylpropyl]amino-3,4-dihydro-2H-1,5-benzoxathiepin;
3-[3-(2-Hydroxy-3-isopropylphenylthio)-2-methylpropyl]amino-3,4-dihydro-2H-1,5-benzoxathiepin;
3-[3-(2-Hydroxy-6-methylphenylthio)-2-methylpropyl]amino-3,4-dihydro-2H-1,5-benzoxathiepin,
deren Additionssalzen und den Hydraten dieser Additionssalze mit pharmazeutisch annehmbaren Mineralsäuren oder organischen Säuren,
sowie deren tautomeren Formen, den Enantiomeren und den Mischungen von Enantiomeren und den reinen Stereoisomeren entweder in racemischer Mischung oder nicht.

4. Verfahren zur Herstellung von Verbindungen der allgemeinen Formel (1) nach irgendeinem der Ansprüche 1 bis 3, in denen R₃ einen verzweigten Alkylrest, der 1 bis 3 Kohlenstoffatome einschließt, oder einen Methoxyrest darstellt und R₄ einen Methylrest darstellt, **dadurch gekennzeichnet, dass** man ein Amin der Formel (I) 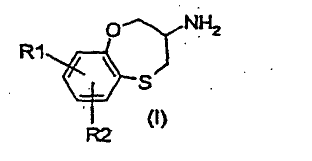 in der R₁, R₂ wie in Anspruch 1 definiert sind, oder eines seiner Salze mit einem Aldehyd der Formel (II) 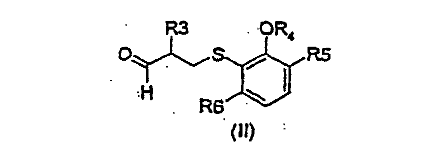 in der R₃ einen verzweigten Alkylrest, der 1 bis 3 Kohlenstoffatome einschließt, oder einen Methoxyrest darstellt, R₄ einen Methylrest darstellt und R₅ und R₆ wie in Anspruch 1 definiert sind,
in Anwesenheit eines Reduktionsmittels bei einer Temperatur von -20°C bis +25°C reagieren lässt,
um eine Verbindung der Formel (1) zu ergeben.

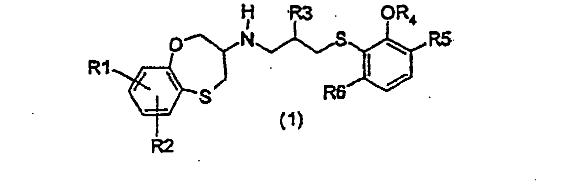

5. Verfahren zur Herstellung von Verbindungen der allgemeinen Formel (1) nach irgendeinem der Ansprüche 1 bis 3, in denen R₃ einen verzweigten Alkylrest, der 1 bis 3 Kohlenstoffatome einschließt, oder einen Methoxyrest darstellt und R₄ ein Wasserstoffatom darstellt, **dadurch gekennzeichnet, dass** man ein Amin der Formel (I) 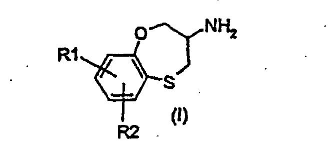 in der R₁, R₂ wie in Anspruch 1 definiert sind, oder eines seiner Salze mit einer Verbindung der Formel (V) 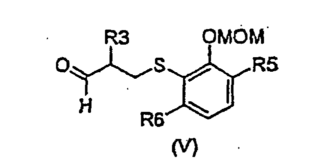 in der R₃ einen verzweigten Alkylrest, der 1 bis 3 Kohlenstoffatome einschließt, oder einen Methoxyrest darstellt und R₅ und R₆ wie in Anspruch 1 definiert sind, in Anwesenheit eines Reduktionsmittels bei einer Temperatur von -20°C bis +25°C reagieren lässt,
um eine Verbindung der Formel (VI) zu ergeben, 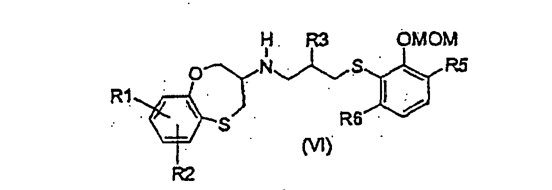 welche man hydrolysiert, um eine Verbindung der Formel (1) zu ergeben 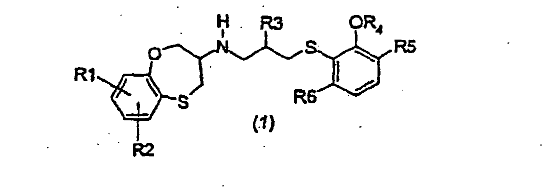 in der R₃ einen verzweigten Alkylrest, der 1 bis 3 Kohlenstoffatome einschließt, oder einen Methoxyrest darstellt und R₄ ein Wasserstoffatom ist.

6. Verfahren zur Herstellung von Verbindungen der allgemeinen Formel (1) nach den Ansprüchen 1 und 2, in denen R₃ eine Hydroxygruppe ist, **dadurch gekennzeichnet, dass** man ein Epoxid der Formel (III) 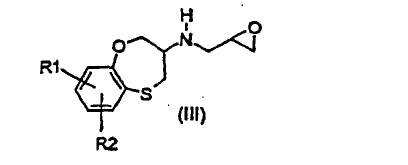 in der R₁ und R₂ wie in Anspruch 1 definiert sind,
mit einem Arylthiol der Formel (IV) 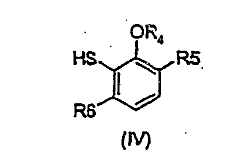 in der R₄, R₅ und R₆ wie in Anspruch 1 definiert sind,
in einem protischen Lösungsmittel in Anwesenheit einer anorganischen Base und bei einer Temperatur von 20°C bis 70°C reagieren lässt,
um eine Verbindung der Formel (1) zu ergeben 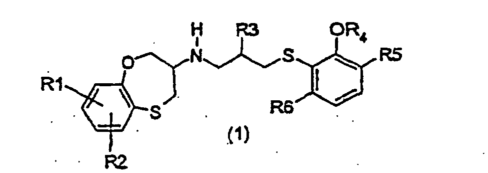 in der R₁, R₂, R₄, R₅ und R₆ wie in Anspruch 1 definiert sind und R₃ eine Hydroxygruppe ist.

7. Derivate der allgemeinen Formel (1) nach irgendeinem der Ansprüche 1 oder 2, **dadurch gekennzeichnet, dass** sie am asymmetrischen Kohlenstoffatom C(3) des Fragments 3,4-Dihydro-2H-1,5-benzoxathiepin die absolute Konfiguration (R) und am asymmetrischen Kohlenstoffatom, welches die Gruppe R₃ trägt, die absolute Konfiguration (S) aufweisen.

8. Derivate der allgemeinen Formel (1) nach Anspruch 7, **dadurch gekennzeichnet, dass** sie aus den folgenden Stereoisomeren ausgewählt sind:
3-(R)-[3-(2-Methoxyphenylthio)-2-(S)-methoxypropyl]amino-3,4-dihydro-2H-1,5-benzoxathiepin;
3-(R)-[3-(2-Methoxyphenylthio)-2-(S)-hydroxypropyl]amino-3,4-dihydro-2H-1,5-benzoxathiepin;
3-(R)-[3-(2-Hydroxyphenylthio)-2-(S)-hydroxypropyl]amino-3,4-dihydro-2H-1,5-benzoxathiepin;
3-(R)-[3-(2-Methoxyphenylthio)-2-(S)-methylpropyl]amino-3,4-dihydro-2H-1,5-benzoxathiepin;
3-(R)-[3-(2-Methoxyphenylthio)-2-(S)-ethylpropyl]amino-3,4-dihydro-2H-1,5-benzoxathiepin;
3-(R)-[3-(2-Methoxyphenylthio)-2-(S)-n-propylpropyl]amino-3,4-dihydro-2H-1,5-benzoxathiepin;
3-(R)-[3-(2-Methoxyphenylthio)-2-(S)-isopropylpropyl]amino-3,4-dihydro-2H-1,5-benzoxathiepin;
3-(R)-[3-(2-Methoxyphenylthio)-2-(S)-methylpropyl]amino-7-methyl-3,4-dihydro-2H-1,5-benzoxathiepin;
3-(R)-[3-(2-Methoxyphenylthio)-2-(S)-methylpropyl]amino-6-methyl-3,4-dihydro-2H-1,5-benzoxathiepin;
3-(R)-[3-(2-Hydroxy-3-methylphenylthio)-2-(S)-methylpropyl]amino-3,4-dihydro-2H-1,5-benzoxathiepin;
3-(R)-[3-(2-Hydroxyphenylthio)-2-(S)-methylpropyl]amino-3,4-dihydro-2H-1,5-benzoxathiepin;
3-(R)-[3-(2-Hydroxy-3-ethylphenylthio)-2-(S)-methylpropyl]amino-3,4-dihydro-2H-1,5-benzoxathiepin;
3-(R)-[3-(2,3-Dihydrobenzofuran-7-thio)-2-(S)-methytpropyl]amino-3,4-dihydro-2H-1,5-benzoxathiepin;
3-(R)-[3-(2-Hydroxy-3-methylphenylthio)-2-(S)-ethylpropyl]amino-3,4-dihydro-2H-1,5-benzoxathiepin;
3-(R)-[3-(2-Hydroxy-3-methylphenylthio)-2-(S)-isopropylpropyl]amino-3,4-dihydro-2H-1,5-benzoxathiepin;
3-(R)-[3-(2-Hydroxy-3-methylphenylthio)-2-(S)-methylpropyl]amino-6-methyl-3,4-dihydro-2H-1,5-benzoxathiepin;
3-(R)-[3-(2-Hydroxy-3-methoxyphenylthio)-2-(S)-methylpropyl]amino-3,4-dihydro-2H-1,5-benzoxathiepin;
3-(R)-[3-(2,3-Dimethoxyphenylthio)-2-(S)-methylpropyl]amino-3,4-dihydro-2H-1,5-benzoxathiepin;
3-(R)-[3-(2-Hydroxy-3-isopropylphenylthio)-2-(S)-methylpropyl]amino-3,4-dihydro-2H-1,5-benzoxathiepin;
3-(R)-[3-(2-Hydroxy-6-methylphenylthio)-2-(S)-methylpropyl]amino-3,4-dihydro-2H-1,5-benzoxathiepin;
deren Additionssalzen und den Hydraten dieser Additionssalze mit pharmazeutisch annehmbaren Mineralsäuren oder organischen Säuren,
sowie deren tautomeren Formen, den Enantiomeren und den Mischungen von Enantiomeren und den reinen Stereoisomeren entweder in racemischer Mischung oder nicht.

9. Amine der Formel (I) 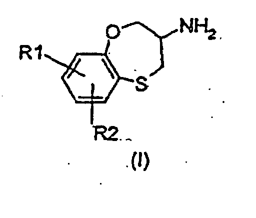 in der
R₁ und R₂, identisch oder verschieden:
- ein Wasserstoffatom;
- ein Fluoratom oder ein Chloratom;
- eine Hydroxygruppe;
- einen Alkylrest, verzweigt oder nicht, der 1 bis 3 Kohlenstoffatome einschließt;
- einen Cyclopropylrest;
- einen Alkoxyrest, verzweigt oder nicht, der 1 bis 3 Kohlenstoffatome einschließt;
- einen Cyclopropoxyrest darstellen oder,
- wenn die Gruppen R₁ und R₂ benachbarte Positionen am aromatischen Cyclus einnehmen, sie mit den Kohlenstoffatomen, welche sie tragen, einen Kohlenstoffcyclus oder einen nicht-aromatischen sauerstoffhaltigen Heterocyclus mit fünf Ringgliedern bilden;
als Zwischenprodukte bei der Synthese von Verbindungen der Formel (1).

10. Amine nach Anspruch 9, in denen das asymmetrische Kohlenstoffatom C(3) die absolute Konfiguration (R) aufweist.

11. Verfahren zur Herstellung von Verbindungen der Formel (I) nach irgendeinem der Ansprüche 9 und 10, **dadurch gekennzeichnet, dass** man N-Boc-(2-hydroxyphenyl)cystein mit der Formel (VIII) 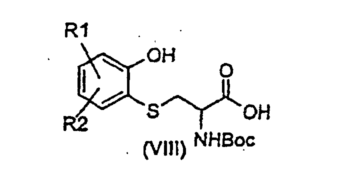 durch Reduktion eines gemischten Zwischenprodukt-Anhydrids, das in situ gebildet wird, mittels eines einfachen oder komplexen Borhydrids gemäß einer "Eintopf"-Technik in einen primären Alkohol der Formel (IX) 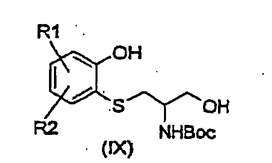 überführt, dann die Verbindung (IX) cyclisiert, um die cyclische Verbindung zu erhalten, die (X) entspricht 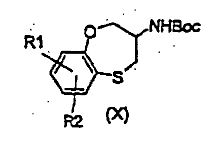 welche man mit einer protischen Säure behandelt, um das Amin der Formel (I) zu erhalten, welches man in eine Salz überführt, wenn man es wünscht.

12. Aldehyde der Formel (II) 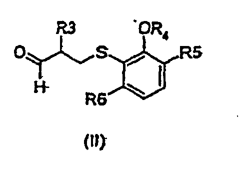 in der
R₃:
- einen Alkylrest, verzweigt oder nicht, der 1 bis 3 Kohlenstoffatome einschließt, oder einen Methoxyrest darstellt,
R₄:
- ein Wasserstoffatom oder einen Methylrest darstellt und
R₅ und R₆, identisch oder verschieden:
- ein Wasserstoffatom;
- einen Alkylrest, verzweigt oder nicht, der 1 bis 3 Kohlenstoffatome einschließt;
- einen Alkoxyrest, verzweigt oder nicht, der 1 bis 3 Kohlenstoffatome einschließt;
- einen Alkylthiorest, verzweigt oder nicht, der 1 bis 3 Kohlenstoffatome einschließt;
- einen Alkylaminorest darstellen,
mit der Maßgabe, dass, wenn R₄ einen Methylrest darstellt, dann R₅ ein Wasserstoffatom, einen Alkoxyrest, der 1 bis 3 Kohlenstoffatome einschließt, einen Alkylthiorest, verzweigt oder nicht, der 1 bis 3 Kohlenstoffatome einschließt, oder einen Alkylaminorest darstellt oder
die Gruppen OR₄ und R₅ mit den Kohlenstoffen, welche sie tragen, einen nicht-aromatischen Heterocyclus mit fünf oder sechs Ringgliedern bilden, der mindestens ein Sauerstoffatom enthält, und R₆ wie vorstehend definiert ist,
nützlich als Zwischenprodukte bei der Synthese von Verbindungen der Formel (1).

13. Aldehyde nach Anspruch 12, in denen das asymmetrische Kohlenstoffatom, das die Gruppe R₃ trägt, die absolute Konfiguration (S) aufweist.

14. Aldehyde der Formel (V) 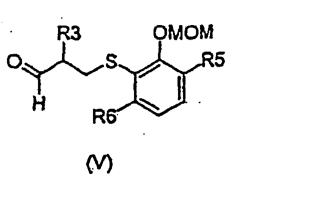 in der
R₃:
- einen Alkylrest, verzweigt oder nicht, der 1 bis 3 Kohlenstoffatome einschließt, oder einen Methoxyrest darstellt und
R₅ und R_{6,} identisch oder verschieden:
- ein Wasserstoffatom;
- einen Alkylrest, verzweigt oder nicht, der 1 bis 3 Kohlenstoffatome einschließt;
- einen Alkoxyrest, verzweigt oder nicht, der 1 bis 3 Kohlenstoffatome einschließt;
- einen Alkylthiorest, verzweigt oder nicht, der 1 bis 3 Kohlenstoffatome einschließt;
- einen Alkylaminorest darstellen,
nützlich als Zwischenprodukte bei der Synthese von Verbindungen der Formel (1).

15. Aldehyde nach Anspruch 14, in denen das asymmetrische Kohlenstoffatom, das die Gruppe R₃ trägt, die absolute Konfiguration (S) aufweist.

16. Verfahren zur Herstellung von Verbindungen der Formel (IIa) nach Anspruch 12, in der R₃ eine Methoxygruppe darstellt, und von Verbindungen der Formel (Va) nach Anspruch 13, in der R₃ eine Methoxygruppe darstellt, **dadurch gekennzeichnet, dass** die primäre Alkohol-Funktion des Zwischenprodukts vom Typ 3-Arylthio-1,2-propandiol (XI) 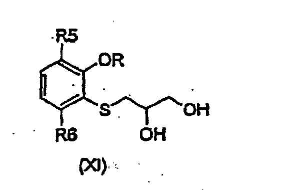 worin R einen Methyl- oder Methoxymethylrest darstellt und R₅ und R₆ wie in Anspruch 1 definiert sind, in Form eines Tritylethers der Formel (XII) 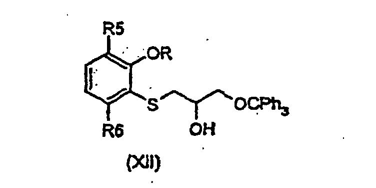 geschützt wird, in Form eines alkalischen, Alkoholats aktiviert. wird, dann mittels eines Methylhalogenids oder -sulfats methyliert wird, um die Verbindung der Formel (XIII) zu ergeben, 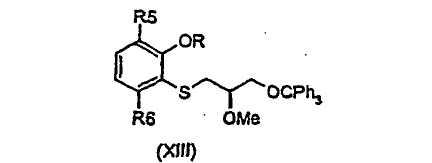 in der R wie vorstehend definiert ist, und dass man die primäre Alkohol-Funktion durch Hydrolyse der Triphenylmethyl-Gruppe in saurem protischem Medium freisetzt und
- wenn R ein Methylrest ist, eine Verbindung der Formel (XIVa1) erhält, 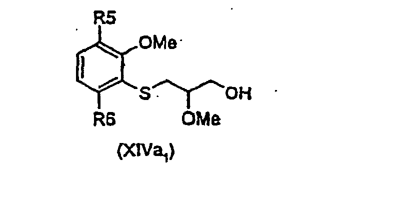 bei der man den primären Alkohol oxidiert und eine Verbindung der Formel (IIa) erhält, 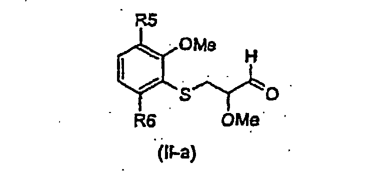
- wenn R ein Methoxymethylrest ist, eine Verbindung der Formel (XIVa2) erhält, 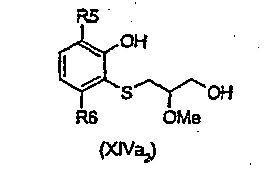 bei der man die Phenol-Funktion durch chemoselektive Alkylierung mittels Chlormethylmethylether schützt und eine Verbindung der Formel (XV) erhält, 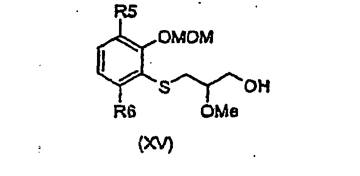 bei der man den primären Alkohol oxidiert und eine Verbindung der Formel (Va) erhält.

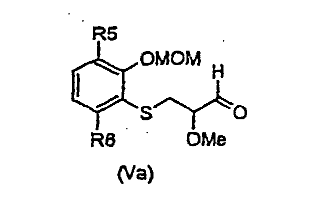

17. Verfahren zur Herstellung von Verbindungen der Formel (II) nach Anspruch 12 und von Verbindungen der Formel (V) nach Anspruch 13, in denen R₃ einen Alkylrest darstellt, **dadurch gekennzeichnet, dass** man
- entweder eine Verbindung der Formel (XVI) 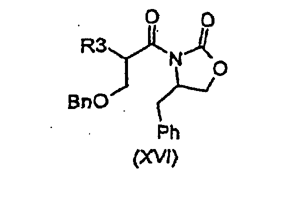 in der R₃ einen Alkylrest, verzweigt oder nicht, darstellt, der 1 bis 3 Kohlenstoffatome einschließt, reduziert
und einen Alkohol der Formel (XVII) 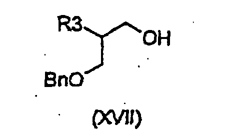 erhält, den man in einen p-Toluolsulfonsäureester (ein Tosylat) der Formel (XVIII) 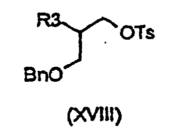 überführt, welchen man in Anwesenheit eines Palladium-Katalysators einer Hydrogenolyse unterzieht, um die Verbindung (XIX) 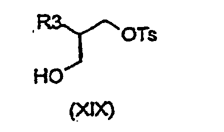 zu ergeben, welche man mit einer Verbindung der Formel (IV), gegebenenfalls in Form eines Alkalisalzes, reagieren lässt, 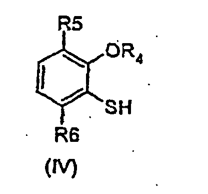 in der
R₄:
- ein Wasserstoffatom oder einen Methylrest darstellt und
R₅ und R₆, identisch oder verschieden:
- ein Wasserstoffatom;
- einen Alkylrest, verzweigt oder nicht, der 1 bis 3 Kohlenstoffatome einschließt;
- einen Alkoxyrest, verzweigt oder nicht, der 1 bis 3 Kohlenstoffatome einschließt;
- einen Alkylthiorest, verzweigt oder nicht, der 1 bis 3 Kohlenstoffatome einschließt;
- einen Alkylaminorest darstellen,
mit der Maßgabe, dass, wenn R₄ einen Methylrest darstellt, dann R₅ ein Wasserstoffatom, einen Alkoxyrest, der 1 bis 3 Kohlenstoffatome einschließt, einen Alkylthiorest, verzweigt oder nicht, der 1 bis 3 Kohlenstoffatome einschließt, oder einen Alkylaminorest darstellt, oder
die Gruppen OR₄ und R₅ mit den Kohlenstoffen, welche sie tragen, einen nicht-aromatischen Heterocyclus mit fünf oder sechs Kettengliedern bilden, der mindestens ein Sauerstoffatom enthält, und R₆ wie vorstehend definiert ist,
- oder dass man 3-Brom-2-methylpropan-1-ol mit einer Verbindung der Formel (IV) reagieren lässt und eine Verbindung der Formel (XX) erhält, 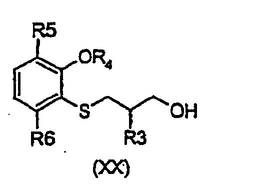 dann, wenn R₄ ein Alkylrest ist oder mit der benachbarten Gruppe R₅ einen Heterocyclus bildet, direkt eine Oxidation der Verbindung der Formel (XX) zu einem Aldehyd der Formel (II) durchführt, 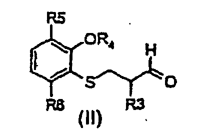 wenn R₄ ein Wasserstoffatom ist, dann die Phenol-Funktion der Verbindung der Formel (XX) in die Verbindung der Formel (XXI) überführt wird, 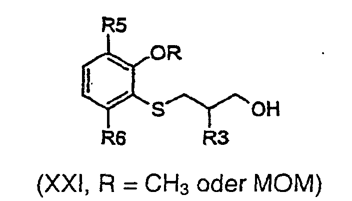 in der R einen Methoxymethylrest (XXIa, R = MOM) oder einen Methylrest (XXIb, R = CH₃) darstellt,
dann der Alkohol (XXIa) zum Aldehyd (II) 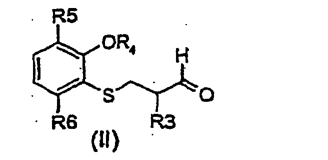 und der Alkohol (XXIb) zum Aldehyd (V) oxidiert wird

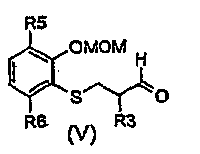

18. Verbindungen nach einem der Ansprüche 1, 2, 3, 7 und 8 als Medikamente.

19. Pharmazeutische Zusammensetzungen, **dadurch gekennzeichnet, dass** sie als Wirkstoff mindestens eine Verbindung nach einem der Ansprüche 1, 2, 3, 7 und 8 in Verbindung mit einem inerten pharmazeutischen Träger oder anderen pharmazeutisch annehmbaren Vehikeln und gegebenenfalls einem anderen Medikament enthalten.

20. Pharmazeutische Zusammensetzung nach Anspruch 19, die bei der Behandlung von stabiler Herzbräune, instabiler Herzbräune, Herzinsuffizienz, dem langen QT-Syndrom angeborenen Ursprungs, Myokardinfarkt und Herzrythmusstörungen nützlich sind.

21. Pharmazeutische Zusammensetzungen nach Anspruch 19, die bei der Behandlung von Zerebralischämie, transitorischen ischämischen Anfällen, Neuropathien traumatischer oder ischämischer Natur und Epilepsie nützlich sind.

22. Pharmazeutische Zusammensetzungen nach Anspruch 19, die bei der Behandlung von Schmerzen neuropathischen Ursprungs und neurodegenerativen Krankheiten nützlich sind.

## Claims

1. 3-Arylthiopropylamino-3,4-dihydro-2H-1,5-benzoxathiepine derivatives of general formula (1) 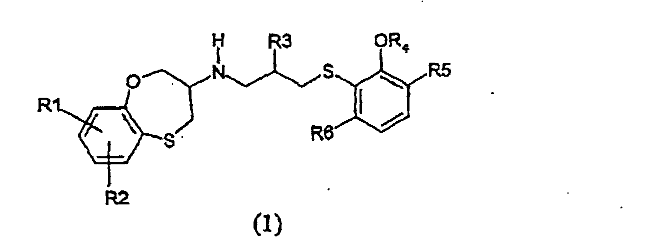 in which
R₁ and R₂, which are identical or different, represent:
a hydrogen atom;
- a fluorine atom or a chlorine atom;
- a hydroxyl group;
- a linear or branched alkyl radical including from 1 to 3 carbon atoms;
- a cyclopropyl radical;
- a cyclopropoxy radical; or
- when the R₁ and R₂ groups occupy adjacent positions on the aromatic ring, then they form, with the carbon atoms which carry them, a nonaromatic five-membered oxygen-comprising heterocycle or carbonaceous ring;
R₃ represents:
- a linear or branched alkyl radical including from 1 to 3 carbon atoms;
- a hydroxyl group or a methoxy radical;
R₄ represents:
- a hydrogen atom or a methyl radical; and
R₅ and R₆, which are identical or different, represent:
- a hydrogen atom;
- a linear or branched alkyl radical including from 1 to 3 carbon atoms;
- a linear or branched alkoxy radical including from 1 to 3 carbon atoms;
- a linear or branched alkylthio radical including from 1 to 3 carbon atoms;
- an alkylamino radical;
provided that, when R₄ represents a methyl radical, then R₅ represents a hydrogen atom, an alkoxy radical including from 1 to 3 carbon atoms, a linear or branched alkylthio radical including from 1 to 3 carbon atoms or an alkylamino radical, or
the OR₄ and R₅ groups form, with the carbons which carry them, a nonaromatic five- or six-membered heterocycle comprising at least one oxygen atom, and R₆ is as defined above,
their addition salts and the hydrates of these addition salts with pharmaceutically acceptable inorganic acids or organic acids,
and their tautomeric forms, the enantiomers and the mixtures of enantiomers, and the stereoisomers, pure or as a racemic or nonracemic mixture.

2. Derivatives according to Claim 1, **characterized in that**
R₁ and R₂, which are identical or different, represent:
- a hydrogen atom;
- a fluorine atom or a chlorine atom;
- a hydroxyl group;
- an alkyl radical chosen from the group consisting of the methyl, ethyl, propyl and isopropyl radicals;
- a cyclopropyl radical;
- an alkoxy radical chosen from the group consisting of the methoxy, ethoxy, propoxy and isopropoxy radicals;
- a cyclopropoxy radical; or
- when the R₁ and R₂ groups occupy adjacent positions on the aromatic ring, then R₁R₂ represent -CH₂CH₂CH₂-, -OCH₂CH₂-, -OCH₂O- or -CH₂CH₂O-;
R₃ represents;
- an alkyl radical chosen from the group consisting of the methyl, ethyl, propyl and isopropyl radicals;
- a hydroxyl group or a methoxy radical;
R₄ represents:
- a hydrogen atom or a methyl radical; and
R₅ and R₆, which are identical or different, represent:
- a hydrogen atom;
- an alkyl radical chosen from the group consisting of the methyl, ethyl and isopropyl radicals;
- an alkoxy radical chosen from the group consisting of the methoxy, ethoxy, propoxy and isopropoxy radicals;
- an alkylthio radical chosen from the group.consisting of the methylthio, ethylthio and isopropylthio radicals;
- an alkylamino radical chosen from the group consisting of the N-methylamino and N,N-dimethylamino radicals; or
R₄R₅ represents a radical chosen from the group consisting of -CH₂CH₂-, -CH₂O-, -CH₂CH₂O-, -CH₂CH₂S- and -CH₂CH₂NR₄-, and R₆ is as defined above,
their addition salts and the hydrates of these addition salts with pharmaceutically acceptable inorganic acids or organic acids,
and their tautomeric forms, the enantiomers and the mixtures of enantiomers, and the stereoisomers, pure or as a racemic or nonracemic mixture.

3. Derivatives according to Claim 1, **characterized in that** they are chosen from the following compounds:
3-[3-(2-methoxyphenylthio)-2-methoxypropyl]amino-3,4-dihydro-2H-1,5-benzoxathiepine;
3-[3-(2-methoxyphenylthio)-2-hydroxypropyl]amino-3,4-dihydro-2H-1,5-benzoxathiepine;
3-[3-(2-hydroxyphenylthio)-2-hydroxypropyl]amino-3,4-dihydro-2H-1,5-benzoxathiepine;
3-[3-(2-methoxyphenylthio)-2-methylpropyl]amino-3,4-dihydro-2H-1,5-benzoxathiepine;
3-[3-(2-methoxyphenylthio)-2-ethylpropyl]amino-3,4-dihydro-2H-1,5-benzoxathiepine;
3-[3-(2-methoxyphenylthio)-2-(n-propyl)propyl]amino-3,4-dihydro-2H-1,5-benzoxathiepine;
3-[3-(2-methoxyphenylthio)-2-(isopropyl)propyl]amino-3,4-dihydro-2H-1,5-benzoxathiepine;
3-[3-(2-methoxyphenylthio)-2-methylpropyl]amino-7-methyl-3,4-dihydro-2H-1,5-benzoxathiepine;
3-[3-(2-methoxyphenylthio)-2-methylpropyl]amino-6-methyl-3,4-dihydro-2H-1,5-benzoxathiepine;
3-[3-(2-hydroxy-3-methylphenylthio)-2-methylpropyl]-amino-3,4-dihydro-2H-1,5-benzoxathiepine;
3-[3-(2-hydroxyphenylthio)-2-methylpropyl]amino-3,4-dihydro-2H-1,5-benzoxathiepine;
3-[3-(2-hydroxy-3-ethylphenylthio)-2-methylpropyl]-amino-3,4-dihydro-2H-1,5-benzoxathiepine;
3-[3-(2,3-dihydrobenzofuran-7-thio)-2-methylpropyl]-amino-3,4-dihydro-2H-1,5-benzoxathiepine;
3-[3-(2-hydroxy-3-methylphenylthio)-2-ethylpropyl]-amino-3,4-dihydro-2H-1,5-benzoxathiepine;
3-[3-(2-hydroxy-3-methylphenylthio)-2-(isopropyl)-propyl]amino-3,4-dihydro-2H-1,5-benzoxathiepine;
3-[3-(2-hydroxy-3-methylphenylthio)-2-methylpropyl]-amino-6-methyl-3,4-dihydro-2H-1,5-benzoxathiepine;
3-[3-(2-hydroxy-3-methoxyphenylthio)-2-methylpropyl]-amino-3,4-dihydro-2H-1,5-benzoxathiepine;
3-[3-(2,3-dimethoxyphenylthio)-2-methylpropyl]amino-3,4-dihydro-2H-1,5-benzoxathiepine;
3-[3-(2-hydroxy-3-(isopropyl)phenylthio)-2-methylpropyl]amino-3,4-dihydro-2H-1,5-benzoxathiepine;
3-[3-(2-hydroxy-6-methylphenylthio)-2-methylpropyl]-amino-3,4-dihydro-2H-1,5-benzoxathiepine,
their addition salts and the hydrates of these addition salts with pharmaceutically acceptable inorganic acids or organic acids,
and their tautomeric forms, the enantiomers and the mixtures of enantiomers, and the stereoisomers, pure or as a racemic or nonracemic mixture.

4. Process for the preparation of the compounds of general formula (1) according to any one of Claims 1 to 3 in which R₃ represents a branched alkyl radical including from 1 to 3 carbon atoms or a methoxy radical and R₄ represents a methyl radical, **characterized in that** an amine of formula (I) 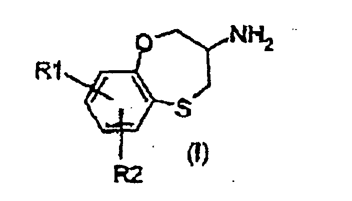 in which R₁ and R₂ are as defined in Claim 1, or one of these salts, is reacted with an aldehyde of formula (II) 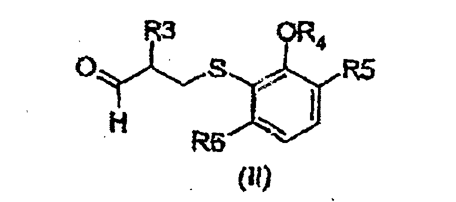 in which R₃ represents a branched alkyl radical including from 1 to 3 carbon atoms or a methoxy radical, R₄ represents a methyl radical and R₅ and R₆ are as defined in Claim 1,
in the presence of a reducing agent and at a temperature of between -20°C and + 25°C, to give a compound of formula (1)

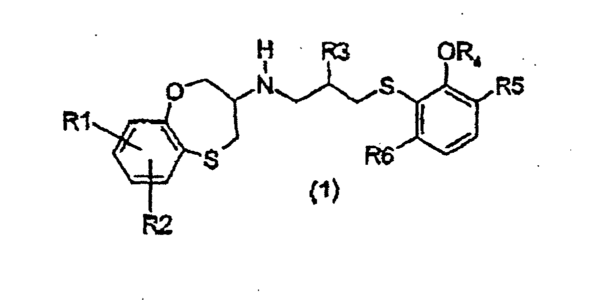

5. Process for the preparation of the compounds of general formula (1) according to any one of Claims 1 to 3 in which R₃ represents a branched alkyl radical including from 1 to 3 carbon atoms or a methoxy radical and R₄ represents a hydrogen atom, **characterized in that** an amine of formula (I) 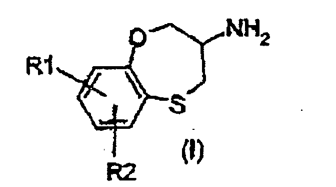 in which R₁ and R₂ are as defined in Claim 1, or one of these salts, is reacted with a compound of formula (V) 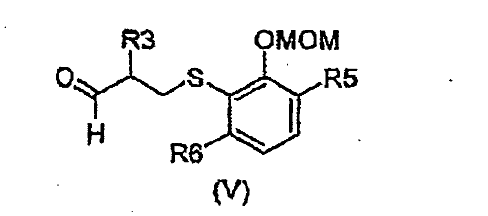 in which R₃ represents a branched alkyl radical including from 1 to 3 carbon atoms or a methoxy radical and R₅ and R₆ are as defined in Claim 1, in the presence of a reducing agent and at a temperature of between - 20°C and + 25°C,
to give a compound of formula (VI) 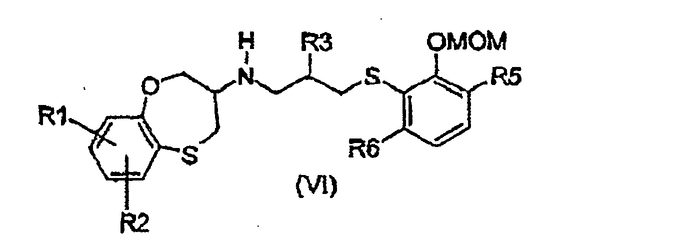 which is hydrolysed to give a compound of formula (1) 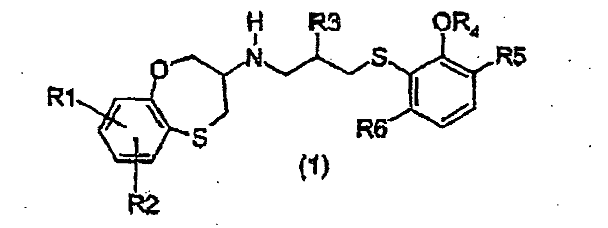 in which R₃ represents a branched alkyl radical including from 1 to 3 carbon atoms or a methoxy radical and R₄ a hydrogen atom.

6. Process for the preparation of the compounds of general formula (1) according to Claims 1 and 2 in which R₃ is a hydroxyl group, **characterized in that** an epoxide of formula (III) 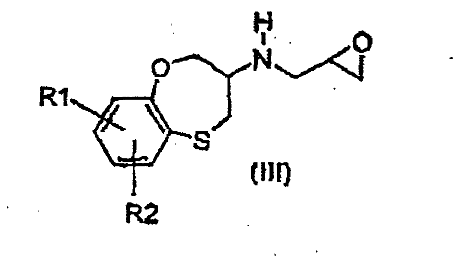 in which R₁ and R₂ are as defined in Claim 1,
is reacted with an arylthiol of formula (IV) 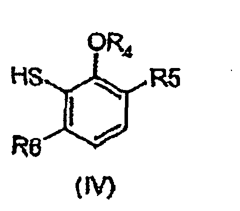 in which R₄, R₅ and R₆ are as defined in Claim 1,
in a protic solvent, in the presence of an inorganic base and at a temperature of between 20°C and 70°C, to give a compound of formula (1) 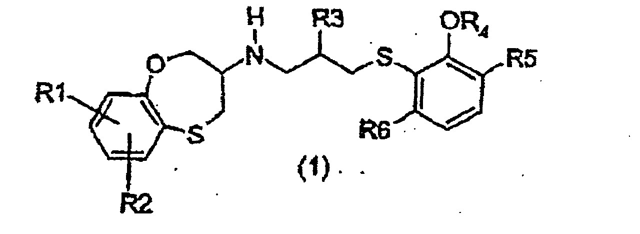 in which R₁, R₂, R₄, R₅ and R₆ are as defined in Claim 1 and R₃ is a hydroxyl group.

7. Derivatives of general formula (1) according to either one of Claims 1 and 2, **characterized in that** they have the (R) absolute configuration at the C(3) asymmetric carbon atom of the 3,4-dihydro-2H-1,5-benzoxathiepine fragment and the (S) absolute configuration at the asymmetric carbon atom which carries the R₃ group.

8. Derivative of general formula (1) according to Claim 7, **characterized in that** it is chosen from the following stereoisomers:
3-(R)-[3-(2-methoxyphenylthio)-2-(S)-methoxypropyl]-amino-3,4-dihydro-2H-1,5-benzoxathiepine;
3-(R)-[3-(2-methoxyphenylthio)-2-(S)-hydroxypropyl]-amino-3,4-dihydro-2H-1,5-benzoxathiepine;
3-(R)-[3-(2-hydroxyphenylthio)-2-(S)-hydroxypropyl]-amino-3,4-dihydro-2H-1,5-benzoxathiepine;
3-(R)-[3-(2-methoxyphenylthio)-2-(S)-methylpropyl]-amino-3,4-dihydro-2H-1,5-benzoxathiepine;
3-(R)-[3-(2-methoxyphenylthio)-2-(S)-ethylpropyl]amino-3,4-dihydro-2H-1,5-benzoxathiepine;
3-(R)-[3-(2-methoxyphenylthio)-2-(S)-(n-propyl)propyl]-amino-3,4-dihydro-2H-1,5-benzoxathiepine;
3-(R)-[3-(2-methoxyphenylthio)-2-(S)-(isopropyl)-propyl]amino-3,4-dihydro-2H-1,5-benzoxathiepine;
3-(R)-[3-(2-methoxyphenylthio)-2-(S)-methylpropyl]-amino-7-methyl-3,4-dihydro-2H-1,5-benzoxathiepine;
3-(R)-[3-(2-methoxyphenylthio)-2-(S)-methylpropyl]-amino-6-methyl-3,4-dihydro-2H-1,5-benzoxathiepine;
3-(R)-[3-(2-hydroxy-3-methylphenylthio)-2-(S)-methylpropyl]amino-3,4-dihydro-2H-1,5-benzoxathiepine;
3-(R)-[3-(2-hydroxyphenylthio)-2-(S)-methylpropyl]-amino-3,4-dihydro-2H-1,5-benzoxathiepine;.
3-(R)-[3-(2-hydroxy-3-ethylphenylthio)-2-(S)-methylpropyl]amino-3,4-dihydro-2H-1,5-benzoxathiepine;
3-(R)-[3-(2,3-dihydrobenzofuran-7-thio)-2-(S)-methylpropyl]amino-3,4-dihydro-2H-1,5-benzoxathiepine;
3-(R)-[3-(2-hydroxy-3-methylphenylthio)-2-(S)-ethylpropyl]amino-3,4-dihydro-2H-1,5-benzoxathiepine;
3-(R)-[3-(2-hydroxy-3-methylphenylthio)-2-(S)-(isopropyl)proryl]amino-3,4-dihydro-2H-1,5-benzoxathiepine;
3-(R)-[3-(2-hydroxy-3-methylphenylthio)-2-(S)-methylpropyl]amino-6-methyl-3,4-dihydro-2H-1,5-benzoxathiepine;
3-(R)-[3-(2-hydroxy-3-methoxyphenylthio)-2-(S)-methylpropyl]amino-3,4-dihydro-2H-1,5-benzoxathiepine;
3-(R)-[3-(2,3-dimethoxyphenylthio)-2-(S)-methylpropyl]-amino-3,4-dihydro-2H-1,5-benzoxathiepine;
3-(R)-[3-(2-hydroxy-3-isopropylphenylthio)-2-(S)-methylpropyl]amino-3,4-dihydro-2H-1,5-benzoxathiepine;
3-(R)-[3-(2-hydroxy-6-methylphenylthio)-2-(S)-methylpropyl]amino-3,4-dihydro-2H-1,5-benzoxathiepine,
their addition salts and the hydrates of these addition salts with pharmaceutically acceptable inorganic acids or organic acids,
and their tautomeric forms, the enantiomers and the mixtures of enantiomers, and the stereoisomers, pure or as a racemic or nonracemic mixture.

9. Amines of formula (I) 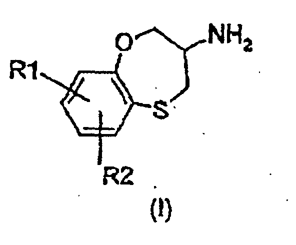 in which
R₁ and R₂, which are identical or different, represent:
- a hydrogen atom;
- a fluorine atom or a chlorine atom;
- a hydroxyl group;
- a linear or branched alkyl radical including from 1 to 3 carbon atoms;
- a cyclopropyl radical;
- a linear or branched alkoxy radical including from 1 to 3 carbon atoms;
- a cyclopropoxy radical; or
- when the R₁ and R₂ groups occupy adjacent positions on the aromatic ring, they form, with the carbon atoms which carry them, a nonaromatic five-membered oxygen-comprising heterocycle or carbonaceous ring;
as intermediates in the synthesis of the compounds of formula (1).

10. Amines according to Claim 9, in which the C(3) asymmetric carbon atom has the (R) absolute configuration.

11. Process for the preparation of the compounds of formula (I) according to either one of Claims 9 and 10, **characterized in that** the N-Boc-(2-hydroxyphenyl)-cysteine of formula (VIII) 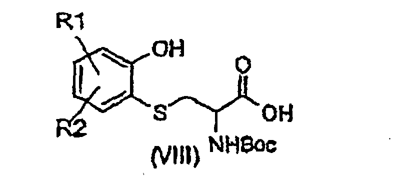 is converted to the primary alcohol of formula (IX) 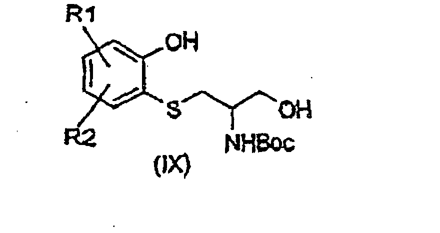 by reduction of an intermediate mixed anhydride, formed in situ, using a simple or complex borohydride according to a one-pot technique,
and then the said compound (IX) is cyclized to produce the corresponding cyclic compound (X) 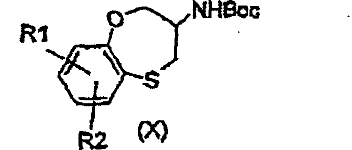 which is treated with a protic acid to produce the amine of formula (I), which is salified, if desired.

12. Aldehydes of formula (II) 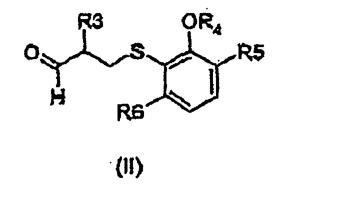 in which
R₃ represents
- a linear or branched alkyl radical including from 1 to 3 carbon atoms or a methoxy radial,
R₄ represents:
- a hydrogen atom or a methyl radical, and
R₅ and R₆, which are identical or different, represent:
- a hydrogen atom;
- a linear or branched alkyl radical including from 1 to 3 carbon atoms;
- a linear or branched alkoxy radical including from 1 to 3 carbon atoms;
- a linear or branched alkylthio radical including from 1 to 3 carbon atoms;
- an alkylamino radical,
provided that, when R₄ represents a methyl radical, then R₅ represents a hydrogen atom, an alkoxy radical including from 1 to 3 carbon atoms, a linear or branched alkylthio radical including from 1 to 3 carbon atoms or an alkylamino radical, or
the OR₄ and R₅ groups form, with the carbons which carry them, a nonaromatic five- or six-membered heterocycle comprising at least one oxygen atom, and R₆ is as defined above,
of use as intermediates in the synthesis of the compounds of formula (1).

13. Aldehydes according to Claim 12, in which the asymmetric carbon atom carrying the R₃ group has the (S) absolute configuration.

14. Aldehydes of formula (V) 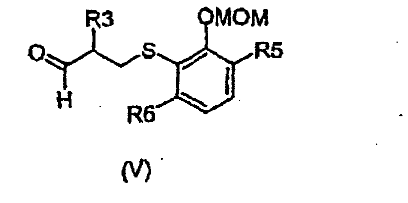 in which
R₃ represents
- a linear or branched alkyl radical including from 1 to 3 carbon atoms or a methoxy radical, and
R₅ and R₆, which are identical or different, represent:
- a hydrogen atom;
- a linear or branched alkyl radical including from 1 to 3 carbon atoms;
- a linear or branched alkoxy radical including from 1 to 3 carbon atoms;
- a linear or branched alkylthio radical including from 1 to 3 carbon atoms;
- an alkylamino radical,
of use as intermediates in the synthesis of the compounds of formula (1).

15. Aldehydes according to Claim 14, in which the asymmetric carbon atom carrying the R₃ group has the (S) absolute configuration.

16. Process for the preparation of the compounds of formula (IIa) according to Claim 12 in which R₃ represents a methoxy group and of the compounds of formula (Va) according to Claim 13 in which R₃ represents a methoxy group, **characterized in that** the primary alcohol functional group of the intermediate of the 3-arylthio-1,2-propanediol type (XI) 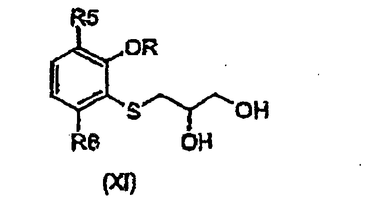 in which R represents a methyl or methoxymethyl radical and R₅ and R₆ are as defined in Claim 1, is protected in the form of the trityl ether of formula (XII), 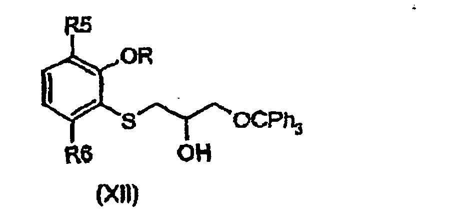 the secondary alcohol functional group is activated in the form of an alkali metal alkoxide and then methylated using a methyl halide or sulfate to give the compound of formula (XIII) 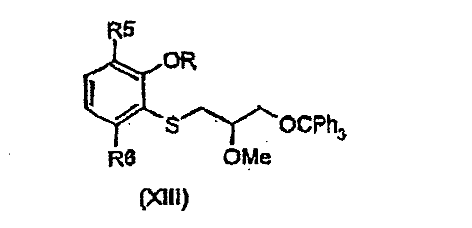 in which R is as defined above, and the primary alcohol functional group is released by hydrolysis of the triphenylmethyl group in a protic acidic medium,
and the following compounds are obtained,
- when R is a methyl radical, a compound of formula (XIVa1) 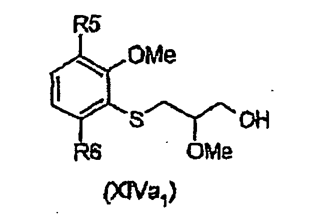 the primary alcohol of which is oxidized and a compound of formula (IIa) is obtained 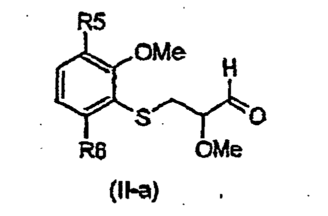
- when R is a methoxymethyl radical, a compound of formula (XIVa2) is obtained 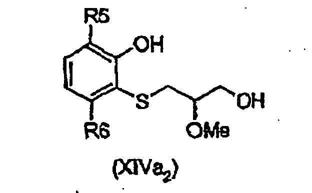 the phenol functional group of which is protected by chemoselective alkylation using chloromethyl methyl ether and a compound of formula (XV) is obtained 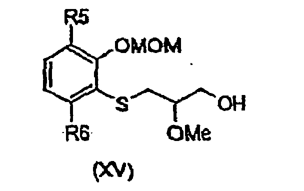 the primary alcohol of which is oxidized and a compound of formula (Va) is obtained

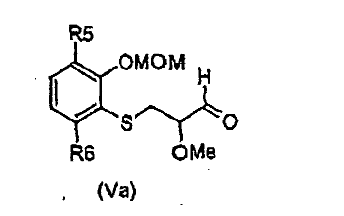

17. Process for the preparation of the compounds of formula (II) according to Claim 12 and of the compounds of formula (V) according to Claim 13 in which R₃ represents an alkyl radical, **characterized in that**
- either a compound of formula (XVI) 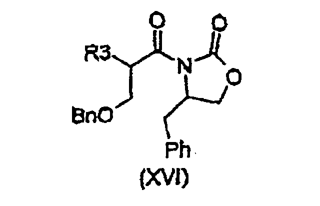
in which R₃ represents a linear or branched alkyl radical including from 1 to 3 carbon atoms, is reduced and an alcohol of formula (XVII) 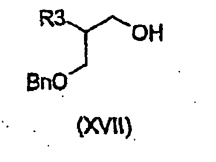 is obtained,
which is converted to a p-toluenesulfonic acid ester (tosylate) of formula (XVIII) 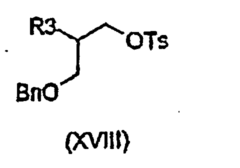 which is subjected to hydrogenolysis in the presence of a palladium catalyst to give the compound (XIX) 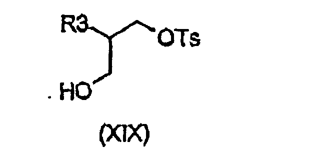 which is reacted with a compound of formula (IV), optionally in the form of an alkali metal salt, 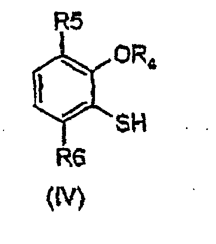 in which
R₄ represents:
- a hydrogen atom or a methyl radical, and
R₅ and R₆, which are identical or different, represent:
- a hydrogen atom;
- a linear or branched alkyl radical including from 1 to 3 carbon atoms;
- a linear or branched alkoxy radical including from 1 to 3 carbon atoms;
- a linear or branched alkylthio radical including from 1 to 3 carbon atoms;
- an alkylamino radical,
provided that, when R₄ represents a methyl radical, then R₅ represents a hydrogen atom, an alkoxy radical including from 1 to 3 carbon atoms, a linear or branched alkylthio radical including from 1 to 3 carbon atoms or an alkylamino radical, or
the OR₄ and R₅ groups form, with the carbons which carry them, a nonaromatic five- or six-membered heterocycle comprising at least one oxygen atom, and R₆ is as defined above,
- or 3-bromo-2-methylpropan-1-ol is reacted with a compound of formula (IV)
and a compound of formula (XX) 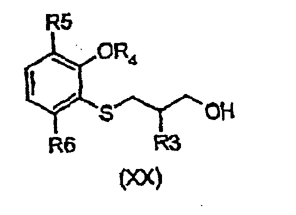 is obtained,
then, when R₄ is an alkyl radical or forms, with the adjacent R₅ group, a heterocycle, the compound of formula (XX) is oxidized directly to the aldehyde of formula (II) 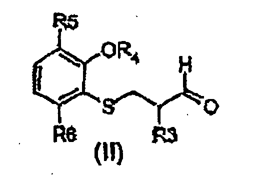 or, when R₄ is a hydrogen atom, then the phenol functional group of the compound of formula (XX) is converted to the compound of formula (XXI) 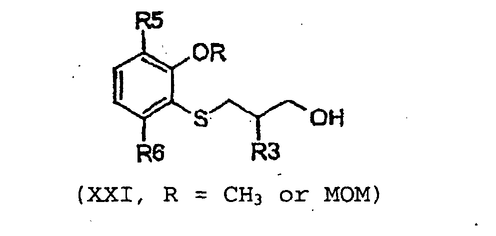
in which R represents a methoxymethyl radical (XXIa, R = MOM) or a methyl radical (XXIb, R = CH₃), then the alcohol (XXIa) is oxidized to the aldehyde (II) 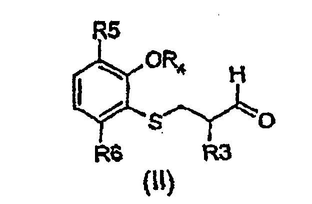 and the alcohol (XXIb) to the aldehyde (V)

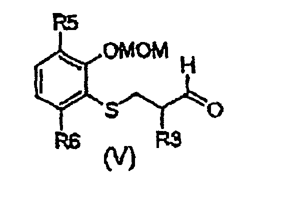

18. Compounds according to one of Claims 1, 2, 3, 7 and 8 as medicaments.

19. Pharmaceutical compositions, **characterized in that** they comprise, as active ingredient, at least one compound according to one of Claims 1, 2, 3, 7 and 8 in combination with an inert pharmaceutical carrier or other pharmaceutically acceptable vehicles and optionally with another medicament.

20. Pharmaceutical compositions according to Claim 19, of use in the treatment of stable angina, unstable angina, cardiac insufficiency, long QT syndrome of congenital origin, myocardial infarction and cardiac rhythm disorders.

21. Pharmaceutical compositions according to Claim 19, of use in the treatment of cerebral ischemia, transitory ischemic attack, neuropathies of a traumatic or ischemic nature, and epilepsy.

22. Pharmaceutical compositions according to Claim 19, of use in the treatment of pain of neuropathic origin and of neurodegenerative diseases.
